# EUROPEAN PATENT SPECIFICATION

(11) **EP 2 964 181 B1**
(45) Date of publication and mention of the grant of the patent: **30.05.2018**
(21) Application number: 14713975.2
(22) Date of filing: 03.03.2014
(51) Int. Cl.: A61K 8/04, A61K 8/58, A61K 8/81, A61Q 5/12, A61Q 5/02

(54) **POLYMERS AND METHODS TO MITIGATE THE LOSS OF SILICONE DEPOSITION FROM KERATINOUS SUBSTRATES**
POLYMERE UND METHODEN UM DEN VERLUST DES SILICON-DEPOTMATERIALS VON KERATINHALTIGEN SUBSTRAT ZU VERRINGERN
POLYMERES ET MÉTHODES POUR ATTÉNUER LA PERTE DE DÉPÔT DE SILICIUM DE SUBSTRAT CERATINEUX

(30) Priority: 08.03.2013 US 201361774844 P
(43) Date of publication of application: 13.01.2016
(73) Proprietor: Lubrizol Advanced Materials, Inc., Cleveland, OH 44141-3247 (US)
(72) Inventor: RAFFERTY, Denise W., Cleveland, Ohio 44141-3247 (US); FIGURA, Brian D., Cleveland, Ohio 44141-3247 (US); YANG, Wei-Yeih, Cleveland, Ohio 44141-3247 (US); CHARI, Krishnan, Cleveland, Ohio 44141-3247 (US)
(74) Representative: dompatent von Kreisler Selting Werner - Partnerschaft von Patent- und Rechtsanwälten mbB
(86) International application number: PCT/US2014/019772
(87) International publication number: WO 2014/137859

(56) References cited:
- WO-A1-2010/026097
- JP-A- 2008 150 359

## Description

### FIELD OF THE INVENTION

The present invention relates to personal care compositions, such as detersive conditioning
compositions for keratinous substrates. More specifically, the invention relates to detersive conditioning compositions suitable for use in personal care cleansing applications. The conditioning cleansing compositions comprise water, at least one detersive surfactant, a silicone, and at least one surfactant activated microgel polymer that mitigates the loss of silicone(s) deposited on keratinous substrates from cleansing compositions.

### BACKGROUND OF THE INVENTION

Historically, it has been considered desirable to cleanse the hair and skin and then to condition them after cleansing. In the past it was necessary to perform these steps in two separate procedures. With the advent of two-in-one cleansers (e.g., conditioning shampoos), it became possible to condition and cleanse simultaneously. Detersive conditioning compositions such as the two-in-one conditioning shampoos are well-known in the art. Two-in-one conditioning shampoos are popular among consumers as a means of conveniently obtaining hair conditioning and cleansing performance from a single hair care product. One of the most important classes of conditioning agents utilized by cleansing product formulators is the silicones. Wide varieties of silicone conditioning agents are known. A general review of silicone conditioning agents is set forth in the Cosmetic Science and Technology Series; Vol. 2, entitled Conditioning Agents for Hair and Skin, edited by Randy Schueller and Perry Romanowski, copyright 1999 by Marcel Dekker, Inc., New York, New York.

During formulation, silicones are suspended in shampoo compositions as dispersed oily hydrophobic emulsion droplets. The conditioning effect is achieved by the silicone material being deposited onto the hair during rinsing, resulting in the formation of a film. Silicone film deposition is known to give good sensory benefits (e.g., smoothness, soft feel), provide improved tangle resistance in wet and dry combing, and repair of damaged hair (e.g. split ends).

The basis for making an emulsion is to stabilize the insoluble silicone material in the aqueous phase. In the early two-in-one formulations, the silicone droplets (particles) were stabilized by adding surfactants to the formulation. The choice and amount of surfactant became critical in determining the type of emulsion formed, and its uniformity and stability. However, such compositions often had relatively low viscosities and were perceived by the consumer to be low in quality as a result. Moreover, obtaining good deposition of the silicone conditioning agent is further complicated by the detersive action of the surfactants in the shampoo. Detersive surfactants by their very nature carry away or remove oil, grease, dirt, and particulate matter from the treated substrate. In doing so, the detersive surfactants can also interfere with deposition of the silicone conditioning agent by carrying away both deposited and non-deposited silicone material during rinsing.

In conditioning applications, the particle size of the silicone was also determined to be important. The effectiveness of the emulsion to provide the conditioning effect depends upon the amount of silicone that is deposited onto the hair. As discussed in U.S. Pat. No. 5,302,658 the larger the particle size of the silicone material, the faster the destabilization or breaking of the emulsion during rinsing, thus increasing the deposition of the silicone onto the hair.

A problem encountered in the formulation of silicone containing cleansing compositions, such as the two-in-one shampoos, is keeping the dispersed water insoluble silicone oily materials suspended and the total product stable while still providing satisfactory rheology (e.g., thickening, yield value, shear thinning and shear flow) as well as detersive and conditioning performance. Poorly stabilized silicone containing shampoos are prone to phase separation leading to the formation of an unattractive silicone layer at the surface of the shampoo which is detrimental to the performance and the consumer appeal of the product. A variety of materials, including rheology modifiers, have been incorporated into silicone containing cleansing compositions for purposes of thickening and stabilization against phase separation.

Rheology modifiers have been used in shampoo products to increase viscosity at low shear rates and to maintain flow properties at higher shear rates. In addition, it has been discovered that certain rheology modifiers not only provide for a thickening effect, but also provide for effective storage stable suspensions of insoluble and particulate materials in aqueous surfactant systems. A select kind of copolymer thickener has been proposed for this purpose. For example, U.S. Pat. No. 6,635,702 discloses a crosslinked acrylic emulsion polymer for use in aqueous surfactant containing compositions to thicken and stabilize products containing insoluble and particulate materials including silicones. U.S. Pat. Appln. Pub. No. 2003/0108503 discloses a cosmetic composition containing a crosslinked acrylic copolymer prepared from methacrylic acid and a C₁-C₄ alkyl acrylate, at least one polymer chosen from cationic and amphoteric polymers and at least one particulate silicone. The compositions are said to be stable, have an attractive visual appearance and can provide good cosmetic properties to the hair, for example, lightness, softness, smooth feel, suppleness and manageability.

While crosslinked acrylic copolymers prepared from methacrylic acid and a C₁-C₄ alkyl acrylate are known to provide storage stable suspensions of silicone particles in aqueous surfactant compositions, it has been found that only silicones of larger particle sizes (e.g., average particle size ≥5 µm) are able to efficiently deposit onto the hair from detersive compositions containing such copolymers. The ability of smaller particle size silicones (e.g., average particle size <5 µm) to efficiently deposit onto hair from such crosslinked acrylic copolymer containing compositions is severely reduced. Without wishing to be bound by theory, it is surmised that crosslinked acrylic copolymers prepared from methacrylic acid and a C₁-C₄ alkyl acrylate are much more efficient in the stabilization of smaller particle sized silicone emulsions such that these emulsions are difficult to break during the shampooing process, resulting in much of the silicone being rinsed away. This deleterious effect becomes more pronounced as the amount of the copolymer in the formulation is increased. In other words, silicone deposition is inversely proportional to the amount of the acrylic stabilizer thickener present. Consequently, less than an optimal amount of silicone conditioning agent is released to the hair and the conditioning benefit is diminished.

U.S. Pat. No. 7,504,094 proposes to solve the problem of small particle size silicone deposition onto hair by providing a combination of a detersive surfactant, a thickener selected from a crosslinked acrylic copolymer prepared from methacrylic acid and a C₁-C₆ alkyl acrylate, and a microemulsion of a particular amino functionalized (aminosilicone) having a particle size ranging from 0.005 to 0.06 µm in a cosmetically acceptable medium. While small particle sized silicone microemulsions are disclosed, the resolution of the small particle size silicone deposition problem is restricted to the selection of a specific amino functionalized silicone.

The foregoing crosslinked acrylic copolymers are pH dependent in that an increase in the viscosity of the compositions in which they are dissolved or dispersed only occurs upon the neutralization of the carboxylic acid moieties on the polymer backbone with an alkaline material. Upon suitable neutralization (pH values ranging from 5.5 to about 7.5), the polymers swell significantly to form a randomly close-packed (RCP) jammed network of swollen cross-linked particles imparting the desired rheological profile.
JP 2008 150359 discloses two-in-one hair shampoo compositions containing an anionic surface active agent, an amphiphilic surfactant, a water-soluble copolymer of vinyl pyrrolidone (VP) and vinyl acetate (VA), wherein the monomer ratio is the range of VP(mo))/VA(mo))=60/40-99/1, and a silicone compound. It is taught that the specific type of polymer is added to make the silicone remain on the hair.

While a variety of polymeric materials have been included in two-in-one shampoo compositions for purposes of thickening, pearlescence, and stabilization and deposition of silicone conditioning agents, there remains a need for a polymer thickener system that provides the desired rheological properties (e.g., thickening, shear thinning, flow and suspension of gaseous and particulate materials), stabilization and deposition of silicone conditioning agents regardless of silicone type and particle size across a broad pH range.

While a certain rheology modifier may thicken or enhance the viscosity of a composition in which it is included, it does not necessarily have desirable yield stress properties. A desirable yield stress property is critical to achieving certain physical and aesthetic characteristics in a liquid medium, such as the indefinite suspension of particles, insoluble liquid droplets, or the stabilization of gas bubbles within a liquid medium. Particles dispersed in a liquid medium will remain suspended if the yield stress (yield value) of the medium is sufficient to overcome the effect of gravity or buoyancy on those particles. Insoluble liquid droplets can be prevented from rising and coalescing and gas bubbles can be suspended and uniformly distributed in a liquid medium using yield value as a formulating tool. A yield stress polymer is used generally to adjust or modify the rheological properties of aqueous compositions. Such properties include, without limitation, viscosity improvement, flow rate improvement, stability to viscosity change over time, and the ability to suspend particles for indefinite periods of time.

Accordingly, there is a need for a yield stress fluid that is not pH dependent and that can be tailored to create a desired yield stress in the cleansing composition in which it is incorporated, and which mitigates the loss of silicone deposited by the cleansing composition.

### SUMMARY OF THE INVENTION

It has been discovered that silicone containing cleansing compositions possessing excellent yield stress and detersive properties are obtained by incorporating at least one nonionic amphiphilic polymer into the formulation to provide stable silicone containing cleansing compositions without interfering with the deposition of the silicone material onto keratinous substrates.
In particular, the present invention relates to the following embodiments:
1. A personal care composition comprising:
   A) at least one detersive surfactant selected from anionic, amphoteric, cationic, or nonionic surfactant;
   B) at least one silicone conditioning agent
   C) water; and
   D) a nonionic, amphiphilic polymer prepared from a polymerizable monomer mixture comprising:
      a) from 55 to 95 wt.%, from 60 to 90 wt.% in another aspect, from 65 to 85 wt.% in still another aspect, and from 70 to 80 wt.% in a further aspect, of at least one vinyl amide monomer (based on the weight of the total monomers present) ;
      b) from 5 to 45 wt.%, from 10 to 40 wt.% in another aspect, from 15 to 35 wt.% in still another aspect, and from 20 to 30 wt.% in a further aspect of at least one vinyl ester of an aliphatic carboxylic acid containing an acyl moiety having 2 to 22 carbon atoms (based on the weight of the total monomers present);
      c) from 0.01 to 1 wt.%, from 0.01 to 0.75 wt.% in another aspect, from 0.1 to 0.5 wt.% in still another aspect, and from 0.15 to 0.3 wt.% in a further aspect of at least one polyunsaturated crosslinking monomer containing at least two polymerizable ethylenically unsaturated moieties (based on the total dry weight of the polymer) selected from polyallyl ethers of trimethylolpropane, polyallyl ethers of pentaerythritol, polyallyl ethers of sucrose having from 2 to 8 allyl groups per molecule, and mixtures thereof;
      d) from 0 to 10 wt.%, from 0.1 to 5 wt.% in another aspect, from 0.5 to 3 wt.% in still another aspect, and from 0.75 to 1 wt.% in a further aspect, of at least one C₁-C₂₂ alkyl (meth)acrylate (based on the weight of the total monomers present);
      e) from 0 to 10 wt.%, and from 0.5, 1, 2, or 3 to 5 wt.% in a further aspect of an alkoxylated associative monomer (based on the weight of the total monomers present);
      f) from 0 to 10 wt.%, and from 0.5, 1, 2, or 3 to 5 wt.% in a further aspect of an alkoxylated semi-hydrophobic monomer (based on the weight of the total monomers present); and
      g) from 0 or 0.5, 1, 2 or 3 to 5 wt.% of at least one vinyl ester of an aliphatic carboxylic acid containing an acyl moiety having 2 to 22 carbon atoms (based on the weight of the total monomers present) other than vinyl acetate.
2. A personal care composition of embodiment 1, wherein said monomer composition further comprises from 0.01 to 15 wt.% of at least one copolymerizable monomer selected from:
   h) at least one C₁-C₅ hydroxyalkyl (meth)acrylate;
   i) at least one (meth)acrylamide selected from (meth)acrylamide, N-(C₁-C₅)alkyl (meth)acrylamide, N,N-di(C₁-C₅)alkyl (meth)acrylamide, N-(C₁-C₅)alkylamino(C₁-C₅)alkyl(meth)acrylamide or N,N-di(C₁-C₅)alkylamino(C₁-C₅)alkyl(meth)acrylamide;
   j) at least one alpha-olefinic monomer; and mixtures thereof.
3. A personal care composition of any of the preceding embodiments, wherein said at least one vinyl amide is selected from N-vinylformamide, N-methyl-N-vinylformamide, N-(hydroxymethyl)-N-vinylformamide, N-vinylacetamide, N-vinylmethylacetamide, N-(hydroxymethyl)-N-vinylacetamide, and mixtures thereof; and said N-vinyl lactam is selected from N-vinyl-2-pyrrolidinone, N-(1-methyl vinyl) pyrrolidinone, N-vinyl-2-piperidone, N-vinyl-2-caprolactam, N-vinyl-5-methyl pyrrolidinone, N-vinyl-3,3-dimethyl pyrrolidinone, N-vinyl-5-ethyl pyrrolidinone and N-vinyl-6-methyl piperidone, and mixtures thereof.
4. A personal care composition of any of the preceding embodiments, wherein said at least one vinyl ester of an aliphatic carboxylic acid containing an acyl moiety having 2 to 22 carbon atoms is selected from vinyl acetate, vinyl propionate, vinyl butyrate, vinyl isobutyrate, vinyl valerate, vinyl hexanoate, vinyl 2-methylhexanate, vinyl 2-ethylhexanoate, vinyl iso-octanoate, vinyl nonanoate, vinyl neodecanoate, vinyl decanoate, vinyl versatate, vinyl laurate, vinyl palmitate, vinyl stearate, and mixtures thereof.
5. A personal care composition of any of the preceding embodiments, wherein the at least one polyunsaturated crosslinking monomer is selected from a monomer having an average of 2 crosslinkable unsaturated functional groups, an average of 3 crosslinkable unsaturated functional groups, and mixtures thereof.
6. A personal care composition of any of the preceding embodiments, wherein said C₁-C₅ hydroxyalkyl (meth)acrylate monomer is selected from 2-hydroxyethyl(meth)acrylate, 3-hydroxypropyl(meth)acrylate, 4-hydroxybutyl(meth)acrylate, and mixtures thereof.
7. A personal care composition of any of the preceding embodiments, wherein said at least one C₁-C₂₂ alkyl (meth)acrylate is selected from methyl (meth)acrylate, butyl (meth)acrylate, sec-butyl (meth)acrylate, iso-butyl (meth)acrylate, hexyl (meth)acrylate), heptyl (meth)acryiate, octyl (meth)acrylate, 2-ethylhexyl (meth)acrylate, decyl (meth)acrylate, isodecyl (meth)acrylate, lauryl (meth)acrylate, tetradecyl (meth)acrylate, hexadecyl (meth)acrylate, stearyl (meth)acrylate, behenyl (meth)acrylate, or mixtures thereof.
8. A personal care composition of any of the preceding embodiments, wherein said associative monomer comprises (i) a polymerizable ethylenically unsaturated end group portion, (ii) a polyoxyalkylene mid-section portion, and (iii) a hydrophobic end group portion containing 7 to 30 carbon atoms.
9. A personal care composition of any of the preceding embodiments, wherein said associative monomer is represented by formulas VII and/or VIIA: 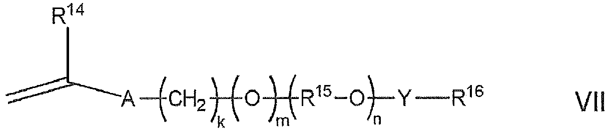 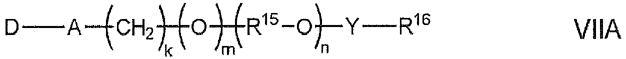
   wherein R¹⁴ is hydrogen or methyl; A is -CH₂C(O)O-, -C(O)O-, -O-, -CH₂O- ,-NHC(O)NH-, -C(O)NH-,
   -Ar-(CE₂)_{z}-NHC(O)O-, -Ar-(CE₂)_{z}-NHC(O)NH-, or -CH₂CH₂NHC(C)-; a divalent alkylene radical containing 1 to 5 carbon atoms; Ar is a divalent arylene (e.g., phenylene); E is H or methyl; z is 0 or 1; k is an integer ranging from 0 to 30, and m is 0 or 1, with the proviso that when k is 0, m is 0, and when k is in the range of 1 to 30, m is 1; D represents a vinyl or an allyl moiety; (R¹⁵-O)ₙ is a polyoxyalkylene moiety, which can be a homopolymer, a random copolymer, or a block copolymer of C₂-C₄ oxyalkylene units, R¹⁵ is a divalent alkylene moiety selected from C₂H₄, C₃H₆, or C₄H₈, and combinations thereof; and n is an integer in the range of 2 to 150 in one aspect, from 10 to 120 in another aspect, and from 15 to 60 in a further aspect; Y is -R¹⁵O-, -R¹⁵NH-, -C(O)-, -C(O)NH-, -R¹⁵NHC(O)NH-, or -C(O)NHC(O)-; R¹⁶ is a substituted or unsubstituted alkyl selected from a C₈-C₃₀ linear alkyl, a C₈-C₃₀ branched alkyl, a C₇-C₃₀ carbocyclic alkyl, a C₂-C₃₀ alkyl-substituted phenyl, an araalkyl substituted phenyl, and an aryl-substituted C₂-C₃₀ alkyl; wherein the R¹⁶ alkyl group, carbocyclic alkyl group, aryl group, phenyl group optionally comprises one or more substituents selected from the group selected from a methyl group, hydroxyl group, an alkoxyl group, benzyl group styryl group, and a halogen group.
10. A personal care composition of any of the preceding embodiments, wherein said associative monomer is represented by formula VIIB: 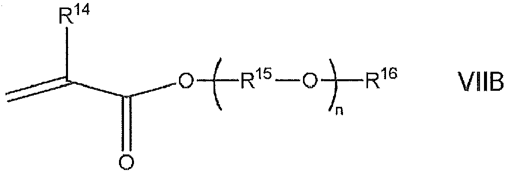 wherein R¹⁴ is hydrogen or methyl; R¹⁵ is a divalent alkylene moiety independently selected from C₂H₄, C₃H₆, and C₄H₈, and n represents an integer ranging from 10 to 60, (R¹⁵-O) can be arranged in a random or a block configuration; R¹⁶ is a substituted or unsubstituted alkyl selected from a C₈-C₃₀ linear alkyl, a C₈-C₃₀ branched alkyl, a C₇-C₃₀ carbocyclic alkyl, a C₂-C₃₀ alkyl-substituted phenyl, an araalkyl substituted phenyl, and an aryl-substituted C₂-C₃₀ alkyl, wherein the R¹⁶ alkyl group, aryl group, phenyl group optionally comprises one or more substituents selected from the group consisting of a hydroxyl group, an alkoxyl group, benzyl group styryl group, and a halogen group.
11. A personal care composition of any of the preceding embodiments, wherein said semi-hydrophobic monomer comprises (i) a polymerizable ethylenically unsaturated end group portion, (ii) a polyoxyalkylene mid-section portion, and (iii) an end group portion selected from hydrogen or an alkyl group containing 1 to 4 carbon atoms.
12. A personal care composition of any of the preceding embodiments, wherein said semi-hydrophobic monomer is selected from at least one monomer represented by formulas VIII and IX: 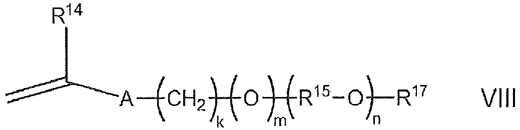 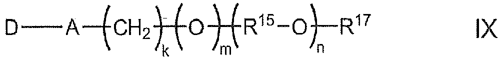
   wherein R¹⁴ is hydrogen or methyl; A is -CH₂C(O)O-, -C(O)O-, -O-, -CH₂O- ,-NHC(O)NH-, -C(O)NH-,
   -Ar-(CE₂)_{z}-NHC(O)O-, -Ar-(CE₂)_{z}-NHC(O)NH-, or-CH₂CH₂NHC(O)-; Ar is a divalent arylene (e.g., phenylene); E is H or methyl; z is 0 or 1; k is an integer ranging from 0 to 30, and m is 0 or 1, with the proviso that when k is 0, m is 0, and when k is in the range of 1 to 30, m is 1; (R¹⁵-O)ₙ is a polyoxyalkylene moiety, which can be a homopolymer, a random copolymer, or a block copolymer of C₂-C₄ oxyalkylene units, R¹⁵ is a divalent alkylene moiety selected from C₂H₄, C₃H₆, or C₄H₈, and combinations thereof; and n is an integer in the range of 2 to 150 in one aspect, from 5 to 120 in another aspect, from 10 to 60 in a further aspect, and from 15 to 30 in a still further aspect; R¹⁷ is selected from hydrogen and a linear or branched C₁-C₄ alkyl group; and D represents a vinyl or an allyl moiety.
13. A personal care composition of any of the preceding embodiments, wherein said semi-hydrophobic monomer is selected from at least one monomer represented by formulas VIIIA and VIIIB:

   CH₂=C(R¹⁴)C(O)O-(C₂H₄O)ₐ(C₃H₆O)_{b}-H VIIIA

   CH₂=C(R¹⁴)C(O)O-(C₂H₄0)ₐ(C₃HₛO)_{b}-CH₃ VIIIB

   wherein R¹⁴ is hydrogen or methyl, and "a" is an integer ranging from 0 or 2 to 120 in one aspect, from 5 to 45 in another aspect, and from 10 to 25 in a further aspect, and "b" is an integer ranging from 0 or 2 to 120 in one aspect, from 5 to 45 in another aspect, and from 10 to 25 in a further aspect, subject to the proviso that "a" and "b" cannot be 0 at the same time.
14. A personal care composition of any of the preceding embodiments, wherein said at least one (meth)acrylamide is selected from N-methyl(meth)acrylamide, N-ethyl(meth)acrylamide, N-propyl(meth)acrylamide, N-isopropyl(meth)acrylamide, N-tert-butyl(meth)acrylamide, N-tert-octyl(meth)acrylamide, N-(2-hydroxyethyl)(meth)acrylamide, N-(3-hydroxypropyl)(meth)acrylamide; N,N-dimethyl(meth)acrylamide, N,N-diethyl(meth)acrylamide, N,N-(di-2-hydroxyethyl)(meth)acrylamide, N,N-(di-3-hydroxypropyl)(meth)acrylamide, N-methyl,N-ethyl(meth)acrylamide; N,N-dimethylaminoethyl(meth)acrylamide, N,N-diethylaminoethyl(meth)acrylamide, N,N-dimethylaminopropyl(meth)acrylamide; and mixtures thereof.
15. A personal care composition of any of the preceding embodiments, wherein said at least one associative monomer is selected from lauryl polyethoxylated (meth)acrylate (LEM), cetyl polyethoxylated (meth)acrylate (CEM), cetearyl polyethoxylated (meth)acrylate (CSEM), stearyl polyethoxylated (meth)acrylate, arachidyl polyethoxylated (meth)acrylate, behenyl polyethoxylated (meth)acrylate (BEM), cerotyl polyethoxylated (meth)acrylate, montanyl polyethoxylated (meth)acrylate, melissyl polyethoxylated (meth)acrylate, phenyl polyethoxylated (meth)acrylate, nonylphenyl polyethoxylated (meth)acrylate, ω-tristyrylphenyl polyoxyethylene (meth)acrylate, where the polyethoxylated portion of the monomer contains from 2 to 150 ethylene oxide units in one aspect, from 5 to 120 in another aspect, from 10 to 60 in a further aspect, and from 15 to 30 in a still further aspect; octyloxy polyethyleneglycol polypropyleneglycol (meth)acrylate, phenoxy polyethylene glycol polypropylene glycol (meth)acrylate, and nonylphenoxy polyethylene glycol polypropylene glycol (meth)acrylate, where the polyethoxylated and/or the polypropoxylated portion of the monomer independently contain 0 or 2 to 120 in one aspect, from 5 to 45 in another aspect, and from 10 to 25 in a further aspect; and mixtures thereof.
16. A personal care composition of any of the preceding embodiments, wherein said at least one semi-hydrophobic monomer is selected from polyethyleneglycol (meth)acrylate, polypropyleneglycol (meth)acrylate, polyethyleneglycol polypropylene glycol methacrylate or methoxypolyethyleneglycol (meth)acrylate, where the polyethoxylated and/or the polypropoxylated porton of the monomer independently contain 0 or 2 to 120 in one aspect, from 5 to 45 in another aspect, and from 10 to 25 in a further aspect; and mixtures thereof.
17. A personal care composition of any of the preceding embodiments, wherein said at least one semi-hydrophobic monomer is selected from a compound having the formula: CH₂=CH-O(CH₂)₄O(C₃H₆O)₄(C₂H₄O)₁₀H; CH₂=CH-O(CH₂)₄O(C₃H₆O)₄(C₂H₄O)₂₀H; CH₂=CH-O(CH₂)₄O(C₃H₆O)₄(C₂H₄O)₃₀H; CH₂=CHCH₂O(C₃H₆O)₄(C₂H₄O)₁₀H; CH₂=CHCH₂O(C₃H₆O)₄(C₂H₄O)₂₀H; CH₂=CHCH₂O(C₃H₆O)₄(C₂H₄O)₃₀H; and CH₂=CHCH₂O(C₃H₆O)₅(C₂H₄O)₅H.
18. A personal care composition of any of the preceding embodiments, wherein said at least one alpha-olefinic monomer is selected from ethylene, propylene, 1-butene, iso-butylene, 1-hexene, 1-heptene, 4-methyl-1-pentene, styrene, alpha-methyl styrene, and mixtures thereof.
19. A personal care composition of any of the preceding embodiments, wherein said nonionic, amphiphilic polymer comprises repeating units prepared from a monomer mixture comprising:
   a) from 55 to 95 wt.%, from 60 to 90 wt.% in another aspect, from 65 to 85 wt.% in still another aspect, and from 70 to 80 wt.% in a further aspect, of N-vinyl pyrrolidone (based on the weight of the total monomers present);
   b) from 5 to 45 wt.%, from 10 to 40 wt.% in another aspect, from 15 to 35 wt.% in still another aspect, and from 20 to 30 wt.% in a further aspect of vinyl acetate (based on the weight of the total monomers present);
   c) from 0.01 to 1 wt.%, from 0.01 to 0.75 wt.% in another aspect, from 0.1 to 0.5 wt.% in still another aspect, and from 0.15 to 0.3 wt.% in a further aspect of at least one polyunsaturated crosslinking monomer containing at least two polymerizable ethylenically unsaturated moieties (based on the total dry wt. of the polymer);
   d) from 0 to 10 wt.%, from 0.1 to 5 wt.% in another aspect, from 0.5 to 3 wt.% in still another aspect, and from 0.75 to 1 wt.% in a further aspect (based on the weight of the total monomers present) of at least one C₁-C₂₂ alkyl (meth)acrylate selected from methyl (meth)acrylate, butyl (meth)acrylate, sec-butyl (meth)acrylate, iso-butyl (meth)acrylate, hexyl (meth)acrylate), heptyl (meth)acrylate, octyl (meth)acrylate, 2-ethylhexyl (meth)acrylate, decyl (meth)acrylate, isodecyl (meth)acrylate, lauryl (meth)acrylate, tetradecyl (meth)acrylate, hexadecyl (meth)acrylate, stearyl (meth)acrylate, or behenyl (meth)acrylate; and
   e) from 0 to 10 wt.%, and from 0.5, 1, 2, or 3 to 5 wt.% in a further aspect (based on the weight of the total monomers present) of an alkoxylated associative monomer selected from lauryl polyethoxylated (meth)acrylate, cetyl polyethoxylated (meth)acrylate, cetearyl polyethoxylated (meth)acrylate, stearyl polyethoxylated (meth)acrylate, arachidyl polyethoxylated (meth)acrylate, behenyl polyethoxylated (meth)acrylate, cerotyl polyethoxylated (meth)acrylate, montanyl polyethoxylated (meth)acrylate, melissyl polyethoxylated (meth)acrylate, phenyl polyethoxylated (meth)acrylate, nonylphenyl polyethoxylated (meth)acrylate, ω-tristyrylphenyl polyoxyethylene methacrylate, where the polyethoxylated portion of the monomer contains from 2 to 150 ethylene oxide units in one aspect, from 5 to 120 in another aspect, from 10 to 60 in a further aspect, and from 15 to 30 in a still further aspect.
20. A personal care composition of any of the preceding embodiments, wherein said nonionic, amphiphilic polymer comprises repeating units prepared from a monomer mixture further comprising from 0.5, 1, 2 or 3 to 5 wt.% (based on the weight of the total monomers present) of a vinyl ester of an aliphatic carboxylic acid containing an acyl moiety having 2 to 22 carbon atoms other than vinyl acetate selected from vinyl propionate, vinyl butyrate, vinyl isobutyrate, vinyl valerate, vinyl hexanoate, vinyl 2-methylhexanate, vinyl 2-ethylhexanoate, vinyl iso-octanoate, vinyl nonanoate, vinyl neodecanoate, vinyl decanoate, vinyl versatate, vinyl laurate, vinyl palmitate, and vinyl stearate.
21. A personal care composition of any of the preceding embodiments, wherein said-at least one C₁-C₂₂ alkyl (meth)acrylate is selected from decyl (meth)acrylate, isodecyl (meth)acrylate, lauryl (meth)acrylate, tetradecyl (meth)acrylate, hexadecyl (meth)acrylate, stearyl (meth)acrylate, behenyl (meth)acrylate, and mixtures thereof.
22. A personal care composition of any of the preceding embodiments, wherein said alkoxylated associative monomer is selected from lauryl polyethoxylated (meth)acrylate, cetyl polyethoxylated (meth)acrylate, cetearyl polyethoxylated (meth)acrylate, stearyl polyethoxylated (meth)acrylate, arachidyl polyethoxylated (meth)acrylate, behenyl polyethoxylated (meth)acrylate, and mixtures thereof.
23. A personal care composition of any of the preceding embodiments, wherein said C₁-C₂₂ alkyl (meth)acrylate is stearyl methacrylate.
24. A personal care composition of any of the preceding embodiments, wherein said alkoxylated associative monomer is behenyl polyethoxylated methacrylate.
25. A personal care composition of any of the preceding embodiments, wherein said the at least one crosslinking monomer is selected from trimethylolpropane tri(meth)acrylate, trimethylolethane tri(meth)acrylate, tetramethylolmethane tri(meth)acrylate, and mixtures thereof.
26. A personal care composition of any of the preceding embodiments, wherein said at least one crosslinking monomer is selected from pentaerythritol diallyl ether, pentaerythritol triallyl ether, pentaerythritol tetraallyl ether; and mixtures thereof.
27. A personal care composition of any of the preceding embodiments, wherein said at least one surfactant is selected from an anionic surfactant, an amphoteric surfactant, and mixtures thereof.
28. A personal care composition of any of the preceding embodiments, wherein the at least one anionic surfactant is ethoxylated.
29. A personal care composition of any of the preceding embodiments, wherein the at least one anionic surfactant contains an average of 1 to 3 moles of ethoxylation.
30. A personal care composition of any of the preceding embodiments, wherein the at least one anionic surfactant contains an average of 1 to 2 moles of ethoxylation.
31. A personal care composition of any of the preceding embodiments, wherein the at least one anionic surfactant is selected from sodium dodecyl sulfate, ammonium dodecyl sulfate, sodium lauryl sulfate, sodium trideceth sulfate, ammonium lauryl sulfate, sodium laureth sulfate, ammonium laureth sulfate or mixtures thereof.
32. A personal care composition of any of the preceding embodiments, wherein the at least one amphoteric surfactant is selected from to amino acid surfactants, betaines, sultaines, alkyl amphocarboxylates, and mixtures thereof.
33. A personal care composition of any of the preceding embodiments, wherein the at least one amphoteric surfactant is cocamidopropyl betaine.
34. A personal care composition of any of the preceding embodiments, wherein the concentration of the at least one surfactant is less than 25 wt.% (active), based on the weight of the total composition.
35. A personal care composition of any of the preceding embodiments, wherein the concentration of surfactant ranges from 6 to 20 wt.% (active), based on the weight of the total composition.
36. A personal care composition of any of the preceding embodiments, wherein the ratio of anionic surfactant to amphoteric surfactant (active) is 10:1 to 2:1 in one aspect, and 9:1, 8:1, 7:1 6:1, 5:1, 4.5:1, 4:1, or 3:1 in another aspect.
37. A personal care composition of any of the preceding embodiments, wherein the amount of nonionic, amphiphilic polymer solids in said composition ranges from 1 to 3 wt.%, based on the weight of the total composition.
38. A personal care composition of any of the preceding embodiments, wherein said monomer mixture further comprises a steric stabilizer.
39. A personal care composition of any of the preceding embodiments, wherein said monomer mixture further comprises a steric stabilizer selected from a copolymer of N-vinyl pyrrolidone/stearyl methacrylate/butyl acrylate, the ester of the reaction product of a C₂₀ to C₂₄ alkyl substituted succinic anhydride and a polyol selected from glycerin and/or a polyglycerol containing 2 to 6 glycerin units, and mixtures thereof.
40. A personal care composition of any of the preceding embodiments, wherein said nonionic, amphiphilic polymer is linear.
41. A personal care composition of any of the preceding embodiments, wherein said nonionic, amphiphilic polymer is crosslinked.
42. A personal care composition of any of the preceding embodiments, wherein said silicone conditioning agent is non-volatile silicone selected from a silicone oil, silicone gum, silicone resin and mixtures thereof.
43. A personal care composition of embodiment 42 further comprising a volatile silicone.
44. A personal care composition of any of the preceding embodiments, wherein said silicone oil is selected from a compound represented by the formula: 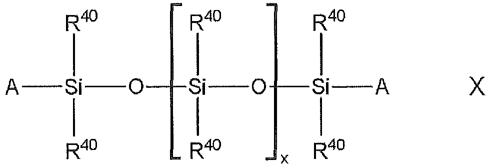 wherein A independently represents hydroxy, methyl, methoxy, ethoxy, propoxy, and phenoxy; R⁴⁰ independently represents methyl, ethyl, propyl, phenyl, methylphenyl, phenylmethyl; and x is an integer ranging from 7 to 8000 in one aspect, from 50 to 5000 in another aspect, form 100 to 3000 in still another aspect, and from 200 to 1000 in a further aspect.
45. A personal care composition of any of the preceding embodiments, wherein said silicone conditioning agent is selected from polydimethylsiloxanes (dimethicones), polydiethylsiloxanes, polydimethyl siloxanes having terminal hydroxyl groups (dimethiconols), polymethylphenylsiloxanes, phenylmethylsiloxanes, and mixtures thereof.
46. A personal care composition of any of embodiments 1 to 45, wherein said silicone conditioning agent is selected from an amino functional polydimethylsiloxane (amodimethicone).
47. A personal care composition of embodiment 46, wherein said amino functional silicone is selected from a compound represented by the formula: 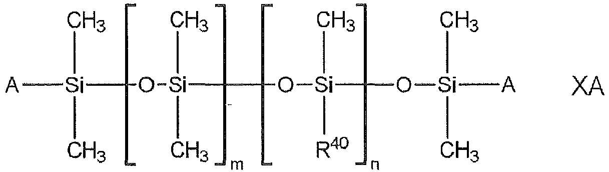 wherein wherein A independently represents hydroxy, methyl, methoxy, ethoxy, propoxy, and phenoxy; and R⁴⁰ is selected from:

   -R⁴¹-N(R⁴²)CH₂CH₂N(R⁴²)₂;

   -R⁴¹-N(R⁴²)₂;

   -R⁴¹-N⁺(R⁴²)₃CA⁻;

   and

   -R⁴¹-N(R⁴²)CH₂CH₂N(R⁴²)H₂CA⁻

   wherein R⁴¹ is a linear or branched, hydroxyl substituted or unsubstituted alkylene or alkylene ether moiety containing 2 to 10 carbon atoms; R⁴² is hydrogen, C₁-C₂₀ alkyl (e.g, methyl), phenyl or benzyl; CA⁻ is a halide ion selected from chlorine, bromine, iodine and fluorine; and the sum of m+n ranges from 7 to 1000 in one aspect, from 50 to 250 in another aspect, and from 100 to 200 in another aspect, subject to the proviso that m or n is not 0.
48. A personal care composition of any of the preceding embodiments, wherein said silicone conditioning agent is present in an amount ranging from 0.01 to 20 wt.% in one aspect, from 0.05 to 15 wt.% in another aspect, from 0.1% to 10 wt.% in still another aspect, and from 1 to 5 wt.% in a further aspect, based on the weight of the total composition.
49. A personal care composition of any of the preceding embodiments, wherein said silicone conditioning agent has a particle size ranging from 0.003 to 500 µm in one aspect, from 0.05 to 200 µm in a second aspect, from 0.25 to 200 µm in a third aspect, from 0.5 to 150 µm in fourth aspect, from 1 to 100 µm in a fifth aspect, from 5 to 80 µm in a six aspect, from 10 to 60 µm in an seventh aspect, and from 20 to 50 µm in a eighth aspect.
50. A personal care composition of any of the preceding embodiments, wherein said silicone conditioning agent is in the form of emulsion droplets.
51. A personal care composition of any of the previous embodiments, further comprising an auxiliary conditioning agent selected from a hydrocarbon oil, an ester oil, and combinations thereof.
52. A personal care composition of any of the previous embodiments, further comprising a cationic polymer.
53. A personal care composition of any of the preceding embodiments, further comprising a pearlizing agent.
54. A personal care composition of embodiment 39, wherein said pearlizing agent is selected from mica, metal oxide coated mica, silica coated mica, bismuth oxychloride coated mica, bismuth oxychloride, myristyl myristate, glass, metal oxide coated glass, various aluminum and magnesium salts, guanine, fish scales, glitter (polyester or metallic), and mixtures thereof.
55. A personal care composition of embodiment 39, wherein said pearlizing agent is selected from monoesters and/or diesters of ethylene glycol, propylene glycol, diethylene glycol, dipropylene glycol, triethylene glycol and tetraethylene glycol with fatty acids containing from 6 to 22 carbon atoms.
56. A personal care composition of embodiment 55, wherein said pearlizing agent is selected from ethylene glycol monostearate (EGMS), ethylene glycol distearate (EGDS), polyethylene glycol monostearate (PGMS), polyethyleneglycol distearate (PGDS), and mixtures thereof.
57. A personal care composition of any of the preceding embodiments, further comprising a particulate material selected from pigments, exfoliating agents, anti-dandruff agents, clay, swellable clay, laponite, gas bubbles, liposomes, UV absorbers, microsponges, cosmetic beads and flakes.
58. A personal care composition of any of the preceding embodiments, wherein said nonionic, amphiphilic polymer is linear and said yield stress of said composition is 0 Pa.
59. A personal care composition of any of the preceding embodiments, wherein said nonionic, amphiphilic polymer is crosslinked and said yield stress of said composition is ≥ 0 Pa in one aspect, at least 0.1 Pa in another aspect, from 0.1 to 20 Pa in still another aspect, from 0.5 Pa to 10 Pa in a further aspect, from 1 to 3 Pa in still a further aspect, and from 1.5 to 3.5 in an additional aspect.
60. A personal care composition of any of the preceding embodiments, wherein said nonionic, amphiphilic polymer is crosslinked and wherein said composition is able to suspend beads of a size between 0.5 and 1.5 mm for at least one month at 23°C wherein the difference in specific gravity between the bead material and water is between +/- 0.01 and 0.5.
61. A personal care composition of any of the preceding embodiments, wherein said nonionic, amphiphilic polymer is crosslinked and wherein said composition is able to suspend microcapsules of a size between 0.5 and 300 µm for at least one month at 23°C wherein the difference in specific gravity between the microcapsule beads and water is between +/- 0.2 and 0.5.
62. A personal care composition of any of the preceding embodiments, wherein said nonionic, amphiphilic polymer is crosslinked and wherein the yield stress of said composition is substantially independent of pH in the pH range of from 2 to 10.
63. A personal care composition of any of the preceding embodiments, wherein said nonionic, amphiphilic polymer is crosslinked and wherein the yield stress of said composition is substantially independent of pH in the pH range of from 3 to 14.
64. A personal care composition of any of the preceding embodiments, wherein said personal care composition is selected from shampoos, baby shampoos, body washes, shower gels, liquid hand soaps, liquid dishwashing detergents, pet cleansing product, moist cleansing wipes, or facial cleansers.
65. A method for mitigating the loss of silicone deposited from a silicone containing conditioning shampoo composition, said method comprising contacting a keratinous substrate with a detersive composition comprising the composition of any of the previous embodiments.
66. A method for mitigating the loss of silicone deposited from a silicone containing conditioning shampoo composition onto a keratinous substrate, said method comprising including therein from 1 to 5 wt.% of a nonionic, amphiphilic dispersion polymer, wherein said polymer is prepared from a monomer mixture comprising:
   a) from 55 to 95 wt.%, from 60 to 90 wt.% in another aspect, from 65 to 85 wt.% in still another aspect, and from 70 to 80 wt.% in a further aspect, of N-vinyl pyrrolidone (based on the weight of the total monomers present);
   b) from 5 to 45 wt.%, from 10 to 40 wt.% in another aspect, from 15 to 35 wt.% in still another aspect, and from 20 to 30 wt.% in a further aspect of vinyl acetate (based on the weight of the total monomers present);
   c) from 0.01 to 1 wt.%, from 0.01 to 0.75 wt.% in another aspect, from 0.1 to 0.5 wt.% in still another aspect, and from 0.15 to 0.3 wt.% in a further aspect of at least one polyunsaturated crosslinking monomer containing at least two polymerizable ethylenically unsaturated moieties (based on the total dry wt. of the polymer) selected from polyallyl ethers of trimethylolpropane, polyallyl ethers of pentaerythritol, polyallyl ethers of sucrose having from 2 to 8 allyl groups per molecule, and mixtures thereof;
   d) from 0 to 10 wt.%, from 0.1 to 5 wt.% in another aspect, from 0.5 to 3 wt.% in still another aspect, and from 0.75 to 1 wt.% in a further aspect (based on the weight of the total monomers present) of at least one C₁-C₂₂ alkyl (meth)acrylate selected from methyl (meth)acrylate, butyl (meth)acrylate, sec-butyl (meth)acrylate, iso-butyl (meth)acrylate, hexyl (meth)acrylate), heptyl (meth)acrylate, octyl (meth)acrylate, 2-ethylhexyl (meth)acrylate, decyl (meth)acrylate, isodecyl (meth)acrylate, lauryl (meth)acrylate, tetradecyl (meth)acrylate, hexadecyl (meth)acrylate, stearyl (meth)acrylate, or behenyl (meth)acrylate; and
   e) from 0 to 10 wt.%, and from 0.5, 1, 2, or 3 to 5 wt.% in a further aspect (based on the weight of the total monomers present) of an alkoxylated associative monomer selected from lauryl polyethoxylated (meth)acrylate, cetyl polyethoxylated (meth)acrylate, cetearyl polyethoxylated (meth)acrylate, stearyl polyethoxylated (meth)acrylate, arachidyl polyethoxylated (meth)acrylate, behenyl polyethoxylated (meth)acrylate, cerotyl polyethoxylated (meth)acrylate, montanyl polyethoxylated (meth)acrylate, melissyl polyethoxylated (meth)acrylate, phenyl polyethoxylated (meth)acrylate, nonylphenyl polyethoxylated (meth)acrylate, w-tristyrylphenyl polyoxyethylene methacrylate, where the polyethoxylated portion of the monomer contains from 2 to 150 ethylene oxide units in one aspect, from 5 to 120 in another aspect, from 10 to 60 in a further aspect, and from 15 to 30 in a still further aspect;
   f) from 0 to 10 wt.%, and from 0.5, 1, 2, or 3 to 5 wt.% in a further aspect (based on the weight of the total monomers present) of a semi-hydrophobic monomer selected from methoxy polyethyleneglycol methacrylate;
   g) from 0 to 10 wt.%, and 0.5, 1, 2 or 3 to 5 wt.% (based on the weight of the total monomers present) of at least one vinyl ester of an aliphatic carboxylic acid containing an acyl moiety having 2 to 22 carbon atoms other than vinyl acetate selected from vinyl propionate, vinyl butyrate, vinyl isobutyrate, vinyl valerate, vinyl hexanoate, vinyl 2-methylhexanate, vinyl 2-ethylhexanoate, vinyl iso-octanoate, vinyl nonanoate, vinyl neodecanoate, vinyl decanoate, vinyl versatate, vinyl laurate, vinyl palmitate, and vinyl stearate.
67. A method of embodiment 66, wherein said monomer mixture further comprises a steric stabilizer selected from a copolymer of N-vinyl pyrrolidone/stearyl methacrylate/butyl acrylate, the ester of the reaction product of a C₂₀ to C₂₄ alkyl substituted succinic anhydride and a polyol selected from glycerin and/or a polyglycerol containing 2 to 6 glycerin units, and mixtures thereof.
68. A method of embodiment 66, wherein said monomer mixture comprises:
   a) 55 to 95 wt.%, of N-vinyl pyrrolidone (based on the weight of the total monomers present);
   b) from 5 to 45 wt.%, in a further aspect of vinyl acetate (based on the weight of the total monomers present);
   c) from 0.1 to 1 wt.% of at least one polyunsaturated crosslinking monomer containing at least two polymerizable ethylenically unsaturated moieties (based on the total dry wt. of the polymer);
   d) from 0 or 1 to 5 wt.% (based on the weight of the total monomers present) of at least one monomer selected from decyl (meth)acrylate, isodecyl (meth)acrylate, lauryl (meth)acrylate, tetradecyl (meth)acrylate, hexadecyl (meth)acrylate, stearyl (meth)acrylate, or behenyl (meth)acrylate;
   e) from 0 or 1 to 5 wt.% (based on the weight of the total monomers present) of an alkoxylated associative monomer selected from lauryl polyethoxylated (meth)acrylate, cetyl polyethoxylated (meth)acrylate, cetearyl polyethoxylated (meth)acrylate, stearyl polyethoxylated (meth)acrylate, behenyl polyethoxylated (meth)acrylate, where the polyethoxylated portion of the monomer contains from 10 to 60 ethylene oxide units;
   f) from 0 or 1 to 5 wt.% (based on the weight of the total monomers present) of a semi-hydrophobic monomer selected from methoxy polyethyleneglycol methacrylate; and
   g) from 0 or 1 to 5 wt.%, (based on the weight of the total monomers present) of at least one vinyl ester of an aliphatic carboxylic acid containing an acyl moiety having 2 to 22 carbon atoms other than vinyl acetate selected from vinyl propionate, vinyl butyrate, vinyl isobutyrate, vinyl valerate, vinyl hexanoate, vinyl 2-methylhexanate, vinyl 2-ethylhexanoate, vinyl iso-octanoate, vinyl nonanoate, vinyl neodecanoate, vinyl decanoate, vinyl versatate, vinyl laurate, vinyl palmitate, and vinyl stearate.

In one aspect, embodiments of the present invention relate to stable cleansing compositions comprising a silicone conditioning agent.

In another aspect, embodiments of the invention relate to a stable cleansing composition comprising a silicone conditioning agent and a nonionic amphiphilic polymer which mitigates the loss of silicone deposition on keratinous substrates.

In still another aspect, embodiments of the invention relate to a thickened stable personal care cleansing composition which provides good conditioning properties to keratinous substrates.

In a further aspect, embodiments of the invention relate to a two-in-one shampoo composition comprising a silicone conditioning agent and a nonionic amphiphilic polymer which mitigates the loss of silicone deposition on the hair and scalp.

In a still further aspect, embodiments of the invention relate to a two-in-one shampoo composition comprising a silicone conditioning agent and a nonionic amphiphilic polymer which mitigates the loss of silicone deposition on the hair and scalp.

In an additional aspect, embodiments of the invention relate to a cleansing composition comprising a silicone conditioning agent and a nonionic amphiphilic polymer which mitigates the loss of silicone deposition on the skin, and provides stable suspensions of pearlescent and other insoluble materials to deliver an aesthetic appearance and good shelf appeal.

In a still additional aspect, embodiments of the invention relate to a method of mitigating the loss of silicone deposition on keratinous substrates by providing a stable personal care detersive composition comprising a silicone conditioning agent and a nonionic amphiphilic polymer which does not unduly interfere with the deposit of silicone materials.

In a still further additional aspect, embodiments of the invention relate to a cleansing composition comprising a silicone conditioning agent and a nonionic amphiphilic polymer which mitigates the loss of silicone deposition on the skin, and provides stable suspensions of pearlescent and other insoluble materials to deliver an aesthetic appearance and good shelf appeal over a wide range of pH values. This affords more flexibility in the type of materials that can be formulated into the cleansing composition as well as an extended range of yield stress properties not typically available with other polymeric thickeners.

In another aspect, an embodiment of the invention relates to a composition and method for reducing the loss of silicone deposited onto a keratinous substrate from a thickened cleansing composition comprising at least one surfactant and at least one silicone conditioning agent, the composition and method comprising combining a crosslinked, nonionic amphiphilic polymer with at least one detersive surfactant selected from anionic surfactants, amphoteric surfactants, nonionic surfactants and combinations of two or more thereof, wherein the concentration of the amphiphilic polymer is no more than 5 wt.%, and the at least one surfactant is no more than 30 wt.% (all weight percentages are based on the total weight of the composition), wherein the yield stress of the composition is at least 0.1 Pa with a shear thinning index of less than 0.5 at shear rates between 0.1 and 1 reciprocal seconds, and wherein the yield stress, elastic modulus and optical clarity of the composition are substantially independent of pH ranging from 2 to 14.

In another aspect, an embodiment of the invention relates to a composition and method for reducing the loss of silicone deposited onto a keratinous substrate from a thickened cleansing composition, the composition and method comprising combining a crosslinked, nonionic amphiphilic polymer with at least one detersive surfactant selected from anionic surfactants, amphoteric surfactants, nonionic surfactants and combinations of two or more thereof, wherein the concentration of the amphiphilic polymer is no more than 5 wt.%, and the at least one surfactant is no more than 30 wt.% (all weight percentages are based on the total weight of the composition), wherein the yield stress of the composition is at least 0.1 Pa with a shear thinning index of less than 0.5 at shear rates between 0.1 and 1 reciprocal seconds, wherein the yield stress, elastic modulus of the composition are substantially independent of pH in the range of 2 to 14, and wherein the composition is able to suspend beads of a size between 0.5 and 1.5 mm where the difference in specific gravity of the beads relative to water is in the range of 0.2 to 0.5, for a period of at least 4 weeks at room temperature.

In another aspect, an embodiment of the invention relates to a composition and method for reducing the loss of silicone deposited onto a keratinous substrate from a cleansing composition, the composition and method comprising combining a linear, nonionic amphiphilic polymer with at least one detersive surfactant selected from anionic surfactants, amphoteric surfactants, nonionic surfactants and combinations of two or more thereof, wherein the concentration of the amphiphilic polymer is no more than 5 wt.%, and the at least one surfactant is no more than 30 wt.% (all weight percentages are based on the total weight of the composition), wherein the yield stress of the composition is 0 Pa.

In one aspect of the present invention, the nonionic amphiphilic is prepared from a free radically polymerizable monomer composition comprising at least one hydrophilic monomer and at least one hydrophobic monomer. In one embodiment the hydrophilic monomer is selected from N-vinyl amides, hydroxy(C₁-C₅)alkyl (meth)acrylates, amino group containing monomers, or mixtures thereof. In one aspect, the hydrophobic monomer is selected from vinyl ester of an aliphatic carboxylic acid containing an acyl moiety having 2 to 22 carbon atoms, esters of (meth)acrylic acid with alcohols containing 1 to 30 carbon atoms, vinyl ethers of alcohols containing 1 to 22 carbon atoms, vinyl aromatic monomers, vinyl halides, vinylidene halides, associative monomers, semi-hydrophobic monomers, or mixtures thereof.

In one aspect, of the present invention, the nonionic, amphiphilic polymer is prepared from a free radically polymerizable monomer composition comprising at least one hydrophilic monomer, at least one hydrophobic monomer, and at least one crosslinking monomer. In one embodiment, the hydrophilic monomer is selected from N-vinyl amides, amino(C₁-C₅)alkyl (meth)acrylates, hydroxy(C₁-C₅)alkyl (meth)acrylates, amino group containing monomers, or mixtures thereof. In one aspect, the hydrophobic monomer is selected from vinyl ester of an aliphatic carboxylic acid containing an acyl moiety having 2 to 22 carbon atoms, esters of (meth)acrylic acid with alcohols containing 1 to 30 carbon atoms, vinyl ethers of alcohols containing 1 to 22 carbon atoms, vinyl aromatic monomers, vinyl halides, vinylidene halides, associative monomers, semi-hydrophobic monomers, or mixtures thereof. In one embodiment, the crosslinking monomer is selected from at least one polyunsaturated monomer containing at least two polymerizable unsaturated moieties.

In one aspect, of the invention, the nonionic amphiphilic polymer is prepared from a free radically polymerizable monomer composition comprising at least one N-vinyl amide monomer, at least one vinyl ester of an aliphatic carboxylic acid containing an acyl moiety having 2 to 22 carbon atoms, and at least one crosslinking monomer, in optional combination with at least one monomer selected from esters of (meth)acrylic acid with alcohols containing 1 to 30 carbon atoms, associative monomers, semi-hydrophobic monomers, or mixtures thereof.

The silicone containing cleansing compositions comprising the nonionic amphiphilic polymer of the invention provide stable formulations without interfering with the deposition of silicone material onto the hair and/or the skin regardless of silicone type and silicone particle size.

The methods, polymers and compositions of the present invention may suitably comprise, consist of, or consist essentially of the components, elements, steps, and process delineations described herein. The invention illustratively disclosed herein suitably may be practiced in the absence of any element which is not specifically disclosed herein.

Unless otherwise stated, all percentages, parts, and ratios expressed herein are based upon weight of the total compositions of the present invention.

When referring to a specified monomer(s) that is incorporated into a polymer of the invention, it will be recognized that the monomer(s) will be incorporated into the polymer as a unit(s) derived from the specified monomer(s) (e.g., repeating unit).

As used herein, the term "amphiphilic polymer" means that the polymeric material has distinct hydrophilic and hydrophobic portions. "Hydrophilic" typically means a portion that interacts intramolecularly with water and other polar molecules. "Hydrophobic" typically means a portion that interacts preferentially with oils, fats or other non-polar molecules rather than aqueous media.

As used herein, the term "hydrophilic monomer" means a monomer that is substantially water soluble. "Substantially water soluble" refers to a material that is soluble in distilled (or equivalent) water, at 25°C, at a concentration of about 3.5% by weight in one aspect, and soluble at about 10% by weight in another aspect (calculated on a water plus monomer weight basis).

As used herein, the term "hydrophobic monomer" means a monomer that is substantially water insoluble. "Substantially water insoluble" refers to a material that is not soluble in distilled (or equivalent) water, at 25°C, at a concentration of 3% by weight in one aspect, and not soluble at 2.5% by weight in another aspect (calculated on a water plus monomer weight basis).

By "nonionic" is meant that a monomer, monomer composition or a polymer polymerized from a monomer composition is devoid of ionic or ionizable moieties ("nonionizable").

An ionizable moiety is any group that can be made ionic by neutralization with an acid or a base.

An ionic or an ionized moiety is any moiety that has been neutralized by an acid or a base.

By "substantially nonionic" is meant that the monomer, monomer composition or polymer polymerized from a monomer composition contains less than 5 wt.% in one aspect, less than 3 wt.% in another aspect, less than 1 wt.% in a further aspect, less than 0.5 wt.% in a still further aspect, less than 0.1 wt.% in an additional aspect, and less than 0.05 wt.% in a further aspect, of an ionizable and/or an ionized moiety.

The prefix "(meth)acryl" includes "acryl" as well as "methacryl". For example, the term (meth)acrylic includes both acrylic and methacrylic, and the term (meth)acrylate includes acrylate as well as methacrylate. By way of further example, the term "(meth)acrylamide" includes both acrylamide and methacrylamide.

The term "keratinous" material as used herein, without limitation, includes hair, nails and skin.

The term "personal care products" as used herein, without limitation, includes cosmetics, toiletries, cosmeceuticals, beauty aids, insect repellents, personal hygiene and cleansing products applied to the body, including the skin, hair, scalp, and nails of humans and animals.

Here, as well as elsewhere in the specification and claims, individual numerical values (including carbon atom numerical values), or limits, can be combined to form additional non-disclosed and/or non-stated ranges.

While overlapping weight ranges for the various components and ingredients that can be contained in the compositions of the invention have been expressed for selected embodiments and aspects of the invention, it should be readily apparent that the specific amount of each component in the disclosed compositions will be selected from its disclosed range such that the amount of each component is adjusted such that the sum of all components in the composition will total 100 weight percent. The amounts employed will vary with the purpose and character of the desired product and can be readily determined by one skilled in the art.

The headings provided herein serve to illustrate the invention.

### BRIEF DESCRIPTION OF THE DRAWINGS

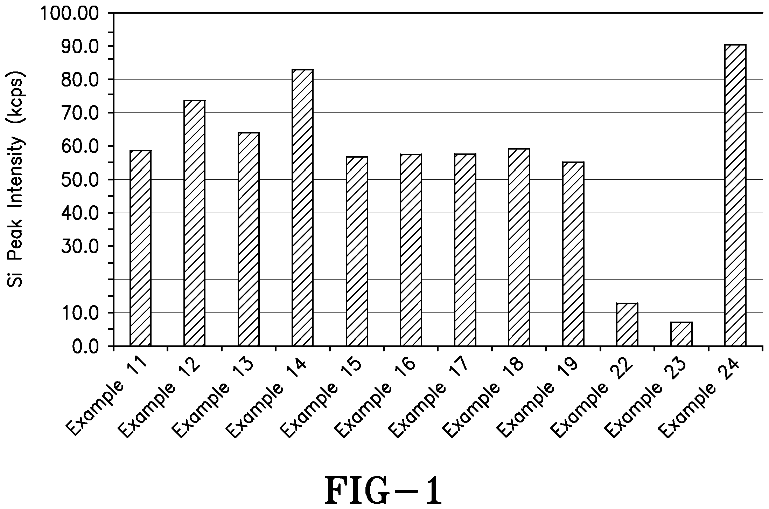

Fig. 1 is a graph depicting the silicon atom deposition onto wool substrates measured by X-Ray fluorescence (XRF) spectroscopy from the silicone containing shampoo compositions of Examples 11-19 which were formulated with the nonionic, amphiphilic polymers of the invention.

### DESCRIPTION OF EXEMPLARY EMBODIMENTS OF THE INVENTION

Exemplary embodiments in accordance with the present invention will be described. Various modifications, adaptations or variations of the exemplary embodiments described herein may become apparent to those skilled in the art as such are disclosed.

### Amphiphilic Polymer

The nonionic, amphiphilic polymers useful in the practice of the invention are polymerized from monomer components that contain free radical polymerizable unsaturation. In one embodiment, the nonionic, amphiphilic polymers useful in the practice of the invention are polymerized from a monomer composition comprising at least one nonionic, hydrophilic unsaturated monomer, and at least one unsaturated hydrophobic monomer. In another embodiment, the nonionic, amphiphilic polymers useful in the practice of the invention are crosslinked. The crosslinked polymers are prepared from a monomer composition comprising at least one nonionic, hydrophilic unsaturated monomer, at least one unsaturated hydrophobic monomer, and at least one polyunsaturated crosslinking monomer.

In one embodiment, the nonionic, amphiphilic polymers can be prepared from a monomer composition typically having a hydrophilic monomer to hydrophobic monomer ratio of from 55:45 wt.% to 95:5 wt.% in one aspect, from 60:40 wt.% to 90:10 wt.% in another aspect, from 65:35 wt.% to 85:15 wt.% in a further aspect, and from 70:30 to 80:20 wt.% in a still further aspect, based on the total weight of the hydrophilic and hydrophobic monomers present. The hydrophilic monomer component can be selected from a single hydrophilic monomer or a mixture of hydrophilic monomers, and the hydrophobic monomer component can be selected from a single hydrophobic monomer or a mixture of hydrophobic monomers.

### Hydrophilic Monomer

Representative hydrophilic monomers include but are not limited to open chain and cyclic N-vinylamides (N-vinyl lactams containing 4 to 9 atoms in the lactam ring moiety, wherein the ring carbon atoms optionally can be substituted by one or more lower alkyl groups such as methyl, ethyl or propyl); amino(C₁-C₅)alkyl (meth)acrylates; hydroxy(C₁-C₅)alkyl (meth)acrylates; amino group containing vinyl monomers selected from (meth)acrylamide, N-(C₁-C₅)alkyl(meth)acrylamides, N,N-di(C₁-C₅)alkyl(meth)acrylamides, N-(C₁-C₅)alkylamino(C₁-C₅)alkyl(meth)acrylamides and N,N-di(C₁-C₅)alkylamino(C₁-C₅)alkyl(meth)acrylamides, wherein the alkyl moieties on the disubstituted amino groups can be the same or different, and wherein the alkyl moieties on the monosubstituted and disubstituted amino groups can be optionally substituted with a hydroxyl group; other monomers include vinyl alcohol; vinyl imidazole; and (meth)acrylonitrile. Mixtures of the foregoing monomers also can be utilized.

Representative open chain N-vinylamides include N-vinylformamide, N-methyl-N-vinylformamide, N-(hydroxymethyl)-N-vinylformamide, N-vinylacetamide, N-vinylmethylacetamide, N-(hydroxymethyl)-N-vinylacetamide, and mixtures thereof. Additionally, monomers containing a pendant N-vinyl lactam moiety can also be employed, e.g., N-vinyl-2-ethyl-2-pyrrolidone (meth)acrylate.

Representative cyclic N-vinylamides (also known as N-vinyl lactams) include N-vinyl-2-pyrrolidinone, N-(1-methyl vinyl) pyrrolidinone, N-vinyl-2-piperidone, N-vinyl-2-caprolactam, N-vinyl-5-methyl pyrrolidinone, N-vinyl-3,3-dimethyl pyrrolidinone, N-vinyl-5-ethyl pyrrolidinone and N-vinyl-6-methyl piperidone, and mixtures thereof.

The hydroxy(C₁-C₅)alkyl (meth)acrylates can be structurally represented by the following formula: 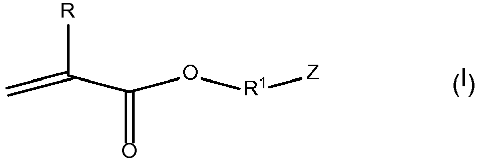 wherein R is hydrogen or methyl and R¹ is an divalent alkylene moiety containing 1 to 5 carbon atoms, and Z is -NH₂ or -OH, wherein the alkylene moiety optionally can be substituted by one or more methyl groups. Representative monomers include 2-aminoethyl(meth)acrylate, 2-hydroxyethyl(meth)acrylate, 3-hydroxypropyl(meth)acrylate, 4-hydroxybutyl(meth)acrylate, and mixtures thereof.

The amino group containing vinyl monomers include (meth)acrylamide, diacetone acrylamide and monomers that are structurally represented by the following formulas:

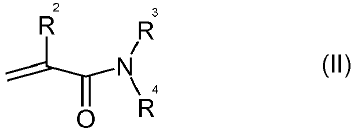

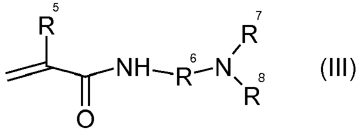

Formula (II) represents N-(C₁-C₅)alkyl(meth)acrylamide or N,N-di(C₁-C₅)alkyl(meth)acrylamide wherein R² is hydrogen or methyl, R³ independently is selected from hydrogen, C₁ to C₅ alkyl and C₁ to C₅ hydroxyalkyl, and R⁴ independently is selected from is C₁ to C₅ alkyl or C₁ to C₅ hydroxyalkyl.

Formula (III) represents N-(C₁-C₅)alkylamino(C₁-C₅)alkyl(meth)acrylamide or N,N-di(C₁-C₅)alkylamino(C₁-C₅)alkyl(meth)acrylamide wherein R⁵ is hydrogen or methyl, R⁶ is C₁ to C₅ alkylene, R⁷ independently is selected from hydrogen or C₁ to C₅ alkyl, and R⁸ independently is selected from C₁ to C₅ alkyl.

Representative N-alkyl(meth)acrylamides include but are not limited to N-methyl(meth)acrylamide, N-ethyl(meth)acrylamide, N-propyl(meth)acrylamide, N-isopropyl(meth)acrylamide, N-tert-butyl(meth)acrylamide, N-(2-hydroxyethyl)(meth)acrylamide, N-(3-hydroxypropyl)(meth)acrylamide, and mixtures thereof.

Representative N,N-dialkyl(meth)acrylamides include but are not limited to N,N-dimethyl(meth)acrylamide, N,N-diethyl(meth)acrylamide, N,N-(di-2-hydroxyethyl)(meth)acrylamide, N,N-(di-3-hydroxypropyl)(meth)acrylamide, N-methyl,N-ethyl(meth)acrylamide, and mixtures thereof.

Representative N,N-dialkylaminoalkyl(meth)acrylamides include but are not limited to N,N-dimethylaminoethyl(meth)acrylamide, N,N-diethylaminoethyl(meth)acrylamide, N,N-dimethylaminopropyl(meth)acrylamide, and mixtures thereof.

### Hydrophobic Monomer

Hydrophobic monomers suitable for the preparation of the crosslinked, nonionic, amphiphilic polymer compositions of the invention are selected from but are not limited to one or more of alkyl esters of (meth)acrylic acid having an alkyl group containing 1 to 30 carbon atoms; vinyl ester of an aliphatic carboxylic acid containing an acyl moiety having 2 to 22 carbon atoms; vinyl ethers of alcohols containing 1 to 22 carbon atoms; vinyl aromatics containing 8 to 20 carbon atoms; vinyl halides; vinylidene halides; linear or branched alpha-monoolefins containing 2 to 8 carbon atoms; an alkoxylated associative monomer having a hydrophobic end group containing 8 to 30 carbon atoms, and mixtures thereof.

### Semi-Hydrophobic Monomer

Optionally, at least one alkoxylated semi-hydrophobic monomer can be used in the preparation of the amphiphilic polymers of the invention. A semi-hydrophobic monomer is similar in structure to an associative monomer, but has a substantially non-hydrophobic end group selected from hydroxyl or a moiety containing 1 to 4 carbon atoms.

In one aspect, of the invention, alkyl esters of (meth)acrylic acid having an alkyl group containing 1 to 22 carbon atoms can be represented by the following formula: 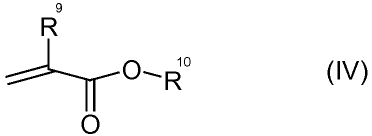 wherein R⁹ is hydrogen or methyl and R¹⁰ is C₁ to C₂₂ alkyl

Representative monomers under formula (IV) include but are not limited to methyl (meth)acrylate, ethyl (meth)acrylate, butyl (meth)acrylate, sec-butyl (meth)acrylate, iso-butyl (meth)acrylate, hexyl (meth)acrylate), heptyl (meth)acrylate, octyl (meth)acrylate, 2-ethylhexyl (meth)acrylate, decyl (meth)acrylate, isodecyl (meth)acrylate, lauryl (meth)acrylate, tetradecyl (meth)acrylate, hexadecyl (meth)acrylate, stearyl (meth)acrylate, behenyl (meth)acrylate, and mixtures thereof.

Vinyl esters of an aliphatic carboxylic acid containing an acyl moiety having 2 to 22 carbon atoms can be represented by the following formula: 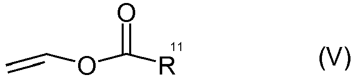 wherein R¹¹ is a C₁ to C₂₁ aliphatic group which can be an alkyl or alkenyl. Formula (V) contains an acyl moiety Representative monomers under formula (V) include but are not limited to vinyl acetate, vinyl propionate, vinyl butyrate, vinyl isobutyrate, vinyl valerate, vinyl hexanoate, vinyl 2-methylhexanoate, vinyl 2-ethylhexanoate, vinyl iso-octanoate, vinyl nonanoate, vinyl neodecanoate, vinyl decanoate, vinyl versatate, vinyl laurate, vinyl palmitate, vinyl stearate, and mixtures thereof.

In one aspect, the vinyl ethers of alcohols containing 1 to 22 carbon atoms can be represented by the following formula: 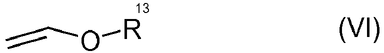 wherein R¹³ is a C₁ to C₂₂ alkyl. Representative monomers of formula (VI) include methyl vinyl ether, ethyl vinyl ether, butyl vinyl ether, isobutyl vinyl ether, 2-ethylhexyl vinyl ether, decyl vinyl ether, lauryl vinyl ether, stearyl vinyl ether, behenyl vinyl ether, and mixtures thereof.

Representative vinyl aromatic monomers include but are not limited to styrene, alpha-methylstyrene, 3-methyl styrene, 4-methyl styrene, 4-propyl styrene, 4-tert-butyl styrene, 4-n-butyl styrene, 4-n-decyl styrene, vinyl naphthalene, and mixtures thereof.

Representative vinyl and vinylidene halides include but are not limited to vinyl chloride and vinylidene chloride, and mixtures thereof.

Representative alpha-olefins include but are not limited to ethylene, propylene, 1-butene, iso-butylene, 1-hexene, and mixtures thereof.

The alkoxylated associative monomer of the invention has an ethylenically unsaturated end group portion (i) for addition polymerization with the other monomers of the invention; a polyoxyalkylene mid-section portion (ii) for imparting selective hydrophilic and/or hydrophobic properties to the product polymer, and a hydrophobic end group portion (iii) for providing selective hydrophobic properties to the polymer.

The portion (i) supplying the ethylenically unsaturated end group can be a residue derived from an α,β-ethylenically unsaturated monocarboxylic acid. Alternatively, portion (i) of the associative monomer can be a residue derived from an allyl ether or vinyl ether; a nonionic vinyl-substituted urethane monomer, such as disclosed in U.S. Reissue Patent No. 33,156 or U.S. Patent No. 5,294,692; or a vinyl-substituted urea reaction product, such as disclosed in U.S. Patent No. 5,011,978.

The mid-section portion (ii) is a polyoxyalkylene segment of 2 to 150 in one aspect, from 10 to 120 in another aspect, and from 15 to 60 in a further aspect of repeating C₂-C₄ alkylene oxide units. The mid-section portion (ii) includes polyoxyethylene, polyoxypropylene, and polyoxybutylene segments, and combinations thereof comprising from 2 to 150 in one aspect, from 5 to 120 in another aspect, from 10 to 60 in a further aspect, and from 15 to 30 in a still further aspect of ethylene, propylene and/or butylene oxide units, arranged in random or block sequences of ethylene oxide, propylene oxide and/or butylene oxide units.

The hydrophobic end group portion (iii) of the associative monomer is a hydrocarbon moiety belonging to one of the following hydrocarbon classes: a C₈-C₃₀ linear alkyl, a C₈-C₃₀ branched alkyl, a C₂-C₃₀ alkyl-substituted phenyl, aryl-substituted C₂-C₃₀ alkyl groups, a C₇-C₃₀ saturated or unsaturated carbocyclic alkyl group. The saturated or unsaturated carbocyclic moiety can be a C₁-C₅ alkyl substituted or unsubstituted monocyclic or bicyclic moiety. In one aspect the bicyclic moiety is selected from bicycloheptyl or bicycloheptenyl. In another aspect the bicycloheptenyl moiety is disubstituted with the alkyl substituent(s). In a further aspect the bicycloheptenyl moiety is disubstituted with methyl on the same carbon atom.

Non-limiting examples of suitable hydrophobic end group portions (iii) of the associative monomers are linear or branched alkyl groups having about 8 to about 30 carbon atoms, such as capryl (C₈), iso-octyl (branched C₈), decyl (C₁₀), lauryl (C₁₂), myristyl (C₁₄), cetyl (C₁₆), cetearyl (C₁₆-C₁₈), stearyl (C₁₈), isostearyl (branched C₁₈), arachidyl (C₂₀), behenyl (C₂₂), lignoceryl (C₂₄), cerotyl (C₂₆), montanyl (C₂₈), melissyl (C₃₀), and the like.

Examples of linear and branched alkyl groups having 8 to 30 carbon atoms that are derived from a natural source include, without being limited thereto, alkyl groups derived from hydrogenated peanut oil, soybean oil and canola oil (all predominately C₁₈), hydrogenated tallow oil (C₁₆-C₁₈), and the like; and hydrogenated C₁₀-C₃₀ terpenols, such as hydrogenated geraniol (branched C₁₀), hydrogenated farnesol (branched C₁₅), hydrogenated phytol (branched C₂₀), and the like.

Non-limiting examples of suitable C₂-C₃₀ alkyl-substituted phenyl groups include octylphenyl, nonylphenyl, decylphenyl, dodecylphenyl, hexadecylphenyl, octadecylphenyl, isooctylphenyl, sec-butylphenyl, and the like.

Exemplary aryl-substituted C₂-C₄₀ alkyl groups include, without limitation, styryl (e.g., 2-phenylethyl), distyryl (e.g., 2,4-diphenylbutyl), tristyryl (e.g., 2,4,6-triphenylhexyl), 4-phenylbutyl, 2-methyl-2-phenylethyl, tristyrylphenolyl, and the like.

Suitable C₇-C₃₀ carbocyclic groups include, without limitation, groups derived from sterols from animal sources, such as cholesterol, lanosterol, 7-dehydrocholesterol, and the like; from vegetable sources, such as phytosterol, stigmasterol, campesterol, and the like; and from yeast sources, such as ergosterol, mycosterol, and the like. Other carbocyclic alkyl hydrophobic end groups useful in the present invention include, without limitation, cyclooctyl, cyclododecyl, adamantyl, decahydronaphthyl, and groups derived from natural carbocyclic materials, such as pinene, hydrogenated retinol, camphor, isobornyl alcohol, norbornyl alcohol, nopol and the like.

Useful alkoxylated associative monomers can be prepared by any method known in the art. See, for example, U.S. Patents No. 4,421,902 to Chang et al.; No. 4,384,096 to Sonnabend; No. 4,514,552 to Shay et al.; No. 4,600,761 to Ruffner et al.; No. 4,616,074 to Ruffner; No. 5,294,692 to Barron et al.; No. 5,292,843 to Jenkins et al*.*; No. 5,770,760 to Robinson; No. 5,412,142 to Wilkerson, III et al.; and No. 7,772,421, to Yang et al.

In one aspect, exemplary alkoxylated associative monomers include those represented by formulas (VII) and (VIIA) as follows: 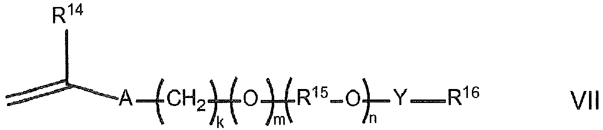 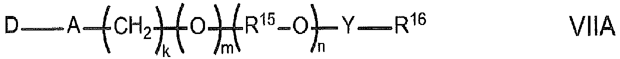 wherein R¹⁴ is hydrogen or methyl; A
is -CH₂C(O)O-, -C(O)O-, -O-, -CH₂O- ,-NHC(O)NH-, -C(O)NH-, -Ar-(CE₂)_{z}-NHC(O)O-, -Ar-(CE₂)_{z}-NHC(O)NH-, or -CH₂CH₂NHC(O)-; Ar is a divalent arylene (e.g., phenylene); E is H or methyl; z is 0 or 1; k is an integer ranging from 0 to 30, and m is 0 or 1, with the proviso that when k is 0, m is 0, and when k is in the range of 1 to 30, m is 1; D represents a vinyl or an allyl moiety; (R¹⁵-O)ₙ is a polyoxyalkylene moiety, which can be a homopolymer, a random copolymer, or a block copolymer of C₂-C₄ oxyalkylene units, R¹⁵ is a divalent alkylene moiety selected from C₂H₄, C₃H₆, or C₄H₈, and combinations thereof; and n is an integer in the range of 2 to 150 in one aspect, from 10 to 120 in another aspect, and from 15 to 60 in a further aspect; Y is - R¹⁵O-, -R¹⁵NH-, -C(O)-, -C(O)NH-, -R¹⁵NHC(O)NH-, -C(O)NHC(O)-, or a divalent alkylene radical containing 1 to 5 carbon atoms, e.g., methylene, ethylene, propylene, butylene, pentylene; R¹⁶ is a substituted or unsubstituted alkyl selected from a C₈-C₃₀ linear alkyl, a C₈-C₃₀ branched alkyl, a C₇-C₃₀ carbocyclic, a C₂-C₃₀ alkyl-substituted phenyl, an araalkyl substituted phenyl, and an aryl-substituted C₂-C₃₀ alkyl; wherein the R¹⁶ alkyl group, aryl group, phenyl group, or carbocyclic group optionally comprises one or more substituents selected from the group consisting of a methyl group, a hydroxyl group, an alkoxyl group, benzyl group phenylethyl group, and a halogen group. In one aspect, Y is ethylene and R¹⁶ is

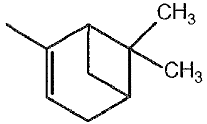

In one aspect, the hydrophobically modified alkoxylated associative monomer is an alkoxylated (meth)acrylate having a hydrophobic group containing 8 to 30 carbon atoms represented by the following Formula VB as follows: 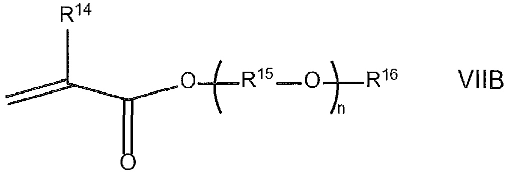 wherein R¹⁴ is hydrogen or methyl; R¹⁵ is a divalent alkylene moiety independently selected from C₂H₄, C₃H₆, and C₄H₈, and n represents an integer ranging from 2 to 150 in one aspect, from 5 to 120 in another aspect, from 10 to 60 in a further aspect, and from 15 to 30 in a still further aspect, (R¹⁵-O) can be arranged in a random or a block configuration; R¹⁶ is a substituted or unsubstituted alkyl selected from a C₈-C₃₀ linear alkyl, a C₈-C₃₀ branched alkyl, an alkyl substituted and unsubstituted C₇-C₃₀ carbocyclic alkyl, a C₂-C₃₀ alkyl-substituted phenyl, and an aryl-substituted C₂-C₃₀ alkyl.

Representative monomers under Formula V include lauryl polyethoxylated (meth)acrylate (LEM), cetyl polyethoxylated (meth)acrylate (CEM), cetearyl polyethoxylated (meth)acrylate (CSEM), stearyl polyethoxylated (meth)acrylate, arachidyl polyethoxylated (meth)acrylate, behenyl polyethoxylated (meth)acrylate (BEM), cerotyl polyethoxylated (meth)acrylate, montanyl polyethoxylated (meth)acrylate, melissyl polyethoxylated (meth)acrylate, phenyl polyethoxylated (meth)acrylate, nonylphenyl polyethoxylated (meth)acrylate, ω-tristyrylphenyl polyoxyethylene (meth)acrylate, where the polyethoxylated portion of the monomer contains 2 to 150 ethylene oxide units in one aspect, from 5 to 120 in another aspect, from 10 to 60 in a further aspect and from 15 to 30 in a still further aspect; octyloxy polyethyleneglycol (8) polypropyleneglycol (6) (meth)acrylate, phenoxy polyethylene glycol (6) polypropylene glycol (6) (meth)acrylate, and nonylphenoxy polyethylene glycol polypropylene glycol (meth)acrylate.

The alkoxylated semi-hydrophobic monomers of the invention are structurally similar to the associative monomer described above, but have a substantially non-hydrophobic end group portion. The alkoxylated semi-hydrophobic monomer has an ethylenically unsaturated end group portion (i) for addition polymerization with the other monomers of the invention; a polyoxyalkylene mid-section portion (ii) for imparting selective hydrophilic and/or hydrophobic properties to the product polymer and a semi- hydrophobic end group portion (iii). The unsaturated end group portion (i) supplying the vinyl or other ethylenically unsaturated end group for addition polymerization is preferably derived from an α,β-ethylenically unsaturated mono carboxylic acid. Alternatively, the end group portion (i) can be derived from an allyl ether residue, a vinyl ether residue or a residue of a nonionic urethane monomer.

The polyoxyalkylene mid-section (ii) specifically comprises a polyoxyalkylene-segment, which is substantially similar to the polyoxyalkylene portion of the associative monomers described above. In one aspect, the polyoxyalkylene portions (ii) include polyoxyethylene, polyoxypropylene, and/or polyoxybutylene units comprising from 2 to 150 in one aspect, from 5 to 120 in another aspect, from 10 to 60, and from 15 to 30 in a still further aspect in a further aspect of ethylene oxide, propylene oxide, and/or butylene oxide units, arranged in random or blocky sequences.

In one aspect, the alkoxylated semi-hydrophobic monomer can be represented by the following formulas: 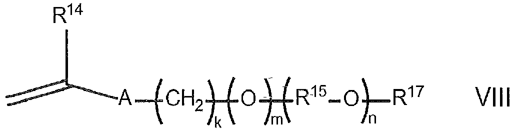 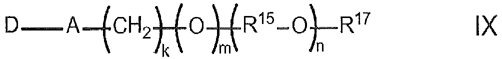 wherein R¹⁴ is hydrogen or methyl; A
is -CH₂C(O)O-, -C(O)O-, -O-, -CH₂O- ,-NHC(O)NH-, -C(O)NH-, -Ar-(CE₂)_{z}-NHC(O)O-, -Ar-(CE₂)_{z}-NHC(O)NH-, or -CH₂CH₂NHC(O)-; Ar is a divalent arylene (e.g., phenylene); E is H or methyl; z is 0 or 1; k is an integer ranging from 0 to 30, and m is 0 or 1, with the proviso that when k is 0, m is 0, and when k is in the range of 1 to 30, m is 1; (R¹⁵-O)ₙ is a polyoxyalkylene moiety, which can be a homopolymer, a random copolymer, or a block copolymer of C₂-C₄ oxyalkylene units, R¹⁵ is a divalent alkylene moiety selected from C₂H₄, C₃H₆, or C₄H₈, and combinations thereof; and n is an integer in the range of 2 to 150 in one aspect, from 5 to 120 in another aspect, and from 10 to 60, and from 15 to 30 in a still further aspect in a further aspect; R¹⁷ is selected from hydrogen and a linear or branched C₁-C₄ alkyl group (e.g., methyl, ethyl, propyl, iso-propyl, butyl, iso-butyl, and tert-butyl); and D represents a vinyl or an allyl moiety.

In one aspect, the alkoxylated semi-hydrophobic monomer under formula VIII can be represented by the following formulas:

CH₂=C(R¹⁴)C(O)O-(C₂H₄O)ₐ(C₃H₆O)_{b}-H VIIIA

CH₂=C(R¹⁴)C(O)O-(C₂H₄O)ₐ(C₃H₆O)_{b}-CH₃ VIIIB

wherein R¹⁴ is hydrogen or methyl, and "a" is an integer ranging from 0 or 2 to 120 in one aspect, from 5 to 45 in another aspect, and from 10 to 25 in a further aspect, and "b" is an integer ranging from 0 or 2 to 120 in one aspect, from 5 to 45 in another aspect, and from 10 to 25 in a further aspect, subject to the proviso that "a" and "b" cannot be 0 at the same time.

Examples of alkoxylated semi-hydrophobic monomers under formula VIIIA include polyethyleneglycol methacrylate available under the product names Blemmer® PE-90 (R¹⁴ = methyl, a = 2, b = 0), PE-200 (R¹⁴ = methyl, a = 4.5, b = 0), and PE-350 (R¹⁴ = methyl a = 8, b = 0,); polypropylene glycol methacrylate available under the product names Blemmer® PP-1000 (R¹⁴ = methyl, b = 4-6, a = 0), PP-500 (R¹⁴ = methyl, a = 0, b = 9), PP-800 (R¹⁴ = methyl, a = 0, b = 13); polyethyleneglycol polypropylene glycol methacrylate available under the product names Blemmer® 50PEP-300 (R¹⁴ = methyl, a = 3.5, b = 2.5), 70PEP-350B (R¹⁴ = methyl, a = 5, b = 2); polyethyleneglycol acrylate available under the product names Blemmer® AE-90 (R¹⁴ = hydrogen, a = 2, b = 0), AE-200 (R¹⁴ = hydrogen, a = 2, b = 4.5), AE-400 (R¹⁴ = hydrogen, a = 10, b = 0); polypropyleneglycol acrylate available under the product names Blemmer® AP-150 (R¹⁴ = hydrogen, a = 0, b = 3), AP-400(R¹⁴ = hydrogen, a = 0, b = 6), AP-550 (R¹⁴ = hydrogen, a = 0, b = 9). Blemmer® is a trademark of NOF Corporation, Tokyo, Japan.

Examples of alkoxylated semi-hydrophobic monomers under formula VIIIB include methoxypolyethyleneglycol methacrylate available under the product names Visiomer® MPEG 750 MA W (R¹⁴ = methyl, a = 17, b = 0), MPEG 1005 MA W (R¹⁴ = methyl, a = 22, b = 0), MPEG 2005 MA W (R¹⁴ = methyl, a = 45, b = 0), and MPEG 5005 MA W (R¹⁴ = methyl, a = 113, b = 0) from Evonik Röhm GmbH, Darmstadt, Germany); Bisomer® MPEG 350 MA (R¹⁴ = methyl, a = 8, b = 0), and MPEG 550 MA (R¹⁴ = methyl, a = 12, b = 0) from GEO Specialty Chemicals, Ambler PA; Blemmer® PME-100 (R¹⁴ = methyl, a = 2, b = 0), PME-200 (R¹⁴ = methyl, a = 4, b = 0), PME-400 (R¹⁴ = methyl, a = 9, b = 0), PME-1000 (R¹⁴ = methyl, a = 23, b = 0), PME-4000 (R¹⁴ = methyl, a = 90, b = 0).

In one aspect, the alkoxylated semi-hydrophobic monomer set forth in formula IX can be represented by the following formulas:

CH₂=CH-O-(CH₂)_{d}-O-(C₃H₆O)ₑ-(C₂H₄O)_{f}-H IXA

CH₂=CH-CH₂-O-(CₛHₛO)_{g}-(C₂H₄0)ₕ-H IXB

wherein d is an integer of 2, 3, or 4; e is an integer in the range of from 1 to 10 in one aspect, from 2 to 8 in another aspect, and from 3 to 7 in a further aspect; f is an integer in the range of from 5 to 50 in one aspect, from 8 to 40 in another aspect, and from 10 to 30 in a further aspect; g is an integer in the range of from 1 to 10 in one aspect, from 2 to 8 in another aspect, and from 3 to 7 in a further aspect; and h is an integer in the range of from 5 to 50 in one aspect, and from 8 to 40 in another aspect; e, f, g, and h can be 0 subject to the proviso that e and f cannot be 0 at the same time, and g and h cannot be 0 at the same time.

Monomers under formulas IXA and IXB are commercially available under the trade names Emulsogen® R109, R208, R307, RAL109, RAL208, and RAL307 sold by Clariant Corporation; BX-AA-E5P5 sold by Bimax, Inc.; and combinations thereof. EMULSOGEN7 R109 is a randomly ethoxylated/propoxylated 1,4-butanediol vinyl ether having the empirical formula CH₂=CH-O(CH₂)₄O(C₃H₆O)₄(C₂H₄O)₁₀H; Emulsogen® R208 is a randomly ethoxylated/propoxylated 1,4-butanediol vinyl ether having the empirical formula CH₂=CH-O(CH₂)₄O(C₃H₆O)₄(C₂H₄O)₂₀H; Emulsogen® R307 is a randomly ethoxylated/propoxylated 1,4-butanediol vinyl ether having the empirical formula CH₂=CH-O(CH₂)₄O(C₃H₆O)₄(C₂H₄O)₃₀H; Emulsogen® RAL109 is a randomly ethoxylated/propoxylated allyl ether having the empirical formula CH₂=CHCH₂O(C₃H₆O)₄(C₂H₄O)₁₀H; Emulsogen® RAL208 is a randomly ethoxylated/propoxylated allyl ether having the empirical formula CH₂=CHCH₂O(C₃H₆O)₄(C₂H₄O)₂₀H; Emulsogen® RAL307 is a randomly ethoxylated/propoxylated allyl ether having the empirical formula CH₂=CHCH₂O(C₃H₆O)₄(C₂H₄O)₃₀H; and BX-AA-E5P5 is a randomly ethoxylated/propoxylated allyl ether having the empirical formula CH₂=CHCH₂O(C₃H₆O)₅(C₂H₄O)₅H.

Referring to the alkoxylated associative and the alkoxylated semi-hydrophobic monomers of the invention, the polyoxyalkylene mid-section portion contained in these monomers can be utilized to tailor the hydrophilicity and/or hydrophobicity of the polymers in which they are included. For example, mid-section portions rich in ethylene oxide moieties are more hydrophilic while mid-section portions rich in propylene oxide moieties are more hydrophobic. By adjusting the relative amounts of ethylene oxide to propylene oxide moieties present in these monomers the hydrophilic and hydrophobic properties of the polymers in which these monomers are included can be tailored as desired.

The amount of alkoxylated associative and/or semi-hydrophobic monomer utilized in the preparation of the polymers of the present invention can vary widely and depends, among other things, on the final rheological and aesthetic properties desired in the polymer. When utilized, the monomer reaction mixture contains one or more monomers selected from the alkoxylated associative and/or semi-hydrophobic monomers disclosed above in amounts ranging from 0.5 to 10 wt.% in one aspect, and from 1, 2 or 3 to 5 wt.% in a further aspect, based on the weight of the total monomers.

### Ionizable Monomer

In one aspect of the invention, the nonionic, amphiphilic polymer compositions of the invention can be polymerized from a monomer composition including 0 to 5 wt.% of an ionizable and/or ionized monomer, based on the weight of the total monomers, so long as the mitigation of silicone deposition loss and/or the yield stress value of the surfactant compositions in which the polymers of the invention are included are not deleteriously affected.

In another aspect, the amphiphilic polymer compositions of the invention can be polymerized from a monomer composition comprising less than 3 wt.% in one aspect, less than 1 wt.% in a further aspect, less than 0.5 wt.% in a still further aspect, less than 0.1 wt.% in an additional aspect, and less than 0.05 wt.% in a further aspect, of an ionizable and/or an ionized moiety, based on the weight of the total monomers.

Ionizable monomers include monomers having a base neutralizable moiety and monomers having an acid neutralizable moiety. Base neutralizable monomers include olefinically unsaturated monocarboxylic and dicarboxylic acids and their salts containing 3 to 5 carbon atoms and anhydrides thereof. Examples include (meth)acrylic acid, itaconic acid, maleic acid, maleic anhydride, and combinations thereof. Other acidic monomers include styrenesulfonic acid, acrylamidomethylpropanesulfonic acid (AMPS® monomer), vinylsulfonic acid, vinylphosphonic acid, allylsulfonic acid, methallylsulfonic acid; and salts thereof.

Acid neutralizable monomers include olefinically unsaturated monomers which contain a basic nitrogen atom capable of forming a salt or a quaternized moiety upon the addition of an acid. For example, these monomers include vinylpyridine, vinylpiperidine, vinylimidazole, vinylmethylimidazole, dimethylaminomethyl (meth)acrylate, dimethylaminoethyl (meth)acrylate, diethylaminomethyl (meth)acrylate and methacrylate, dimethylaminoneopentyl (meth)acrylate, dimethylaminopropyl (meth)acrylate, and diethylaminoethyl (meth)acrylate.

### Crosslinking Monomer

In one embodiment, the crosslinked, nonionic, amphiphilic polymers useful in the practice of the invention are polymerized from a monomer composition comprising a first monomer comprising at least one nonionic, hydrophilic unsaturated monomer, at least one nonionic, unsaturated hydrophobic monomer, and mixtures thereof, and a third monomer comprising at least one polyunsaturated crosslinking monomer. The crosslinking monomer(s) is utilized to polymerize covalent crosslinks into the polymer backbone. The crosslinking monomer is a polyunsaturated compound containing at least 2 unsaturated moieties. According to the invention the crosslinking momoners are selected from polyallyl ethers of sucrose having from 2 to 8 allyl groups per molecule, polyallyl ethers of pentaerythritol such as pentaerythritol diallyl ether, pentaerythritol triallyl ether, and pentaerythritol tetraallyl ether, and combinations thereof; polyallyl ethers of trimethylolpropane such as trimethylolpropane diallyl ether, trimethylolpropane
triallyl ether, and combinations thereof.

Mixtures of two or more of the foregoing polyunsaturated compounds can also be utilized to crosslink the nonionic, amphiphilic polymers of the invention. In one aspect, the mixture of unsaturated crosslinking monomer contains an average of 2 unsaturated moieties. In another aspect, the mixture of crosslinking monomers contains an average of 2.5 unsaturated moieties. In still another aspect, the mixture of crosslinking monomers contains an average of about 3 unsaturated moieties. In a further aspect, the mixture of crosslinking monomers contains an average of 3.5 unsaturated moieties.
In one embodiment of the invention, the amount of the crosslinking monomer ranges from 0.01 to 1 wt.% in one aspect, from 0.01 to 0.75 wt.% in another aspect, from 0.1 to 0.5 in still another aspect, and from 0.15 to 0.3 wt.% in a still further aspect, all weight percentages are based on the dry weight of the nonionic, amphiphilic polymer of the invention.

In another embodiment of the invention, the crosslinking monomer component contains an average of 3 unsaturated moieties and can be used in an amount ranging from 0.01 to 0.3 wt.% in one aspect, from 0.02 to 0.25 wt.% in another aspect, from 0.05 to 0.2 wt.% in a further aspect, and from 0.075 to 0.175 wt.% in a still further aspect, and from 0.1 to 0.15 wt.% in another aspect, based upon the dry weight of the, nonionic, amphiphilic polymer of the invention.

In one aspect, the crosslinking monomer is selected from pentaerythritol triallylether and polyallyl ethers of sucrose having 3 allyl groups per molecule.

### Amphiphilic Polymer Synthesis

The linear (non-crosslinked) and crosslinked, nonionic, amphiphilic polymers of the present invention can be made using conventional free-radical dispersion polymerization techniques. The polymerization process is carried out in the absence of oxygen under an inert atmosphere such as nitrogen. The polymerization can be carried out in a suitable organic solvent system such as a hydrocarbon solvent, organic solvent, or mixtures thereof. The polymerization reactions are initiated by any means which results in the generation of a suitable free-radical. Thermally derived radicals, in which the radical species is generated from thermal, homolytic dissociation of peroxides, hydroperoxides, persulfates, percarbonates, peroxyesters, hydrogen peroxide and azo compounds can be utilized. The initiators can be water soluble or water insoluble depending on the solvent system employed for the polymerization reaction.

The initiator compounds can be utilized in an amount of up to 30 wt.% in one aspect, 0.01 to 10 wt.% in another aspect, and 0.2 to 3 wt.% in a further aspect, based on the total weight of the dry polymer.

Exemplary free radical water soluble initiators include, but are not limited to, inorganic persulfate compounds, such as ammonium persulfate, potassium persulfate, and sodium persulfate; peroxides such as hydrogen peroxide, benzoyl peroxide, acetyl peroxide, and lauryl peroxide; organic hydroperoxides, such as cumene hydroperoxide and t-butyl hydroperoxide; organic peracids, such as peracetic acid, and water soluble azo compounds, such as 2,2'-azobis(tert-alkyl) compounds having a water solubilizing substituent on the alkyl group. Exemplary free radical oil soluble compounds include, but are not limited to 2,2'-azobisisobutyronitrile, and the like. The peroxides and peracids can optionally be activated with reducing agents, such as sodium bisulfite, sodium formaldehyde, or ascorbic acid, transition metals, hydrazine, and the like.

In one aspect, azo polymerization catalysts include the Vazo^{®} free-radical polymerization initiators, available from DuPont, such as Vazo^{®} 44 (2,2'-azobis(2-(4,5-dihydroimidazolyl)propane), Vazo^{®} 56 (2,2'-azobis(2-methylpropionamidine) dihydrochloride), Vazo^{®} 67 (2,2'-azobis(2-methylbutyronitrile)), and Vazo^{®} 68 (4,4'-azobis(4-cyanovaleric acid)).

Optionally, the use of known redox initiator systems as polymerization initiators can be employed. Such redox initiator systems include an oxidant (intiator) and a reductant. Suitable oxidants include, for example, hydrogen peroxide, sodium peroxide, potassium peroxide, t-butyl hydroperoxide, t-amyl hydroperoxide, cumene hydroperoxide, sodium perborate, perphosphoric acid and salts thereof, potassium permanganate, and ammonium or alkali metal salts of peroxydisulfuric acid, typically at a level of 0.01% to 3.0% by weight, based on dry polymer weight, are used. Suitable reductants include, for example, alkali metal and ammonium salts of sulfur-containing acids, such as sodium sulfite, bisulfite, thiosulfate, hydrosulfite, sulfide, hydrosulfide or dithionite, formadinesulfinic acid, hydroxymethanesulfonic acid, acetone bisulfite, amines such as ethanolamine, glycolic acid, glyoxylic acid hydrate, ascorbic acid, isoascorbic acid, lactic acid, glyceric acid, malic acid, 2-hydroxy-2-sulfinatoacetic acid, tartaric acid and salts of the preceding acids typically at a level of 0.01% to 3.0% by weight, based on dry polymer weight, is used. In one aspect, combinations of peroxodisulfates with alkali metal or ammonium bisulfites can be used, for example, ammonium peroxodisulfate and ammonium bisulfite. In another aspect, combinations of hydrogen peroxide containing compounds (t-butyl hydroperoxide) as the oxidant with ascorbic or erythorbic acid as the reductant can be utilized. The ratio of peroxide-containing compound to reductant is within the range from 30:1 to 0.05:1.

Examples of suitable hydrocarbon solvents or diluents that can be utilized in the polymerization medium are aromatic solvents such as toluene, o-xylene, p-xylene, cumene, chlorobenzene, and ethylbenzene, aliphatic hydrocarbons, such as pentane, hexane, heptane, octane, nonane, decane, and the like, halogenated hydrocarbons, such as methylene chloride, alicyclic hydrocarbons, such as cyclopentane, methyl cyclopentane, cyclohexane, cycloheptane, cyclooctane, cyclononane, cyclodecane, and the like, and mixtures thereof. Suitable organic solvents include acetone, cyclohexanone, tetrahydrofuran, dioxane, glycols and glycol derivatives, polyalkylene glycols and derivatives thereof, diethyl ether, tert-butyl methyl ether, methyl acetate, ethyl acetate, propyl acetate, isopropyl acetate, butyl acetate, butyl propionate, and mixtures thereof. Mixtures of hydrocarbon solvents and organic solvents are also useful.

In the dispersion polymerization process, it can be advantageous to stabilize the monomer/polymer droplets or particles by means of surface active auxiliaries. Typically, these are emulsifiers, protective colloids or dispersion stabilizing polymers. The surface active auxiliaries used can be anionic, nonionic, cationic or amphoteric. Examples of anionic emulsifiers are alkylbenzenesulfonic acids, sulfonated fatty acids, sulfosuccinates, fatty alcohol sulfates, alkylphenol sulfates and fatty alcohol ether sulfates. Examples of usable nonionic emulsifiers are alkylphenol ethoxylates, primary alcohol ethoxylates, fatty acid ethoxylates, alkanolamide ethoxylates, fatty amine ethoxylates, EO/PO block copolymers and alkylpolyglucosides. Examples of cationic and amphoteric emulsifiers used are quaternized amine alkoxylates, alkylbetaines, alkylamidobetaines and sulfobetaines.

Examples of typical protective colloids are cellulose derivatives, polyethylene glycol, polypropylene glycol, copolymers of ethylene glycol and propylene glycol, polyvinyl acetate, poly(vinyl alcohol), partially hydrolyzed poly(vinyl alcohol), polyvinyl ether, starch and starch derivatives, dextran, polyvinylpyrrolidone, polyvinylpyridine, polyethyleneimine, polyvinylimidazole, polyvinylsuccinimide, polyvinyl-2-methylsuccinimide, polyvinyl-1,3-oxazolid-2-one, polyvinyl-2-methylimidazoline and maleic acid or anhydride copolymers. The emulsifiers or protective colloids are customarily used in concentrations from 0.05 to 20 wt.%, based on the weight of the total monomers.

The polymerization can be carried out the presence of chain transfer agents. Suitable chain transfer agents include, but are not limited to, thio- and disulfide containing compounds, such as C₁-C₁₈ alkyl mercaptans, such as tert-butyl mercaptan, n-octyl mercaptan, n-dodecyl mercaptan, tert-dodecyl mercaptan hexadecyl mercaptan, octadecyl mercaptan; mercaptoalcohols, such as 2-mercaptoethanol, 2-mercaptopropanol; mercaptocarboxylic acids, such as mercaptoacetic acid and 3-mercaptopropionic acid; mercaptocarboxylic acid esters, such as butyl thioglycolate, isooctyl thioglycolate, dodecyl thioglycolate, isooctyl 3-mercaptopropionate, and butyl 3-mercaptopropionate; thioesters; C₁-C₁₈ alkyl disulfides; aryldisulfides; polyfunctional thiols such as trimethylolpropane-tris-(3-mercaptopropionate), pentaerythritol-tetra-(3-mercaptopropionate), pentaerythritol-tetra-(thioglycolate), pentaerythritol-tetra-(thiolactate), dipentaerythritol-hexa-(thioglycolate), and the like; phosphites and hypophosphites; C₁-C₄ aldehydes, such as formaldehyde, acetaldehyde, propionaldehyde; haloalkyl compounds, such as carbon tetrachloride, bromotrichloromethane, and the like; hydroxylammonium salts such as hydroxylammonium sulfate; formic acid; sodium bisulfite; isopropanol; and catalytic chain transfer agents such as, for example, cobalt complexes (e.g., cobalt (II) chelates).

The chain transfer agents are generally used in amounts ranging from 0.1 to 10 wt.%, based on the total weight of the monomers present in the polymerization medium.

In another aspect of the invention, the nonionic, amphiphilic polymer is obtained by free-radical mediated dispersion polymerization in a non-aqueous medium that is a solvent for the monomers but a substantially nonsolvent for the resulting polymers. Non-aqueous dispersion polymerization is discussed in detail in the book Dispersion Polymerization in Organic Media, edited by K. E. G. Barrett and published by John Wiley & Sons, New York, 1975. In a typical procedure for preparing a dispersion polymer, an organic solvent containing the polymerizable monomers, any polymerization additives such as processing aids, chelants, pH buffers and a stabilizer polymer is charged to an oxygen purged, temperature controlled reactor equipped with a mixer, a thermocouple, a nitrogen purging tube, and a reflux condenser. The reaction medium is mixed vigorously, heated to the desired temperature, and then a free-radical initiator is added. The polymerization is usually conducted at reflux temperature to prevent oxygen from inhibiting the reaction. Reflux temperature typically falls in the range of from 40°C to 200°C in one aspect, and from 60°C to 140°C in another aspect, depending on the boiling point of the solvents comprising the non-aqueous medium in which the polymer is prepared. The reaction medium is continually purged with nitrogen while maintaining temperature and mixing for several hours. After this time, the mixture is cooled to room temperature, and any post-polymerization additives are charged to the reactor. Hydrocarbons are preferably used as the dispersion solvent. The reaction time required in such a polymerization will vary with the reaction temperature employed, initiator system, and initiator level. Generally, this reaction time will vary from 20 minutes up to 30 hours. Commonly, it will be preferred to utilize a reaction time from 1 up to 6 hours.

Typically, polymerization of the monomers used to prepare the polymers is initiated by free-radical initiators that are soluble in the non-aqueous medium. Examples include azo compound initiators such as 2,2'-azobis (2,4-dimethylpentanenitrile), 2,2'-azobis (2-methylbutanenitrile), and 2,2'-azobis(2-methylbutyronitrile). The initiators can be used in customary amounts, for example 0.05 to 7 wt.%, based on the amount of monomers to be polymerized.

In one aspect, the solvent is a hydrocarbon selected from aliphatic and cycloaliphatic solvents, as well as mixtures thereof. Exemplary hydrocarbon solvents include pentane, hexane, heptane, octane, nonane, decane, cyclopentane, methyl cyclopentane, cyclohexane, cycloheptane, cyclooctane, cyclononane, cyclodecane, and their mixtures.

In another aspect, the solvent is an organic solvent selected from acetone, cyclohexanone, tetrahydrofuran, dioxane, glycols and glycol derivatives, polyalkylene glycols and derivatives thereof, diethyl ether, tert-butyl methyl ether, methyl acetate, methyl acetate, ethyl acetate, propyl acetate, isopropyl acetate, butyl acetate, butyl propionate, ethanol, isopropanol, water, and mixtures thereof.

The amount of solvent utilized normally will be in excess of the monomers to be polymerized and the proportion can vary from at least 1 wt.% of the monomer components and 99 wt.% solvent, up to 65 wt.% polymerizable monomer components and 35 wt.% solvent. In another aspect, a concentration of 10 to 60 wt.% polymerizable monomer components can be employed, where the weight percent is based on the total amount of monomer and solvent charged to the reaction vessel.

When mixtures of organic solvents and hydrocarbon solvents are utilized, the organic solvents and the hydrocarbon solvents can be premixed or can be added separately to the reaction mixture and the polymerization reaction can be carried out thereafter. The relative weight ratio of the at least one organic solvent to at the least one hydrocarbon solvent can be in the range of from 95/5 to 1/99 in one aspect, from 80/20 to 5/95 in another aspect, and from 2:1 to 1:2 in a further aspect.

In one aspect the ratio of hydrocarbon solvent to organic solvent is 70/30 wt./wt. In one aspect the hydrocarbon solvent is selected from cyclohexane and the organic solvent is selected from ethyl acetate.

The stabilizer, typically a block or graft copolymer, prevents settling of the desired solid polymer product produced during the reaction. The block copolymer dispersion stabilizer can be selected from a variety of polymers containing at least two blocks wherein at least one of said blocks ("A" block) is soluble in the dispersion medium and at least another of said blocks ("B" block) is insoluble in the dispersion medium, and the stabilizer acts to disperse polymer products which are formed in the stabilizer's presence. The insoluble "B" block provides an anchor segment for attachment to the obtained polymer product, thus reducing the solubility of the polymerized product in the dispersion medium. The soluble "A" block of the dispersion stabilizer provides a sheath around the otherwise insoluble polymer and maintains the polymeric product as numerous small discrete particles rather than an agglomerated or highly coalesced mass. Details of the mechanism of such steric stabilization are described in Napper, D.H., "Polymeric Stabilization of Colloidal Dispersions," Academic Press, New York, N.Y., 1983. Representative stabilizers useful in the dispersion polymerization process of the invention are disclosed in U.S. Patent Nos. 4,375,533; 4,419,502; 4,526,937; 4,692,502; 5,288,814; 5,349,030; 5,373,044; 5,468,797; and 6,538,067.

In one aspect of the invention, the steric stabilizer is selected from poly(12-hydroxystearic acid) such as disclosed in U.S. Patent No. 5,288,814. In another aspect of the invention, the steric stabilizer comprises the ester of the reaction product of a C₁₈-C₂₄ hydrocarbyl substituted succinic acid or the anhydride thereof with a polyol such as disclosed in U.S. Patent No. 7,044,988. In another aspect, the steric stabilizer comprises the ester of the reaction product of a C₂₀ to C₂₄ alkyl substituted succinic anhydride and a polyol selected from glycerin and/or a polyglycerol containing 2 to 6 glycerin units.

In still another aspect, the steric stabilizer is a copolymer of N-vinyl pyrrolidone/stearyl methacrylate/butyl acrylate. In one aspect the comonomers are incorporated into the stabilizer polymer in a weight ratio of 50/30/20, respectively. Mixtures of this steric stabilizer with esters and half esters of the reaction product of the C₁₂ to C₃₀ alkenyl substituted succinic anhydride and a polyol selected from C₂ to C₄ glycols are also contemplated.

The amount of steric stabilizer used in the polymerization process of this invention will cause variations in the size and specific surface area of the disperse polymer. In general, the amount of stabilizer utilized can range from 0.1 to 10 wt.% based on the dry polymer weight. Of course, smaller particles of disperse polymer require more stabilizer than large particles of disperse polymer.

In one feature, the nonionic, amphiphilic polymer suitable for use in the compositions of the invention is selected from a dispersion polymer that is prepared from a polymerizable monomer mixture comprising a combination of at least one vinyl lactam, at least one vinyl ester of an aliphatic carboxylic acid containing an acyl moiety having 2 to 22 carbon atoms, and an optional monomer selected from at least one crosslinking monomer, at least one C₁-C₃₀ alkyl ester of (meth)acrylic acid, at least one alkoxylated associative monomer, at least one alkoxylated semi-hydrophobic monomer, and mixtures thereof.

The amount of the at least one vinyl lactam monomer in the monomer mixture ranges from 55 to 95 wt.% in one aspect, from 60 to 90 wt.% in another aspect, from 65 to 85 wt.% in a further aspect, and from 70 to 80 wt.% in a still further aspect, all weight percentages are based on the total weight of the monomers in the monomer mixture. In one aspect, the at least one vinyl lactam monomer is selected from N-vinyl pyrrolidone.

The amount of the at least one vinyl ester of an aliphatic carboxylic acid containing an acyl moiety having 2 to 22 carbon atoms in the monomer mixture ranges from 5 to 45 wt.% in one aspect, from 10 to 40 wt.% in another aspect, from 15 to 35 wt.% in a further aspect, and from 20 to 30 wt.% in a still further aspect, all weight percentages are based on the total weight of the monomers in the monomer mixture. In one aspect the at least one vinyl ester of an aliphatic carboxylic acid containing an acyl moiety having 2 to 22 carbon atoms is selected from vinyl acetate.

The amount of the at least one crosslinking monomer present in the monomer mixture ranges from 0 to about 1 wt.% in one aspect, from 0.01 to 0.75 wt.% in another aspect, from 0.1 to 0.5 in still another aspect, and from 0.15 to 0.3 wt.% in a still further aspect, all weight percentages are based on the dry weight of the nonionic, amphiphilic polymer of the invention. In one aspect, the crosslinking monomer is selected from trimethylolpropane tri(meth)acrylate, trimethylolethane tri(meth)acrylate, tetramethylolmethane tri(meth)acrylate, pentaerythritol triallylether and polyallyl ethers of sucrose having 3 allyl groups per molecule.

The amount of the at least one C₁-C₃₀ alkyl ester of (meth)acrylic acid monomer ranges 0 to 10 wt.% in one aspect, from 0.1 to 5 wt.% in another aspect, from 0.5 3 wt.% in a further aspect, and from 0.75 to 1 wt.% in a still further aspect, all weight percentages are based on the total weight of the monomers in the monomer mixture. In one feature of the invention a suitable monomer is selected from at least one C₁ to C₂₂ alkyl ester of (meth)acrylic acid. In another feature a suitable monomer is selected from a C₁₀ to C₂₂ alkyl ester of (meth)acrylic acid. Exemplary monomers include methyl (meth)acrylate, ethyl (meth)acrylate, butyl (meth)acrylate, sec-butyl (meth)acrylate, iso-butyl (meth)acrylate, hexyl (meth)acrylate), heptyl (meth)acrylate, octyl (meth)acrylate, 2-ethylhexyl (meth)acrylate, decyl (meth)acrylate, isodecyl (meth), lauryl (meth)acrylate, tetradecyl (meth)acrylate, hexadecyl (meth)acrylate, stearyl (meth)acrylate, behenyl (meth)acrylate, and mixtures thereof.

The amount of the at least one alkoxylated associative monomer ranges from 0 to 8 wt.% in one aspect, and from 0.5, 1, 2 or 3 to 5 wt.% in a further aspect, based on the weight of the total monomers in the monomer mixture. In one aspect, the at least one alkoxylated associative monomer is selected from lauryl polyethoxylated methacrylate (LEM), cetyl polyethoxylated methacrylate (CEM), cetearyl polyethoxylated methacrylate (CSEM), stearyl polyethoxylated (meth)acrylate, arachidyl polyethoxylated (meth)acrylate, behenyl polyethoxylated methacrylate (BEM), or mixtures thereof, wherein the amount of ethoxylation ranges from 5 to 60 ethylene oxide units.

The amount of the at least one alkoxylated semi-hydrophobic monomer in the monomer mixture ranges from 0 to 10 wt.% in one aspect, and from 0.5, 1, 2 or 3 to 5 wt.% in a further aspect, based on the weight of the total monomers in the monomer mixture. In one aspect a suitable alkoxylated semi-hydrophobic monomer is selected from at least one monomer conforming to formulas VIIIA and VIIIB described above.

The weight percentages of the monomers set forth above and throughout the specification that are contained in the polymerizable monomer mixture are selected from the disclosed ranges such that the sum of the total amount of monomers in the monomer mixture is 100 wt.%.

The cleansing compositions of the invention comprise at least one detersive surfactant, at least one silicone conditioning agent, at least one nonionic amphiphilic polymer which mitigates the loss of silicone deposition on keratinous substrates, water, and optional adjuvants and additives known in the personal care formulation art.

In one general aspect of the invention the nonionic, amphiphilic polymer component of the cleansing composition is prepared by polymerizing a monomer mixture comprising:
a) from 55 to 95 wt.% N-vinyl pyrrolidone;
b) from 5 to 45 wt.% of vinyl acetate;
c) from 0.01 or 0.1, or 0.15, or 0.3, or 0.75 to 1 wt.%, of at least one above-mentioned polyunsaturated crosslinking monomer containing at least two polymerizable ethylenically unsaturated moieties;
d) from about 0 or 0.5, 1, 2 or 3 to about 5 wt.% of at least one C₁ to C₂₂ alkyl ester of (meth)acrylic acid;
e) from 0 or 0.5, 1, 2 or 3 to 5 wt.% of at least one alkoxylated associative monomer;
f) from 0 or 0.5, 1, 2 or 3 to 5 wt.% of at least one alkoxylated semi-hydrophobic monomer;
g) from 0 or 0.5, 1, 2 or 3 to 5 wt.% of a vinyl ester of an aliphatic carboxylic acid containing an acyl moiety having 2 to 22 carbon atoms other than vinyl acetate; and combinations of monomers c) through g).

### Detersive Compositions

Surprisingly, the present nonionic, amphiphilic, polymers can be activated by a surfactant to provide a stable yield stress cleansing composition with desirable rheological and aesthetic properties and the ability to suspend particulate and insoluble materials in an aqueous medium for indefinite periods of time independent of pH. The yield stress value, elastic modulus and optical clarity are substantially independent of pH in the compositions in which the present polymers are included. The nonionic, amphiphilic, polymers of the invention are useful in the pH range of from 2 to 14 in one aspect, from 3 to 11 in another aspect, and from 4 to 9 in a further aspect. Unlike the pH responsive crosslinked polymers (acid or base sensitive) that require neutralization with an acid or a base to impart a desired rheological profile, the rheological profiles of the the crosslinked, nonionic, amphiphilic polymers of the invention are substantially independent of pH. By substantially independent of pH is meant that the surfactant containing composition within which the polymer of the invention is included imparts a desired rheological profile (e.g., a yield stress of at least 0.1 Pa in one aspect, at least at least 0.5 Pa in another aspect, at least 1 Pa in still another aspect, and at least 2 Pa in a further aspect) across a wide pH range (e.g., from 2 to 14) wherein the standard deviation in yield stress values across the pH range is less than 1 Pa in one aspect, less than 0.5 Pa in another aspect, and less than 0.25 Pa in a further aspect of the invention.

Suitable anionic detersive surfactant components for use in the hair conditioning shampoo composition include those which are known for use in hair care or other personal care cleansing compositions.

In one exemplary aspect of the invention, the cleansing compositions comprise: i) at least one nonionic, amphiphilic, polymer of the invention; ii) at least one surfactant selected from at least one anionic surfactant, at least one amphoteric surfactant, at least one nonionic surfactant, and combinations thereof; iii) a silicone conditioning agent; and iv) water.

In another exemplary aspect of the invention, the cleansing compositions comprise: i) at least one nonionic, amphiphilic polymer of the invention; ii) at least one anionic surfactant; iii) at least one silicone conditioning agent; and iv) water.

In another exemplary aspect of the invention, the cleansing compositions comprise: i) at least one nonionic, amphiphilic, polymer of the invention; ii) at least one anionic surfactant and at least one amphoteric surfactant; iii) at least one silicone conditioning agent; and iv) water.

In another exemplary aspect of the invention, the cleansing compositions comprise: i) at least one nonionic, amphiphilic polymer of the invention; ii) at least one anionic surfactant, iii) at least one nonionic surfactant; iv) a silicone conditioning agent; and v) water.

In another exemplary aspect of the invention, the cleansing compositions comprise: i) at least one nonionic, amphiphilic polymer of the invention; ii) at least one anionic surfactant, iii) at least one amphoteric surfactant; iv) at least one nonionic surfactant; v) at least one silicone conditioning agent; and vi) water.

In another exemplary aspect of the invention, the cleansing compositions comprise: i) at least one nonionic, amphiphilic polymer of the invention; ii) at least one anionic ethoxylated surfactant; iii) an optional nonionic surfactant; iv) at least one silicone conditioning agent and v) water. In one aspect, the average degree of ethoxylation of the anionic ethoxylated surfactant can range from 1 to 3. In another aspect, the average degree of ethoxylation is 2.

In another exemplary aspect of the invention, the cleansing compositions comprise: i) at least one crosslinked, nonionic, amphiphilic polymer of the invention; ii) at least one anionic ethoxylated surfactant; iii) at least one amphoteric surfactant; iv) at least one silicone conditioning agent; v) an optional nonionic surfactant; and vi) water. In one aspect, the average degree of ethoxylation of the anionic ethoxylated surfactant can range from 1 to 3. In another aspect, the average degree of ethoxylation is 2.

In another exemplary aspect of the invention, the cleansing compositions comprise: i) at least one nonionic, amphiphilic polymer of the invention; ii) at least one anionic non-ethoxylated surfactant; iii) at least one anionic ethoxylated surfactant; iv) an optional nonionic surfactant; v) at least one silicone conditioning agent; and vi) water. In one aspect, the average degree of ethoxylation of the anionic ethoxylated surfactant can range from 1 to 3. In another aspect, the average degree of ethoxylation is 2.

In another exemplary aspect of the invention, the cleansing compositions comprise: i) at least one nonionic, amphiphilic polymer of the invention; ii) at least one anionic non-ethoxylated surfactant; iii) at least one anionic ethoxylated surfactant; iv) at least one amphoteric surfactant; v) an optional nonionic surfactant; vi) at least one silicone conditioning agent; and vii) water. In one aspect, the average degree of ethoxylation in the anionic ethoxylated surfactant can range from 1 to 3. In another aspect, the average degree of ethoxylation is 2.

In one aspect, the amount of nonionic, amphiphilic polymer that can be incorporated into the surfactant containing cleansing compositions of the invention ranges from 0.5 to 5 wt.% polymer solids (100 % active polymer) based on the weight of the total composition. In another aspect, the amount of polymer utilized in the formulation ranges from 0.75 wt.% to 3.5 wt.%. In still another aspect, the amount of amphiphilic polymer employed in the cleansing composition ranges from 1 to 3 wt.%. In a further aspect, the amount of polymer employed in the cleansing composition ranges from 1.5 wt.% to 2.75 wt.%. In a still further aspect, the amount of polymer utilized in the cleansing composition ranges from 2 to 2.5 wt.%.

In one aspect, the at least one nonionic, amphiphilic polymer utilized in formulating the cleansing compositions of the invention is linear. In one aspect, the number average molecular weight (Mₙ) of the linear copolymeric mitigants of the present invention as measured by gel permeation chromatography (GPC) calibrated with a poly(methyl methacrylate) (PMMA) standard is 500,000 daltons or less. In another aspect the molecular weight is 100,000 daltons or less. In still another aspect, the molecular weight ranges between 5,000 and 80,000 daltons, in a further aspect between 10,000 and 50,000 daltons, and in a still further aspect between 15,000 and 40,000 daltons.

In another aspect, the at least one nonionic, amphiphilic polymer utilized in formulating the mild cleansing compositions of the invention is crosslinked. The crosslinked nonionic, amphiphilic polymers of the invention are random copolymers and have weight average molecular weights ranging from above 500,000 to at least about a 4.5 billion Daltons or more in one aspect, and from 600,000 to about 1 billion Daltons in another aspect, and from 1,000,000 to 3,000,000 Daltons in a further aspect, and from 1,500,000 to 2,000,000 Daltons in a still further aspect (see TDS-222, October 15, 2007, Lubrizol Advanced Materials, Inc.).

### Detersive Surfactants

The surfactants utilized to formulate the cleansing and conditioning compositions of the invention are chosen from at least one detersive surfactant selected from anionic surfactants, amphoteric surfactants, nonionic surfactants, and mixtures thereof.

Non-limiting examples of anionic surfactants are disclosed in McCutcheon's Detergents and Emulsifiers, North American Edition, 1998, published by Allured Publishing Corporation; and McCutcheon's, Functional Materials, North American Edition (1992). The anionic surfactant can be any of the anionic surfactants known or previously used in the art of aqueous surfactant compositions. Suitable anionic surfactants include but are not limited to alkyl sulfates, alkyl ether sulfates, alkyl sulfonates, alkaryl sulfonates, α-olefin-sulfonates, alkylamide sulfonates, alkarylpolyether sulphates, alkylamidoether sulphates, alkyl monoglyceryl ether sulfates, alkyl monoglyceride sulfates, alkyl monoglyceride sulfonates, alkyl succinates, alkyl sulfosuccinates, alkyl sulfosuccinamates, alkyl ether sulphosuccinates, alkyl amidosulfosuccinates; alkyl sulphoacetates, alkyl phosphates, alkyl ether phosphates, alkyl ether carboxylates, alkyl amidoethercarboxylates, N-alkylamino acids, N-acyl amino acids, alkyl peptides, N-acyl taurates, alkyl isethionates, carboxylate salts wherein the acyl group is derived from fatty acids; and the alkali metal, alkaline earth metal, ammonium, amine, and triethanolamine salts thereof.

In one aspect, the cation moiety of the forgoing salts is selected from sodium, potassium, magnesium, ammonium, mono-, di- and triethanolamine salts, and mono-, di-, and tri-isopropylamine salts. The alkyl and acyl groups of the foregoing surfactants contain from 6 to 24 carbon atoms in one aspect, from 8 to 22 carbon atoms in another aspect and from 12 to 18 carbon atoms in a further aspect and can be saturated or unsaturated. The aryl groups in the surfactants are selected from phenyl or benzyl. The ether containing surfactants set forth above can contain from 1 to 10 ethylene oxide and/or propylene oxide units per surfactant molecule in one aspect, and from 1 to 3 ethylene oxide units per surfactant molecule in another aspect.

Examples of suitable anionic surfactants include but are not limited to the sodium, potassium, lithium, magnesium, and ammonium salts of laureth sulfate, trideceth sulfate, myreth sulfate, C₁₂-C₁₃ pareth sulfate, C₁₂-C₁₄ pareth sulfate, and C₁₂-C₁₅ pareth sulfate, ethoxylated with 1, 2, 3, 4 or 5 moles of ethylene oxide; sodium, potassium, lithium, magnesium, ammonium, and triethanolamine lauryl sulfate, coco sulfate, tridecyl sulfate, myrstyl sulfate, cetyl sulfate, cetearyl sulfate, stearyl sulfate, oleyl sulfate, and tallow sulfate, disodium lauryl sulfosuccinate, disodium laureth sulfosuccinate, sodium cocoyl isethionate, sodium C₁₂-C₁₄ olefin sulfonate, sodium laureth-6 carboxylate, sodium methyl cocoyl taurate, sodium cocoyl glycinate, sodium myristyl sarcocinate, sodium dodecylbenzene sulfonate, sodium cocoyl sarcosinate, sodium cocoyl glutamate, potassium myristoyl glutamate, triethanolamine monolauryl phosphate, and fatty acid soaps, including the sodium, potassium, ammonium, and triethanolamine salts of a saturated and unsaturated fatty acids containing from 8 to 22 carbon atoms.

The term "amphoteric surfactant" as used herein, is also intended to encompass zwitterionic surfactants, which are well known to formulators skilled in the art as a subset of amphoteric surfactants. Non-limiting examples of amphoteric surfactants are disclosed *McCutcheon's Detergents and Emulsifiers,* North American Edition, supra, and McCutcheon's, *Functional Materials,* North American Edition, supra. Suitable examples include but are not limited to amino acids (e.g., N-alkyl amino acids and N-acyl amino acids), betaines, sultaines, and alkyl amphocarboxylates.

Amino acid based surfactants suitable in the practice of the present invention include surfactants represented by the formula: 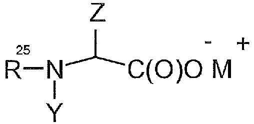 wherein R²⁵ represents a saturated or unsaturated hydrocarbon group having 10 to 22 carbon atoms or an acyl group containing a saturated or unsaturated hydrocarbon group having 9 to 22 carbon atoms, Y is hydrogen or methyl, Z is selected from hydrogen, -CH₃, -CH(CH₃)₂, -CH₂CH(CH₃)₂, -CH(CH₃)CH₂CH₃, - CH₂C₆H₅, -CH₂C₆H₄OH, -CH₂OH, -CH(OH)CH₃, -(CH₂)₄NH₂, -(CH₂)₃NHC(NH)NH₂, -CH₂C(O)O⁻M⁺, -(CH₂)₂C(O)O⁻M⁺. M is a salt forming cation. In one aspect, R²⁵ represents a radical selected from a linear or branched C₁₀ to C₂₂ alkyl group, a linear or branched C₁₀ to C₂₂ alkenyl group, an acyl group represented by R²⁶C(O)-, wherein R²⁶ is selected from a linear or branched C₉ to C₂₂ alkyl group, a linear or branched C₉ to C₂₂ alkenyl group. In one aspect, M⁺ is a cation selected from sodium, potassium, ammonium, and the ammonium salt of mono-, di-, and triethanolamine (TEA).

The amino acid surfactants can be derived from the alkylation and acylation of α-amino acids such as, for example, alanine, arginine, aspartic acid, glutamic acid, glycine, isoleucine, leucine, lysine, phenylalanine, serine, tyrosine, and valine. Representative N-acyl amino acid surfactants are, but not limited to the mono- and di- carboxylate salts (e.g., sodium, potassium, ammonium and TEA) of N-acylated glutamic acid, for example, sodium cocoyl glutamate, sodium lauroyl glutamate, sodium myristoyl glutamate, sodium palmitoyl glutamate, sodium stearoyl glutamate, disodium cocoyl glutamate, disodium stearoyl glutamate, potassium cocoyl glutamate, potassium lauroyl glutamate, and potassium myristoyl glutamate; the carboxylate salts (e.g., sodium, potassium, ammonium and TEA) of N-acylated alanine, for example, sodium cocoyl alaninate, and TEA lauroyl alaninate; the carboxylate salts (e.g., sodium, potassium, ammonium and TEA) of N-acylated glycine, for example, sodium cocoyl glycinate, and potassium cocoyl glycinate; the carboxylate salts (e.g., sodium, potassium, ammonium and TEA) of N-acylated sarcosine, for example, sodium lauroyl sarcosinate, sodium cocoyl sarcosinate, sodium myristoyl sarcosinate, sodium oleoyl sarcosinate, and ammonium lauroyl sarcosinate; and mixtures of the foregoing surfactants.

The betaines and sultaines useful in the present invention are selected from alkyl betaines, alkylamino betaines, and alkylamido betaines, as well as the corresponding sulfobetaines (sultaines) represented by the formulas: 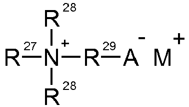 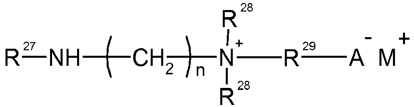 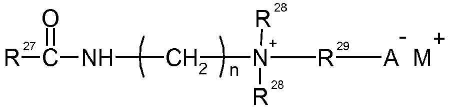 wherein R²⁷ is a C₇-C₂₂ alkyl or alkenyl group, each R²⁸ independently is a C₁-C₄ alkyl group, R²⁹ is a C₁-C₅ alkylene group or a hydroxy substituted C₁-C₅ alkylene group, n is an integer from 2 to 6, A is a carboxylate or sulfonate group, and M is a salt forming cation. In one aspect, R²⁷ is a C₁₁-C₁₈ alkyl group or a C₁₁-C₁₈ alkenyl group. In one aspect, R²⁸ is methyl. In one aspect, R²⁹ is methylene, ethylene or hydroxy propylene. In one aspect, n is 3. In a further aspect, M is selected from sodium, potassium, magnesium, ammonium, and mono-, di- and triethanolamine cations.

Examples of suitable betaines include, but are not limited to, lauryl betaine, coco betaine, oleyl betaine, cocohexadecyl dimethylbetaine, lauryl amidopropyl betaine, cocoamidopropyl betaine (CAPB), and cocamidopropyl hydroxysultaine.

The alkylamphocarboxylates such as the alkylamphoacetates and alkylamphopropionates (mono- and disubstituted carboxylates) can be represented by the formula: 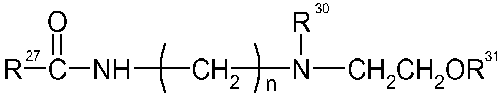 wherein R²⁷ is a C₇-C₂₂ alkyl or alkenyl group, R³⁰ is -CH₂C(O)O⁻M⁺,-CH₂CH₂C(O)O⁻M⁺, or -CH₂CH(OH)CH₂SO₃⁻M⁺, R³¹ is hydrogen or -CH₂C(O)O⁻ M⁺, and M is a cation selected from sodium, potassium, magnesium, ammonium, and ammonium salts of mono-, di- and triethanolamine.

Exemplary alkylamphocarboxylates include, but are not limited to, sodium cocoamphoacetate, sodium lauroamphoacetate, sodium capryloamphoacetate, disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, and disodium capryloamphodipropionate.

Non-limiting examples of nonionic surfactants are disclosed in *McCutcheon's Detergents and Emulsifiers,* North American Edition, 1998, supra; and McCutcheon's, *Functional Materials,* North American, supra. Additional Examples of nonionic surfactants are described in U.S. Patent No. 4,285,841, to Barrat et al., and U.S. Patent. No. 4,284,532, to Leikhim et al.,. Nonionic surfactants typically have a hydrophobic portion, such as a long chain alkyl group or an alkylated aryl group, and a hydrophilic portion containing various degrees of ethoxylation and/or propoxylation (e.g., 1 to 50) ethoxy and/or propoxy moieties. Examples of some classes of nonionic surfactants that can be used include, but are not limited to, ethoxylated alkylphenols, ethoxylated and propoxylated fatty alcohols, polyethylene glycol ethers of methyl glucose, polyethylene glycol ethers of sorbitol, ethylene oxide-propylene oxide block copolymers, ethoxylated esters of fatty acids, condensation products of ethylene oxide with long chain amines or amides, condensation products of ethylene oxide with alcohols, and mixtures thereof.

Suitable nonionic surfactants include, for example, alkyl polysaccharides, alcohol ethoxylates, block copolymers, castor oil ethoxylates, ceto/oleyl alcohol ethoxylates, cetearyl alcohol ethoxylates, decyl alcohol ethoxylates, dinonyl phenol ethoxylates, dodecyl phenol ethoxylates, endcapped ethoxylates, ether amine derivatives, ethoxylated alkanolamides, ethylene glycol esters, fatty acid alkanolamides, fatty alcohol alkoxylates, lauryl alcohol ethoxylates, mono-branched alcohol ethoxylates, nonyl phenol ethoxylates, octyl phenol ethoxylates, oleyl amine ethoxylates, random copolymer alkoxylates, sorbitan ester ethoxylates, stearic acid ethoxylates, stearyl amine ethoxylates, tallow oil fatty acid ethoxylates, tallow amine ethoxylates, tridecanol ethoxylates, acetylenic diols, polyoxyethylene sorbitols, and mixtures thereof. Various specific examples of suitable nonionic surfactants include, but are not limited to, methyl gluceth-10, PEG-20 methyl glucose distearate, PEG-20 methyl glucose sesquistearate, ceteth-8, ceteth-12, dodoxynol-12, laureth-15, PEG-20 castor oil, polysorbate 20, steareth-20, polyoxyethylene-10 cetyl ether, polyoxyethylene-10 stearyl ether, polyoxyethylene-20 cetyl ether, polyoxyethylene- 10 oleyl ether, polyoxyethylene-20 oleyl ether, an ethoxylated nonylphenol, ethoxylated octylphenol, ethoxylated dodecylphenol, or ethoxylated fatty (C₆-C₂₂) alcohol, including 3 to 20 ethylene oxide moieties, polyoxyethylene-20 isohexadecyl ether, polyoxyethylene-23 glycerol laurate, polyoxyethylene-20 glyceryl stearate, PPG-10 methyl glucose ether, PPG-20 methyl glucose ether, polyoxyethylene-20 sorbitan monoesters, polyoxyethylene-80 castor oil, polyoxyethylene-15 tridecyl ether, polyoxyethylene-6 tridecyl ether, laureth-2, laureth-3, laureth-4, PEG-3 castor oil, PEG 600 dioleate, PEG 400 dioleate, poloxamers such as poloxamer 188, polysorbate 21, polysorbate 40, polysorbate 60, polysorbate 61, polysorbate 65, polysorbate 80, polysorbate 81, polysorbate 85, sorbitan caprylate, sorbitan cocoate, sorbitan diisostearate, sorbitan dioleate, sorbitan distearate, sorbitan fatty acid ester, sorbitan isostearate, sorbitan laurate, sorbitan oleate, sorbitan palmitate, sorbitan sesquiisostearate, sorbitan sesquioleate, sorbitan sesquistearate, sorbitan stearate, sorbitan triisostearate, sorbitan trioleate, sorbitan tristearate, sorbitan undecylenate, or mixtures thereof.

Alkyl glycoside nonionic surfactants can also be employed and are generally prepared by reacting a monosaccharide, or a compound hydrolyzable to a monosaccharide, with an alcohol such as a fatty alcohol in an acid medium. For example, U.S. Patent Nos. 5,527,892 and 5,770,543 describe alkyl glycosides and/or methods for their preparation. Suitable examples are commercially available under the names of Glucopon™ 220, 225, 425, 600 and 625, PLANTACARE®, and PLANTAPON®, all of which are available from Cognis Corporation of Ambler, Pennsylvania.

In another aspect, nonionic surfactants include, but are not limited to, alkoxylated methyl glucosides such as, for example, methyl gluceth-10, methyl gluceth-20, PPG-10 methyl glucose ether, and PPG-20 methyl glucose ether, available from Lubrizol Advanced Materials, Inc., under the trade names, Glucam® E10, Glucam® E20, Glucam® P10, and Glucam® P20, respectively; and hydrophobically modified alkoxylated methyl glucosides, such as PEG 120 methyl glucose dioleate, PEG-120 methyl glucose trioleate, and PEG-20 methyl glucose sesquistearate, available from Lubrizol Advanced Materials, Inc., under the trade names, Glucamate® DOE-120, Glucamate™ LT, and Glucamate™ SSE-20, respectively, are also suitable. Other exemplary hydrophobically modified alkoxylated methyl glucosides are disclosed in U.S. Patent Nos. 6,573,375 and 6,727,357.

Other useful nonionic surfactants include water soluble silicones such as PEG-10 Dimethicone, PEG-12 Dimethicone, PEG-14 Dimethicone, PEG-17 Dimethicone, PPG-12 Dimethicone, PPG-17 Dimethicone and derivatized/functionalized forms thereof such as Bis-PEG/PPG-20/20 Dimethicone Bis-PEG/PPG-16/16 PEG/PPG-16/16 Dimethicone, PEG/PPG-14/4 Dimethicone, PEG/PPG-20/20 Dimethicone, PEG/PPG-20/23 Dimethicone, and Perfluorononylethyl Carboxydecyl PEG-10 Dimethicone.

Other useful nonionic surfactants include water soluble silicones such as PEG-10 Dimethicone, PEG-12 Dimethicone, PEG-14 Dimethicone, PEG-17 Dimethicone, PPG-12 Dimethicone, PPG-17 Dimethicone and derivatized/functionalized forms thereof such as Bis-PEG/PPG-20/20 Dimethicone Bis-PEG/PPG-16/16 PEG/PPG-16/16 Dimethicone, PEG/PPG-14/4 Dimethicone, PEG/PPG-20/20 Dimethicone, PEG/PPG-20/23 Dimethicone, and Perfluorononylethyl Carboxydecyl PEG-10 Dimethicone.

The amount of the at least one surfactant (active weight basis) utilized in formulating the cleansing compositions of the invention ranges from 1 to 30 wt.% based on the weight of the total composition. In another aspect, the amount of the at least one surfactant utilized in the formulation of the cleansing composition ranges from 3 to 25 wt.%. In still another aspect, the amount of the at least one surfactant employed in the cleansing composition ranges from 5 to 22 wt.%. In a further aspect, the amount of the at least one surfactant utilized ranges from 6 to 20 wt.%. In still a further aspect, the amount of at least one surfactant is 10, 12, 14, 16, and 18 wt.% based on the total weight of the cleansing composition.

In one embodiment of the invention, the weight ratio (based on active material) of anionic surfactant (non-ethoxylated and/or ethoxylated) to amphoteric surfactant can range from 10:1 to 2:1 in one aspect, and can be 9:1, 8:1, 7:1 6:1, 5:1, 4.5:1, 4:1, or 3:1 in another aspect. When employing an ethoxylated anionic surfactant in combination with a non-ethoxylated anionic surfactant and an amphoteric surfactant, the weight ratio (based on active material) of ethoxylated anionic surfactant to non-ethoxylated anionic surfactant to amphoteric surfactant can range from 3.5:3.5:1 in one aspect to 1:1:1 in another aspect.

In one embodiment, the yield stress value of the cleansing composition containing the linear, nonionic, amphiphilic polymers of the invention is about 0 Pa.

In one embodiment, the yield stress value of the cleansing composition containing the crosslinked nonionic, amphiphilic polymers of the invention is at least 0.1 Pa in one aspect, 0.5 Pa in one aspect, at least 1 Pa in another aspect and at least 1.5 Pa in a further aspect. In another embodiment, the yield stress of the cleansing composition ranges from 0.1 to 20 Pa in one aspect, from 0.5 Pa to 10 Pa in another aspect, from 1 to 3 Pa in a further aspect, and from 1.5 to 3.5 in a still further aspect.

Optionally, the cleansing and conditioning compositions of the invention can contain an electrolyte. Suitable electrolytes are known compounds and include salts of multivalent anions, such as potassium pyrophosphate, potassium tripolyphosphate, and sodium or potassium citrate, salts of multivalent cations, including alkaline earth metal salts such as calcium chloride and calcium bromide, as well as zinc halides, barium chloride and calcium nitrate, salts of monovalent cations with monovalent anions, including alkali metal or ammonium halides, such as potassium chloride, sodium chloride, potassium iodide, sodium bromide, and ammonium bromide, alkali metal or ammonium nitrates, and blends thereof. The amount of the electrolyte used will generally depend on the amount of the amphiphilic polymer incorporated, but may be used at concentration levels of from 0.1 to 4 wt.% in one aspect and from 0.2 to 2 wt.% in another aspect, based on the weight of the total composition.

The cleansing composition must be easily pourable with a shear thinning index of less than 0.5 at shear rates between 0.1 and 1 reciprocal second. The cleansing and compositions of the invention can be utilized in combination with an auxiliary rheology modifier (thickener) to enhance the yield value of a thickened liquid. In one aspect, the polymers of the invention can be combined with an auxiliary nonionic rheology modifier. In one aspect, an auxiliary nonionic rheology modifier to attain a desired yield stress value when a linear nonionic, amphiphilic polymer is utilized. Any rheology modifier is suitable including, but are not limited to, natural gums (e.g., polygalactomannan gums selected from fenugreek, cassia, locust bean, tara and guar), modified cellulose (e.g., ethylhexylethylcellulose (EHEC), hydroxybutylmethylcellulose (HBMC), hydroxyethylmethylcellulose (HEMC), hydroxypropylmethylcellulose (HPMC), methyl cellulose (MC), hydroxyethylcellulose (HEC), hydroxypropylcellulose (HPC) and cetyl hydroxyethylcellulose); and mixtures thereofmethylcellulose, polyethylene glycols (e.g., PEG 4000, PEG 6000, PEG 8000, PEG 10000, PEG 20000), polyvinyl alcohol, polyacrylamides (homopolymers and copolymers), and hydrophobically modified ethoxylated urethanes (HEUR). The rheology modifier can be utilized in an amount ranging from 0.5 to 25 wt.% in one aspect, from 1 to 15 wt.% in another aspect, and from 2 to 10 wt.% in a further aspect, and from 2.5 to 5 wt.% based on the weight of the total weight of the composition.

The linear and crosslinked, nonionic, amphiphilic polymers of the invention can be used in any cleansing or detersive application where the mitigation of silicone deposition loss is desired. The linear polymers of the invention can be utilized in any cleansing or detersive conditioning formulations where mitigation of the loss of silicone deposition is desirable but an increase in yield value or thickening is not. The crosslinked, nonionic, amphiphilic polymers of the invention can be used in any cleansing or detersive conditioning formulation to mitigate silicone deposition loss and which a concomitant enhancement of yield stress properties is desired.

### Silicone Conditioning Agents

The cleansing composition of the present invention further includes a silicone conditioning agent in the form of silicone particles or droplets. The silicone conditioning agent is intermixed in the composition so as to be in the form of dispersed, insoluble particles or droplets. In one aspect of the invention the silicone oil can be in the form of pre-formed emulsified droplets or microemulsions.

The silicone conditioning agent may comprise volatile silicones, non-volatile silicones, and mixtures thereof. If volatile silicones are present, they are typically employed as a solvent or carrier for commercially available forms of non-volatile silicone fluid conditioning agents such as oils and gums. Volatile silicone fluids are often included in the conditioning package to improve silicone fluid deposition efficacy or to enhance the shine, sheen or glossiness of the hair. Volatile silicone materials are frequently included in formulations to enhance sensory attributes (e.g., feel) on the scalp and skin.

In one aspect, the silicone conditioning agent is non-volatile and insoluble in the aqueous personal care cleansing composition and includes silicone oils, gums, resins and mixtures thereof. By non-volatile is meant that the silicone has a very low vapor pressure at ambient temperature conditions (e.g., less than 2 mm Hg at 20°C). The non-volatile silicone conditioning agent has a boiling point above 250°C in one aspect, above 260°C in another aspect, and above 275°C in a further aspect. Background information on silicones including sections discussing silicone oils, gums, and resins, as well as their manufacture, are found in Encyclopedia of Polymer Science and Engineering, vol. 15, 2d ed., pp 204-308, John Wiley & Sons, Inc. (1989).

The total concentration of silicone particles in the compositions of the present invention should be sufficient to provide the desired conditioning performance to the skin and hair, and generally ranges from 0.01 to 20 wt.% in one aspect, from 0.05 to 15 wt.% in another aspect, from 0.1 % to 10 wt.% in still another aspect, and from 1 to 5 wt.% in a further aspect, based on the weight of the total composition.

The silicones used in the present invention have an average particle size or droplet size ranging from 0.003 to 500 µm in a first aspect, from 0.05 to 200 µm in a second aspect, from 0.25 to 200 µm in a third aspect, from 0.5 to 150 µm in a fourth aspect, from 1 to 100 µm in a fifth aspect, from 5 to 80 µm in a sixth aspect, from 10 to 60 µm in an seventh aspect, and from 20 to 50 µm in an eighth aspect.

Silicone emulsions have an average silicone particle (droplet) size of less than 30 µm, less than 20 µm in another aspect, and less than 10 µm in a further aspect. In another aspect of the invention, the average silicone particle size of the silicone emulsion is less than 2 µm, and in another it ranges from 0.01 to 1 µm. Silicone emulsions having an average silicone particle (droplet) size of 0.15 µm or less are generally termed microemulsions and generally have an average particle size ranging from 0.003 to 0.15 µm.

The average particle size of the silicone conditioning agent particles can be measured by light scattering techniques well-known in the art for determining average particle size for emulsified liquids. One such method involves measuring particle size by means of a laser light scattering technique using a Horiba model LA 910 laser scattering particle size distribution analyzer (Horiba Instruments, Inc., Irvine, California).

### Silicone Oils

In one aspect, the silicone conditioning agent is silicone oil. In one aspect the silicone oil is a polyorganosiloxane material. The non-volatile silicone conditioning agents have a viscosity ranging from about above 25 to 1,000,000 mPa·s at 25°C in one aspect, from 100 to 600,000 mPa·s in another aspect, and from 1000 to 100,000 mPa·s still another aspect, from 2,000 to 50,000 mPa·s in yet another aspect, and from 4,000 to 40,000 mPa·s in a further aspect. The viscosity is measured by means of a glass capillary viscometer as described by Dow Corning Corporate Test Method CTM004, dated July 20, 1970. In one aspect the silicone oils have an average molecular weight below 200,000 daltons. The average molecular weight can typically range from 400 to 199,000 daltons in one aspect, from 500 to 150,000 daltons in another aspect, from 1,000 to 100,000 daltons in still another aspect, from 5,000 to 65,000 daltons in a further aspect.

In one aspect, silicone oils suitable as conditioning agents are polyorganosiloxane materials selected from polyalkylsiloxanes, polyarylsiloxanes, polyalkylarylsiloxanes, hydroxyl terminated polyalkylsiloxanes, polyarylalkylsiloxanes, amino functional polyalkylsiloxanes, quaternary functional polyalkylsiloxanes, and mixtures thereof.

In one aspect, the silicone conditioning agent includes polyorganosiloxanes represented by Formula X: 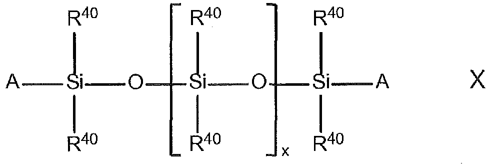 wherein A independently represents hydroxy, methyl, methoxy, ethoxy, propoxy, and phenoxy; R⁴⁰ independently represents methyl, ethyl, propyl, phenyl, methylphenyl, phenylmethyl, a primary, secondary or tertiary amine, a quaternary group selected from a group selected from:

-R⁴¹-N(R⁴²)CH₂CH₂N(R⁴²)₂;

-R⁴¹-N(R⁴²)₂;

-R⁴¹-N⁺(R⁴²)₃CA⁻;

and

-R⁴¹-N(R⁴²)CH₂CH₂N(R⁴²)H₂CA⁻

wherein R⁴¹ is a linear or branched, hydroxyl substituted or unsubstituted alkylene or alkylene ether moiety containing 2 to 10 carbon atoms; R⁴² is hydrogen, C₁-C₂₀ alkyl (e.g, methyl), phenyl or benzyl; q is an integer ranging from 2 to 8; CA⁻ is a halide ion selected from chlorine, bromine, iodine and fluorine; and x is an integer ranging from 7 to 8000 in one aspect, from 50 to 5000 in another aspect, form 100 to 3000 in still another aspect, and from 200 to 1000 in a further aspect.

In one aspect, the amino functional silicone is represented by Formula XA: 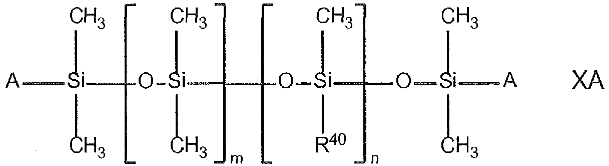 wherein A independently represents hydroxy, methyl, methoxy, ethoxy, propoxy, and phenoxy; and R⁴⁰ is selected from:

-R⁴¹-N(R⁴²)CH₂CH₂N(R⁴²)₂;

-R⁴¹-N(R⁴²)₂;

-R⁴¹-N⁺(R⁴²)₃CA⁻;

and

-R⁴¹-N(R⁴²)CH₂CH₂N(R⁴²)H₂CA⁻

wherein R⁴¹ is a linear or branched, hydroxyl substituted or unsubstituted alkylene or alkylene ether moiety containing 2 to 10 carbon atoms; R⁴² is hydrogen, C₁-C₂₀ alkyl (e.g, methyl), phenyl or benzyl; CA⁻ is a halide ion selected from chlorine, bromine, iodine and fluorine; and the sum of m+n ranges from 7 to 1000 in one aspect, from 50 to 250 in another aspect, and from 100 to 200 in another aspect, subject to the proviso that m or n is not 0. In one aspect A is hydroxy and R⁴⁰ is - (CH₂)₃NH(CH₂)₃NH₂. In another aspect A is methyl and R⁴⁰ is - (CH₂)₃NH(CH₂)₃NH₂. In still another aspect A is methyl and R⁴⁰ is a quaternary ammonium moiety represented by -(CH₂)₃OCH₂CH(OH)CH₂N⁺(R⁴²)₃CA⁻; wherein R⁴² and CA⁻ are as previously defined.

Exemplary silicone oil conditioning agents include, but are not limited to, polydimethylsiloxanes (dimethicones), polydiethylsiloxanes, polydimethyl siloxanes having terminal hydroxyl groups (dimethiconols), polymethylphenylsiloxanes, phenylmethylsiloxanes, amino functional polydimethylsiloxanes (amodimethicones), and mixtures thereof.

### Silicone Gums

Another silicone conditioning agent useful in the invention is a silicone gum. A silicone gum is a polyorganosiloxane material of the same general structure of the silicone oils set forth under Formula XII wherein A independently represents hydroxy, methyl, methoxy, ethoxy, propoxy, and phenoxy; R⁴⁰ independently represents methyl, ethyl, propyl, phenyl, methylphenyl, phenylmethyl, and vinyl. Silicone gums have a viscosity measured at 25°C of greater than 1,000,000 mPa·s. The viscosity can be measured by means of a glass capillary viscometer as described above for the silicone oils. In one aspect the silicone gums have an average molecular weight 200,000 daltons and above. The molecular weight can typically range from 200,000 to 1,000,000 daltons. It is recognized that the silicone gums described herein can also have some overlap with the silicone oils described previously. This overlap is not intended as a limitation on any of these materials.

Suitable silicone gums for use in the silicone component of compositions of the invention are polydimethylsiloxanes (dimethicones), optionally having terminal end groups such as hydroxyl (dimethiconols), polymethylvinylsiloxane, polydiphenylsiloxane, and mixtures thereof.

### Silicone Resins

Silicone resins can be included as a silicone conditioning agent suitable for use in the compositions of the present invention. These resins are crosslinked polysiloxanes. The crosslinking is introduced through the incorporation of trifunctional and tetrafunctional silanes with monofunctional and/or difunctional silanes during manufacture of the silicone resin. As is well understood in the art, the degree of crosslinking that is required in order to result in a silicone resin will vary according to the specific silane units incorporated into the silicone resin. In general, silicone materials which have a sufficient level of trifunctional and tetra-functional siloxane monomer units (and hence, a sufficient level of crosslinking) such that they form a rigid or hard film are considered to be silicone resins. The ratio of oxygen atoms to silicon atoms is indicative of the level of crosslinking in a particular silicone material. Silicone materials which have at least 1.1 oxygen atoms per silicon atom will generally be silicone resins herein. In one aspect, the ratio of oxygen:silicon atoms is at least 1.2:1.0. Silanes used in the manufacture of silicone resins include monomethyl-, dimethyl-, trimethyl-, monophenyl-, diphenyl-, methylphenyl-, monovinyl-, and methylvinyl-chlorosilanes, and terachlorosilane, with the methyl substituted silanes being most commonly utilized.

Silicone materials and silicone resins can be identified according to a shorthand nomenclature system known to those of ordinary skill in the art as "MDTQ" nomenclature. Under this naming system, the silicone is described according to the presence of various siloxane monomer units which make up the silicone. The "MDTQ" nomenclature system is described in the publication entitled *"Silicones: Preparation, Properties and Performance";* Dow Corning Corporation, 2005, and in U.S. Pat. No. 6,200,554.

Exemplary silicone resins for use in the compositions of the present invention include, but are not limited to MQ, MT, MTQ, MDT and MDTQ resins. In one aspect, methyl is the silicone resin substituent. In another aspect, the silicone resin is selected from a MQ resins, wherein the M:Q ratio is from 0.5:1.0 to 1.5:1.0 and the average molecular weight of the silicone resin is from 1000 to 10,000 daltons.

### Volatile Silicones

The optional volatile silicones referred to above include linear and cyclic polydimethylsiloxanes (cyclomethicones), and mixtures thereof. The term "volatile" means that the silicone has a measurable vapor pressure, or a vapor pressure of at least 267 Pa (2 mm of Hg) at 20°C. The volatile silicones have a viscosity of 25 mPa·s or less at 25°C in one aspect, from 0.65 to 10 mPa·s in another aspect, from 1 to 5 mPa·s in still another aspect, and from 1.5 to 3.5 mPa·s in a further aspect. A description of linear and cyclic volatile silicones is found in Todd and Byers, "Volatile Silicone Fluids for Cosmetics", Cosmetics and Toiletries, Vol. 91(1), pp. 27-32 (1976), and in Kasprzak, "Volatile Silicones", Soap/Cosmetics/Chemical Specialities, pp. 40-43 (December 1986).

The linear volatile silicones are silicone fluids, as described above in Formula XII but having viscosities of not more than 25 mPa·s. The cyclomethicones typically contain 3 to 7 dimethyl substituted silicon atoms in one aspect and from 3 to 5 in another aspect, alternating with oxygen atoms, in a cyclic ring structure.

### Water

The cleansing compositions of the invention are aqueous based systems comprising water as the carrier. The exact level of water will vary with the levels of the remaining components formulated into the composition. Generally, the cleansing compositions of the invention comprise from 10 to 95 wt.% in one aspect, from 50 to 92 wt.% in another aspect, and from 60 to 90 wt.% water in a further aspect.

In one embodiment, the crosslinked, nonionic, amphiphilic polymers of the invention can be utilized to mitigate silicone deposition loss as well as to stably suspend particulate materials and insoluble droplets within a surfactant containing cleansing and cleaning composition formulated for the personal care and home care industries.

In the personal care formulations, the crosslinked, nonionic, amphiphilic polymers of the invention can be utilized to mitigate silicone deposition loss, improve mildness and the yield stress properties of cleansing compositions for the hair and skin, and can be utilized for the stable suspension of insoluble silicones, opacifiers and pearlescent agents (e.g., mica, coated mica, ethylene glycol monostearate (EGMS), ethylene glycol distearate (EGDS), polyethylene glycol monostearate (PGMS) or polyethyleneglycol distearate (PGDS)), pigments, exfoliants, anti-dandruff agents, clay, swellable clay, laponite, gas bubbles, liposomes, microsponges, cosmetic beads, cosmetic microcapsules, and flakes, and are discussed in more detail below. The cleansing compositions may be in the form of a body wash, shower gel, bubble bath, two-in-one shampoo, conditioner, facial scrub, moisture rinse, make-up removal product, and the like.

Exemplary cosmetic bead components include, but are not limited to, agar beads, alginate beads, jojoba beads, gelatin beads, Styrofoam™ beads, polyacrylate, polymethylmethacrylate (PMMA), polyethylene beads, Unispheres™ and Unipearls™ cosmetic beads (Induchem USA, Inc., New York, NY), Lipocapsule™, Liposphere™, and Lipopearl™ microcapsules (Lipo Technologies Inc., Vandalia, OH), and Confetti II™ dermal delivery flakes (United-Guardian, Inc., Hauppauge, NY). Beads can be utilized as aesthetic materials or can be used to encapsulate benefit agents to protect them from the deteriorating effects of the environment or for optimal delivery, release and performance in the final product.

In one aspect, the cosmetic beads range in size from 0.5 to 1.5 mm. In another aspect, the difference in specific gravity of the bead and water is between +/-0.01 and 0.5 in one aspect and from +/- 0.2 to 0.3 g/ml in another aspect.

In one aspect, the microcapsules range in size from 0.5 to 300 µm. In another aspect, the difference in specific gravity between the microcapsules and water is from +/- 0.01 to 0.5. Non-limiting examples of microcapsule beads are disclosed in U.S. Patent No. 7,786,027.

In one aspect of the invention, the amount of particulate component and/or insoluble droplets can range from 0.1% to 25 wt.% in one aspect, from 0.5 to 20 wt.% in another aspect, and from 1 or 5 to 10 wt.% in a further aspect, based on the total weight of the composition.

### Other Optional Components

In addition to the components described above, the cleansing compositions may further comprise one or more other optional components that are known or otherwise suitable for use on the hair, scalp or skin and which do not interfere with the deposition properties of the composition. Non-limiting examples of such optional components are disclosed in the International Cosmetic Ingredient Dictionary, Fifth Edition, 1993, and the Cosmetic, Toiletry, and Fragrance Association (CTFA) Cosmetic Ingredient Handbook, Second edition, 1992. Exemplary optional components are disclosed below.

### Conditioning Oils

A further component that may be used in the compositions of the invention is a conditioning oil (other than a silicone) selected from a hydrocarbon oil or an ester oil. These auxiliary conditioning agent materials may enhance the conditioning benefits of the silicone materials used in the cleansing compositions of the invention.

Suitable hydrocarbon oils have at least 12 carbon atoms, and include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched chain isomers of these compounds, as well as of higher chain length hydrocarbons, can also be used. Also suitable are polymeric hydrocarbons of C₂-C₆ alkenyl monomers, such as polyisobutylene.

Suitable ester oils have at least 10 carbon atoms, and include esters with hydrocarbyl chains derived from fatty acids or alcohols. In one aspect the ester oils conform to the formula R'C(O)OR in which R' and R independently represent alkyl or alkenyl radicals and the sum of carbon atoms in R' and R is at least 10 in one aspect, and at least 20 in another aspect. Dialkyl and trialkyl and alkenyl esters of polycarboxylic acids can also be used.

In another aspect ester oils are fatty esters of mono-, di- and triglycerides, more specifically the mono-, di-, and tri-esters of glycerol derived from long chain carboxylic acids such as C₁-C₂₂ carboxylic acids. Examples of such materials include cocoa butter, palm stearin, sunflower oil, soybean oil and coconut oil.

Mixtures of any of the above described hydrocarbon and ester oils also can be used. The total combined amount of hydrocarbon oil and/or ester oil in compositions of the invention may suitably range from 0.05 to 10 wt.% in one aspect, from 0.2 to 5 wt.%, and especially from 0.5 to 3 wt.% based on the weight of the total composition.

### Cationic Polymers

Cationic polymers are components that can enhance the delivery of conditioning agents and/or provide auxiliary conditioning benefits to the hair, scalp or skin to improve and enhance the conditioning benefits delivered by the silicone conditioning agents of the invention. Cationic polymer refers to polymers containing at least one cationic moiety or at least one moiety that can be ionized to form a cationic moiety. Typically, these cationic moieties are nitrogen containing groups such as quaternary ammonium or protonated amino groups. The cationic protonated amines can be primary, secondary, or tertiary amines. The cationic polymer typically has a cationic charge density ranging from 0.2 to 7 meq/g at the pH of the intended use of the composition. The average molecular weight of the cationic polymer ranges from 5,000 daltons to 10,000,000 daltons.

Non-limiting examples of such polymers are described in the CTFA *International Cosmetic Ingredient Dictionary*/*Handbook* via the CTFA website as well as the CTFA Cosmetic Ingredient Handbook, Ninth Ed., Cosmetic and Fragrance Assn., Inc., Washington D.C. (2002) can be used.

Non-limiting examples of suitable cationic polymers include copolymers of vinyl monomers having cationic protonated amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone or vinyl pyrrolidone.

Suitable cationic protonated amino and quaternary ammonium monomers, for inclusion in the cationic polymers of the composition herein, include vinyl compounds substituted with dialkylaminoalkyl acrylate, dialkylaminoalkyl methacrylate, monoalkylaminoalkyl acrylate, monoalkylaminoalkyl methacrylate, trialkyl methacryloxyalkyl ammonium salt, trialkyl acryloxyalkyl ammonium salt, diallyl quaternary ammonium salts, and vinyl quaternary ammonium monomers having cyclic cationic nitrogen-containing rings such as pyridinium, imidazolium, and quaternized pyrrolidone, e.g., alkyl vinyl imidazolium, alkyl vinyl pyridinium, alkyl vinyl pyrrolidone salts.

Other suitable cationic polymers for use in the compositions include copolymers of 1-vinyl-2-pyrrolidone and 1-vinyl-3-methylimidazolium salt (e.g., chloride salt) (CTFA, Polyquaternium-16); copolymers of 1-vinyl-2-pyrrolidone and dimethylaminoethyl methacrylate (CTFA, Polyquaternium-11); cationic diallyl quaternary ammonium-containing polymers, including, for example, dimethyldiallylammonium chloride homopolymer, copolymers of acrylamide and dimethyldiallylammonium chloride (CTFA, Polyquaternium-6 and Polyquaternium-7, respectively); amphoteric copolymers of acrylic acid including copolymers of acrylic acid and dimethyldiallylammonium chloride (CTFA, Polyquaternium-22); terpolymers of acrylic acid with dimethyldiallylammonium chloride and acrylamide (CTFA, Polyquaternium-39); terpolymers of acrylic acid with methacrylamidopropyl trimethylammonium chloride and methylacrylate (CTFA, Polyquaternium-47); terpolymers of acrylic acid, methacrylamidopropyl trimethylammonium chloride and acrylamide (CTFA, Polyquaternium-53). In one aspect suitable cationic substituted monomers are the cationic substituted dialkylaminoalkyl acrylamides, dialkylaminoalkyl methacrylamides, and combinations thereof.

Other suitable cationic polymers for use in the composition include polysaccharide polymers, such as cationic cellulose derivatives and cationic starch derivatives modified with a quaternary ammonium halide moiety. Exemplary cationic cellulose polymers are salts of hydroxyethyl cellulose reacted with trimethyl ammonium substituted epoxide (CTFA, Polyquaternium-10). Other suitable types of cationic cellulose include the polymeric quaternary ammonium salts of hydroxyethyl cellulose reacted with lauryl dimethyl ammonium substituted epoxide (CTFA, Polyquaternium-24).

Other suitable cationic polymers include cationic polygalactomannan derivatives such as guar gum derivatives and cassia gum derivatives, e.g., guar hydroxypropyltrimonium chloride, hydroxypropyl hydroxypropyltrimonium chloride guar and cassia hydroxypropyltrimonium chloride, respectively. Guar hydroxypropyltrimonium chloride is commercially available under the Jaguar™ trade name series from Rhodia Inc. and the N-Hance trade name series from Ashland Inc. Cassia hydroxypropyltrimonium chloride is commercially available under the Sensomer™ trade name series from Lubrizol Advanced Materials, Inc.

The amount of cationic polymer that may be utilized in the cleansing compositions of the invention range from 0.01 to 10 wt.% in one aspect, from 0.05 to 3 wt.% in another aspect, and from 0.1 to 1 wt.% in a further aspect, based on the weight of the total composition.

### Pigments

Exemplary pigments are metal compounds or semi metallic compounds and may be used in ionic, nonionic or oxidized form. The pigments can be in this form either individually or in admixture or as individual mixed oxides or mixtures thereof, including mixtures of mixed oxides and pure oxides. Examples are the titanium oxides (e.g., TiO₂), zinc oxides (e.g., ZnO), aluminum oxides (for example, Al₂O₃), iron oxides (for example, Fe₂O₃), manganese oxides (e.g., MnO), silicon oxides (e.g., SiO₂), silicates, cerium oxides, zirconium oxides (e.g., ZrO₂), barium sulfate (BaSO₄), nylon-12, and mixtures thereof.

Other examples of pigments include thermochromic dyes that change color with temperature, calcium carbonate, aluminum hydroxide, calcium sulfate, kaolin, ferric ammonium ferrocyanide, magnesium carbonate, carmine, barium sulfate, mica, bismuth oxychloride, zinc stearate, manganese violet, chromium oxide, titanium dioxide nanoparticles, barium oxide, ultramarine blue, bismuth citrate, hydroxyapatite, zirconium silicate, carbon black particles, and the like.

If utilized, the amount of pigment employed in the formulation should be sufficient to provide the desired product aesthetic effect and is well within the skill in the formulation art. In one aspect the amount of pigment typically utilized in the compositions of the invention range from 0.5 wt.% to 20 wt.% in one aspect, from 1 to 15 wt.% in another aspect, and from 5 to 10 wt.% in a further aspect, based on the total weight of the composition.

### Exfoliating Agents

Cosmetically useful particulate exfoliating agents are known in the art, and the selection and amount is determined by the exfoliating effect desired from the use of the composition, as recognized by those skilled in the cosmetic arts. Useful exfoliating agents include, but are not limited to, natural abrasives, inorganic abrasives, synthetic polymers, and the like, and mixtures thereof. Representative exfoliants include, but are not limited to, ground or powdered pumice, stone, zeolites, nut shells (e.g., almond, pecan, walnut, coconut, and the like), nut meals (e.g., almond, and the like), fruit pits (e.g., apricot, avocado, olive, peach, and the like), hulls, seed and kernel (e.g., oat bran, corn meal, rice bran, grape seed, kiwi seed, wheat, jojoba seed, loofah seed, rose hip seed, and the like), plant matter (e.g., tea tree leaves, corn cob, fruit fibers, seaweed, loofah sponge, microcrystalline cellulose, and the like), bivalve shells (oyster shell, and the like), diatomaceous earth, calcium carbonate, dicalcium pyrophosphate, chalk, silica, kaolin clay, silicic acid, aluminum oxide, stannic oxide, sea salt (e.g., Dead Sea salt), talc, sugars (e.g., table, brown, and the like), polyethylene, polystyrene, microcrystalline polyamides (nylons), microcrystalline polyesters, polycarbonates, and stainless steel fibers. The foregoing exfoliants can be used in the form of granules, powders, flours, and fibers.

The exfoliating agents for use in the present invention include inorganic physical abrasive type exfoliating agents, a number of which are presented above. In this aspect of the present invention, the exfoliating agent comprises from 0.1 to 20 wt.% in one aspect, and from 0.5 to 10 wt.% in another aspect, based on the weight of the composition.

### Anti-Dandruff Agents

Any suitable anti-dandruff agent can be employed in the compositions of the present invention. The anti-dandruff agents may be insoluble or water soluble. Exemplary anti-dandruff agents include, but are not limited to, sulfur, zinc pyrithione, zinc omadine, miconazole nitrate, selenium sulfide, piroctone olamine, N, N- bis(2- hydroxyethyl)undecenamide, cade oil, pine tar, Allium cepa extract Picea abies extract, and Undecyleneth-6, and the like, and mixtures thereof.

In one aspect of the invention, the anti-dandruff agents can be incorporated into the cleansing composition in an amount ranging from 0.001 to 10 wt.% in one aspect, from 0.1 to 5 wt.% in another aspect, and from 0.5 to 3 wt.% in a further aspect, based on the total weight of the stabilized composition.

### Pearlescent/Opacifying Agents

Some formulations are often opacified by deliberately incorporating pearlescent materials therein to achieve a cosmetically attractive pearl-like appearance, known as pearlescence. An opacifier often is included in a composition to mask an undesirable aesthetic property, such as to improve the color of a composition that is darkened due to the presence of a particular ingredient, or to mask the presence of a particulate material in the composition. Opacifiers also are included in aqueous compositions to improve the aesthetics and consumer acceptance of an otherwise esthetically unpleasing composition. For example, an opacifier can impart a pearlescent appearance to a clear composition, thereby communicating an appearance of creaminess, mildness and body to the consumer. Persons skilled in the art are aware of problems faced by formulators in consistently preparing a stable pearlescent formulation. A detailed discussion is found in the article "Opacifiers and pearling agents in shampoos" by Hunting, Cosmetic and Toiletries, Vol. 96, pages 65-78 (July 1981).

The opacifying or pearlescent material includes organic compounds and inorganic compounds. Typical examples of organic compounds are monoesters and/or diesters of ethylene glycol, propylene glycol, diethylene glycol, dipropylene glycol, triethylene glycol or tetraethylene glycol with fatty acids containing from 6 to 22 carbon atoms in one aspect, and from 12 to 18 carbon atoms in another aspect. Such fatty acids include caproic acid, caprylic acid, 2-ethyhexanoic acid, capric acid, lauric acid, isotridecanoic acid, myristic acid, palmitic acid, palmitoleic acid, stearic acid, isostearic acid, oleic acid, elaidic acid, petroselic acid, linoleic acid, linolenic acid, arachic acid, gadoleic acid, behenic acid, erucic acid, and mixtures thereof. In one aspect, ethylene glycol monostearate (EGMS) and/or ethylene glycol distearate (EGDS) and/or polyethylene glycol monostearate (PGMS) and/or polyethyleneglycol distearate (PGDS) are suitable pearlescent agents used in the composition.

Inorganic pearlescent agents include those selected from the group consisting of mica, metal oxide coated mica, silica coated mica, bismuth oxychloride coated mica, bismuth oxychloride, myristyl myristate, glass, metal oxide coated glass, various aluminum and magnesium salts, guanine, fish scales, glitter (polyester or metallic) and mixtures thereof.

Suitable micas include muscovite or potassium aluminum hydroxide fluoride. The platelets of mica can be coated with a thin layer of metal oxide. Metal oxides are selected from the group consisting of rutile, titanium dioxide, ferric oxide, tin oxide, alumina and mixtures thereof.

A representative listing of opacifiers is found in the CTFA Cosmetic Ingredient Handbook, J. Nikitakis, ed., 1988, at page 75. Other pearlescent or opacifying materials are disclosed in U.S. Pat. No. 4,654,207; U.S. Pat. No. 5,019,376; and U.S. Pat. No. 5,384,114.

In one aspect, the amount of the pearlescent or opacifying material can be used in amounts ranging from 0.01 to 10 wt.% in one aspect, from 0.1 % to 5 wt.% in another aspect, and from 0.5 to 3 wt.% in a further aspect, based upon the total weight of the composition.

Additional optional components for inclusion in the compositions of the invention the following may be mentioned: fragrances, chelating agents, auxiliary suspending agents and viscosity modifiers, such as guar and xanthan gums, emulsifiers, preservatives, amino acids, peptides, proteins, provitamins such as panthenol, vitamins, herb and plant extracts, humectants such as glycerine, and mixtures thereof.

The amount of these additives range from 0 to 20 wt.% in one aspect, from 0.1 to 10 wt.%, and from 0.5 to 5 wt.%, based on the total weight of the composition. The amount of each additive is readily determined by one skilled in the formulation art, depending on the nature and intended function for the additive.

The personal care cleansing compositions of the invention may be used, for example, for washing and depositing silicone conditioning agents on keratin materials such as the hair, the skin, the eyelashes, the eyebrows, the nails, the lips, face, or the scalp. The compositions are applied topically to the desired area of the skin or hair in an amount sufficient to provide effective cleansing and effective delivery of silicone from the product. The compositions can be applied directly to the skin or hair or indirectly via the use of a cleansing puff, washcloth, sponge or other implement. The compositions may be in the form of a body wash, shower gel, bubble bath, two-in-one shampoo, conditioner, facial scrub, moisture rinse, make-up removal product, and the like. The compositions are preferably diluted with water prior to, during, or after topical application, and then subsequently the skin or hair rinsed or dried off, preferably rinsed off of the applied surface using water.

The present invention may also be useful in rinse-off applications other than personal care compositions including pet care, auto care, home care and medical applications.

Shampoo embodiments of the invention can be formulated as two-in-one shampoos, baby shampoos, conditioning shampoos, bodifying shampoos, moisturizing shampoos, temporary hair color shampoos, three-in-one shampoos, anti-dandruff shampoos, hair color maintenance shampoos, acid (neutralizing) shampoos, medicated shampoos, and salicylic acid shampoos, and the like.

### Liquid Fatty Acid Soap Based Cleansers

In one aspect, a personal care cleansing composition in which the polymer of this invention is useful is a fatty acid soap based cleanser. Typical components of a fatty acid based soap cleanser, in addition to the stabilizer/thickener polymer of the invention are: at least one fatty acid salt; a silicone conditioning agent, water, an optional surfactant or mixture of surfactants; a sufficient amount of a pH adjusting agent (base and/or acid) to attain a pH of above 7 in one aspect, from 7.5 to 14 in another aspect, from 8 to 12 in still another aspect, and from 8.5 to 10 in a further aspect. Optional ingredients may be included such as the adjuvants, additives and benefit agents discussed above, and mixtures thereof, including conditioning oils, deposition aids, particulates and insoluble materials (e.g., pigments, anti-dandruff agents, pearlescent materials, opacifying materials, gas bubbles, cosmetic beads, flakes, and capsules), fragrances, chelating agents, auxiliary suspending agents and viscosity modifiers, emulsifiers, preservatives, amino acids, peptides, proteins, provitamins, vitamins, herb and plant extracts, humectants, and mixtures thereof.

In one aspect, the fatty acid soaps are selected from at least one the fatty acid salt (e.g., sodium, potassium, and ammonium) containing from 8 to 22 carbon atoms. In another aspect of the invention the liquid soap composition contains at least one fatty acid salt containing from 12 to 18 carbon atoms. The fatty acids utilized in the soaps can be saturated and unsaturated and can be derived from synthetic sources, as well as from the saponification of fats and natural oils by a suitable base (e.g., sodium, potassium and ammonium hydroxides). Exemplary saturated fatty acids include but are not limited to octanoic, decanoic, lauric, myristic, pentadecanoic, palmitic, margaric, steric, isostearic, nonadecanoic, arachidic, behenic, and the like, and mixtures thereof. Exemplary unsaturated fatty acids include but are not limited to the salts (e.g., sodium, potassium, ammonium) of myristoleic, palmitoleic, oleic, linoleic, linolenic, and the like, and mixtures thereof. The fatty acids can be derived from animal fat such as tallow or from vegetable oil such as coconut oil, red oil, palm kernel oil, palm oil, cottonseed oil, olive oil, soybean oil, peanut oil, corn oil, and mixtures thereof. The amount of fatty acid soap that can be employed in the liquid cleansing compositions of this embodiment ranges from 1% to 50% by weight in one aspect, from 10% to 35% by weight in another aspect, and from 12% to 25% by weight in a further aspect of the invention, based on the weight of the total composition.

The optional anionic surfactant can be present in the soap composition in an amount ranging from 1% to 25% by weight in one aspect, from 5% to 20% by weight in another aspect, and from 8% to 15% by weight in a further aspect, based on the weight of the total weight of the soap composition. Mixtures of anionic and amphoteric surfactants can be used. The ratio of anionic surfactant to amphoteric surfactant can range from 1:1 to 10:1 in one aspect, from 2.25:1 to 9:1 in another aspect, and from 4.5:1 to 7:1 in a further aspect.

In the foregoing soap embodiments of the invention, the amount of stabilizer/thickener polymer can range from 0.5% to 5% by weight in one aspect, from 1% to 3% by weight in another aspect, and from 1.5% to 2.5% by weight in a further aspect, based on the total weight of the soap composition.

The liquid fatty acid soap based cleanser embodiments of the invention can be formulated as body washes, bath gels, shower gels, liquid hand soaps, body scrubs; bubble baths, facial scrubs, and foot scrubs, two-in-one shampoos, baby shampoos, conditioning shampoos, bodifying shampoos, moisturizing shampoos, temporary hair color shampoos, three-in-one shampoos, anti-dandruff shampoos, hair color maintenance shampoos, acid (neutralizing) shampoos, medicated shampoos, and salicylic acid shampoos, and the like.

Advantageously, the nonionic, amphoteric polymer component of the invention is not pH dependent so the relatively high (basic) pH needed to maintain the fatty acid soap in liquid form does not affect the rheological properties imparted by the polymer.

### Multi-Phase Appearance

Visually distinct, multiple phase compositions where one phase is clear and another phase is opaque or pearlized are also envisioned. In one embodiment of the invention, a pattern comprising phases that are visually distinct from each other may be formed by mixing clear and opaque and/or pearlescent components. The visual distinction between each phase can be in color, texture, density, and the type of insoluble component contained therein. The specific pattern can be chosen from a wide variety of patterns, including, but not limited to the following examples: striped, marbled, rectilinear, interrupted striped, check, mottled, marbled, veined, clustered, speckled, geometric, spotted, ribbons, helical, swirl, arrayed, variegated, textured, grooved, ridged, waved, sinusoidal, spiral, twisted, curved, cycle, streaks, striated, contoured, anisotropic, laced, weave or woven, basket weave, spotted, and tessellated. The pattern results from the combination of the "multi-phase" composition by a method of manufacture described in U.S. Patent No. 6,213,166 (Thibiant et al.), U.S. Patent Publication No. US 2004/0219119 (Wei et al.), and U.S. Patent Publication No. US2011/0117225 (Wei et al.).

By the term "multi-phase" as used herein, is meant that each phase of the present compositions occupy separate but distinct physical spaces inside the package in which they are stored, but are in direct contact with one another (i.e., they are not separated by a barrier and they are not emulsified or mixed to any significant degree). In one embodiment of the present invention, the "multi-phase" compositions comprise at least two visually distinct phases, which are present within the container as a visually distinct pattern.

Each visually distinct phase can also include different insoluble materials and/or particulates such as pigments, cosmetic beads, cosmetic flakes, mica, air bubbles, exfoliants, pearlescent materials, opacifiers, silicones, botanicals, benefit agents, and the like as described herein and in the art.

Compositions of this invention demonstrate excellent stability with time in suspending insoluble components and/or benefit agents and stabilizing the visually distinct phases. Multiple-phase compositions are disclosed in U.S. Published Patent Application Nos. 2006/0079417, 2006/0079418, 2006/0079419, 2006/0079420, 2006/0079421, 2006/0079422, 2007/0009463, 2007/0072781, 2007/0280976, and 2008/0317698 to the Procter and Gamble Company.

Desirably, the stable multi-phase embodiments of the invention comprise at least two visually distinct phases that are packaged in a transparent or translucent container or package such that the consumer can view the pattern through the container or package.

This invention is illustrated by the following examples that are merely for the purpose of illustration. Unless specifically indicated otherwise, parts and percentages are given by weight.

### Test Methods

### Wool Swatch Preparation Procedure for Silicone Deposition Testing

Wool swatches (Wool muslin, STC EMPA 217), cut into swatches 2 in. x 6 in. with pinked edges; Testfabrics, Inc., West Pittston, PA, (www.testfabrics.com) are prewashed with a stripping shampoo (surfactant isopropanol mixture containing 10 wt.% sodium lauryl sulfate and 10 wt.% isopropanol) and thoroughly rinsed under warm tap water to remove the shampoo. Excess water is removed by squeezing the swatch with the fingers. The damp swatch is placed on top of a weighing dish and 0.25 g of the test shampoo formulation is applied evenly down the length of the swatch. The shampoo is massaged into the swatch and the swatch is then rinsed under warm tap water for approximately 60 seconds. The treatment step is repeated a second time for a total of two wash/rinse cycles.

### Silicone Deposition Measurement

The amount of silicone (silicon atoms) deposited on wool muslin swatch samples treated with a 2-in-1 shampoo composition is measured by X-Ray fluorescence (XRF) spectroscopy. A wavelength dispersive XRF spectrometer (PANalytical Axios Advanced Sequential 4 kW spectrometer - Model Number PW4400) interfaced with SuperQ 4 software application and fitted with a rhodium tube with an InSb crystal is utilized to facilitate high sensitivity for silicon atom detection corresponding to Si K alpha band. The samples are analyzed using a qualitative program to measure intensities across a two-theta scan range from 139.75° to 147.99° with a peak maximum at 144.53°. The samples are scanned in a vacuum environment using a tube voltage of 25 kV and a current of 160 mA. Scanning speed is 0.05 °2-Theta/sec. with 0.02 °2-Theta step size.

X-rays from the instrument excite silicon atoms deposited on the surface of the wool swatch causing them to emit energy and fluoresce. The silicon fluorescence is detected and recorded as counts per second. Higher count rates are indicative of higher silicon atom deposition. The amount of silicon atoms detected is directly proportional to the amount of silicone conditioner deposited on the wool. Samples for XRF analysis are prepared by folding each of the treated wool swatches and placing the folded swatch into a sample cup having a 6 µ thick polyethylene support substrate formed into the bottom. A polyethylene spacer is placed on each swatch to hold it onto the substrate.

An average reading of 3 wool swatches per formulation is reported. Wool swatches are an economical model substrate for human hair. The absolute silicone deposition values will be higher for wool swatches than human hair tresses, but the relative silicone deposition values for shampoo formulations containing small particle size silicone are similar for wool swatches and hair.

### Yield Stress

The yield stress values of these polymers are determined by oscillatory and steady shear measurements on a controlled stress rheometer (TA Instruments AR1000N rheometer, New Castle, DE) utilizing parallel plate geometry (40 mm stainless steel plate with a a 1000 µm gap) at 25°C. The oscillatory measurements are performed at a fixed frequency of 1 rad/sec. The elastic and viscous moduli (G' and G" respectively) are obtained as a function of increasing stress amplitude. In cases where the swollen polymer particles create a network, G' is larger than G" at low stress amplitudes but decreases at higher amplitudes crossing G" because of rupture of the network. The stress corresponding to the crossover of G' and G" is noted as the yield stress.

### Viscosity (Brookfield)

Brookfield rotating spindle method (all viscosity measurements reported herein are conducted by the Brookfield method whether mentioned or not): The viscosity measurements are calculated in mPa·s, employing a Brookfield rotating spindle viscometer, Model RVT (Brookfield Engineering Laboratories, Inc.), at about 20 revolutions per minute (rpm), at ambient room temperature of about 20 to 25°C (hereafter referred to as viscosity). Spindle sizes are selected in accordance with the standard operating recommendations from the manufacturer. Generally, spindle sizes are selected as follows:

| Spindle Size No. | Viscosity Range (mPa·s) |
|---|---|
| 1 | 1 - 50 |
| 2 | 500 - 1,000 |
| 3 | 1,000 - 5,000 |
| 4 | 5,000 - 10,000 |
| 5 | 10,000 - 20,000 |
| 6 | 20,000 - 50,000 |
| 7 | >50,000 |

The spindle size recommendations are for illustrative purposes only. The artisan of ordinary skill in the art will select a spindle size appropriate for the system to be measured.

### Abbreviations

The following abbreviations and trade names are utilized in the examples.

**ABBREVIATIONS and Trade Names**

| Abbreviation/Trade Name | Chemical Name |
|---|---|
| VA | Vinyl acetate |
| SMA | Stearyl methacrylate |
| BEM | Behenyl ethoxylated-25 methacrylate |
| NVP | N-vinyl-2-pyrrolidone (N-vinyl pyrrolidone) |
| VA-10 | Vinyl neodecanoate |
| APE | Allyl pentaerythritol |
| CYCLO | Cyclohexane |
| EA | Ethyl Acetate |
| PGS | Reaction product C₂₀-C₂₄ substituted succinic anhydride and glycerin and or polyglycerol containing 2 to 6 glycerin units utilized as a process aid |
| Calfax® DB-45 surfactant | Sodium dodecyl diphenyl oxide disulfonate (Pilot Chemical Company) |
| Sulfochem™ ES-2 CWK anionic surfactant (28% active) | Sodium Laureth-2 sulfate (Lubrizol Advanced Materials, Inc.) |
| Chembetaine™ C amphoteric surfactant (35% active) | Cocamidopropylbetaine (Lubrizol Advanced Materials, Inc.) |
| Dow Corning® DC 2-1352 silicone emulsion (60% active) | INCI Name¹: Dimethicone (and) Laureth-23 (and) C12-15 Pareth-3 (Dow Corning Corporation) (0.5 µm) |
| Jaguar® C13-S cationic guar | Guar Hydroxypropyltrimonium Chloride (Rhodia Group) |
| Glydant® Plus preservative (2.0 w/w aqueous solution) | DMDM Hydantoin (and) lodopropynyl Butylcarbamate (Lonza Group Ltd.) |
| Carbopol® Aqua SF-1 polymer | INCI Name¹: Acrylates Copolymer. A crosslinked copolymer of two or more monomers consisting of acrylic acid, methacrylic acid or one of their simple esters; Supplied as a polymer emulsion 30 wt.% active solids (Lubrizol Advanced Materials, Inc.) |

| | |
|---|---|
| INCI name is the International Nomenclature Cosmetic Ingredient name assigned to a cosmetic ingredient by the International Nomenclature Committee of the Cosmetic, Toiletry, and Fragrance Association (CTFA), Washington, DC, USA, now known as the Personal Care Products Council (PCPC). INCI Names and their definitions are published in the International Cosmetic Ingredient Dictionary and Handbook. | |

### Examples 1-10

A free radical initiated dispersion polymerization is utilized to prepare the nonionic, amphiphilic polymer component of the invention. The polymerization reactor consists of a water-cooled resin kettle equipped with a reflux condenser, nitrogen purging tube, a mechanical agitator and a thermal-couple connected to a temperature control module. Admixtures of monomers, crosslinkers, and processing aids set forth in Table 1 are added to the resin kettle, followed by the polymerization solvent. The quantities of these components in wt.% for the various polymer preparations are shown in the table. While the reaction medium is heated to the target polymerization temperature, the reactor is purged with nitrogen for at least half an hour. As the reactor temperature reaches the set polymerization temperature, typically at about 67°C, the initiator solution 2,2'-azobis(2-methylbutyronitrile) (0.12 wt.% based on the dry weight of the polymer) is injected into the reaction kettle to start the polymerization. The polymerization reaction is continued for at least 6 hours at 67°C before a series of shots of additional initiator solution are injected into the reactor to remove residual monomers. The total polymer solids in the final dispersion is typically at about 30 wt.%. Upon the completion of the reaction, the polymerization solvent is removed by rotary evaporator under vacuum to recover a polymer powder, which is gently milled to a finer powdered product.

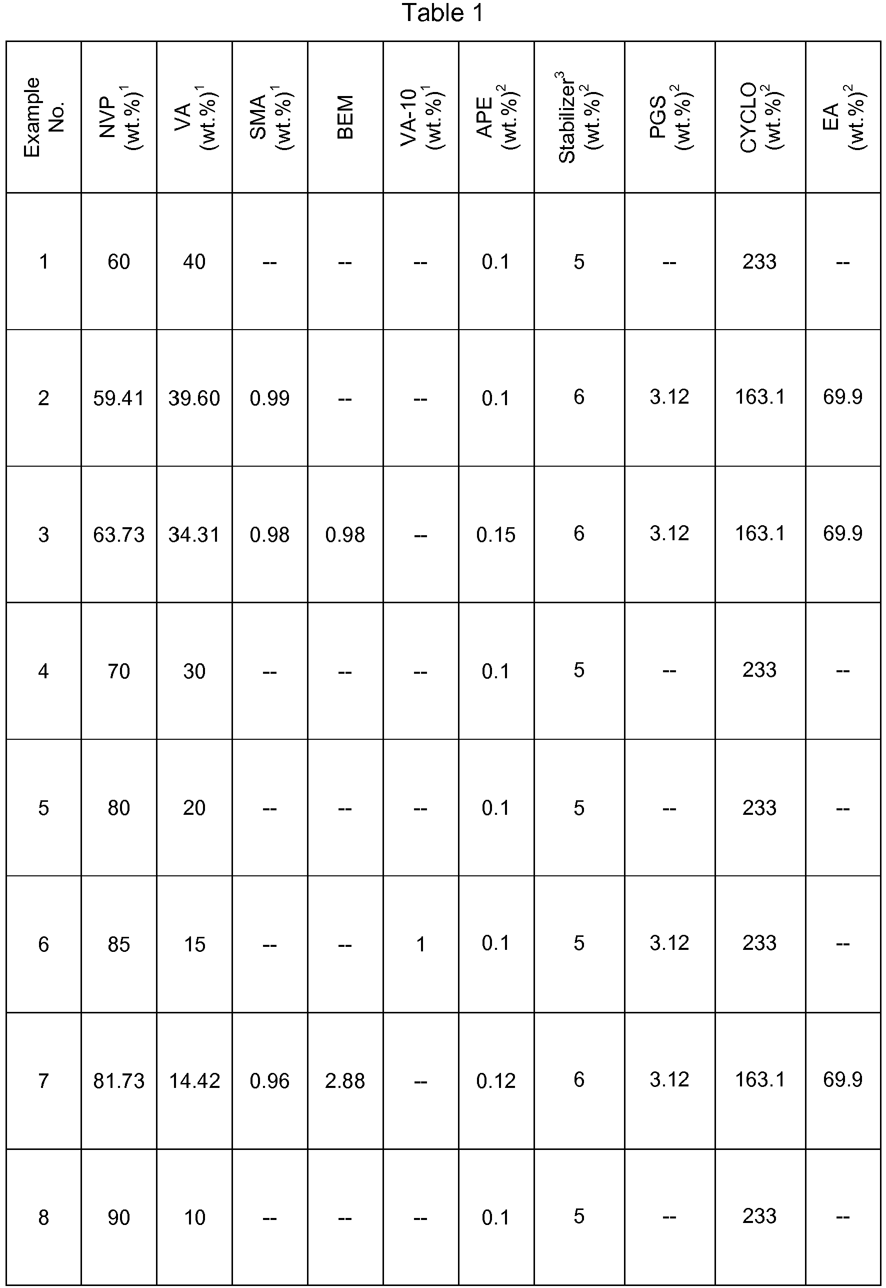

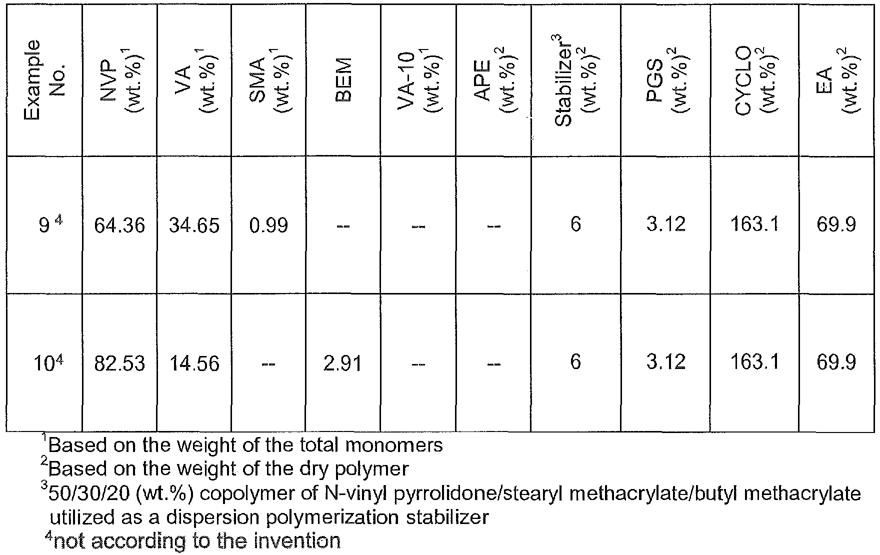

### Examples 11-24

Two-in-one conditioning shampoos containing the nonionic, amphiphilic polymers prepared in accordance with Examples 1-10 are formulated with the components set forth in Table 2.

**Table 2**

| Component | Amount Active (wt.%) |
|---|---|
| PART A | |
| D.I. Water | q.s. to 100 |
| Powdered Dispersion Polymer | Table 3 |
| Sulfochem™ ES-2 CWK anionic surfactant | 14 |
| Chembetaine™ C amphoteric surfactant | 3 |

| PART B | |
|---|---|
| Jaguar® C13-S cationic guar deposition aid | 0.25 |

| PART C | |
|---|---|
| Dow Corning® silicone emulsion (DC 2-1352) | 2 |
| Glydant® Plus preservative | 0.22 |

| PART D | |
|---|---|
| NaOH (18% aqueous wt./wt.) pH adjusting agent | q.s. to pH 6 |

The shampoo compositions are prepared in accordance with the following procedure:

### Part A:

1) Homogeneously disperse the polymer in D.I. water;
2) Add anionic and amphoteric surfactants and mix for 15 min.

### Part B:

3) Prepare a 2 wt.% (wt./wt.) dispersion of cationic guar in D.I. water and mix with Part A components.

### Part C:

4) Add Part C components to Part AB component mixture and mix until homogeneous;
4) Adjust the pH of the ABC component mixture with NaOH to a pH of about 6.

Identical two-in-one comparative shampoo compositions are similarly prepared except that one is formulated with a commercially available thickener polymer (INCI Name: Acrylates Copolymer) and a control shampoo formulation is formulated without any polymer. The commercially available thickener polymer is widely employed in shampoo compositions for its ability to stably suspend silicones and other particulate materials such as pearlescence agents (e.g., mica and/or beads).

The amount of silicone (silicon atoms) deposited on wool muslin swatch samples treated with the 2-in-1 shampoo compositions is measured by X-Ray fluorescence (XRF) spectroscopy in accordance with the test methodology set forth above. In addition, the Brookfield viscosity (BV) and yield stress of each shampoo formulation is measured 24 hours after formulation. The results are reported in the table below.

**Table 3**

| Ex. No. | Polymer Ex. No. | Polymer (wt.%) | Si Peak Intensity (kcps) | BV Viscosity (mPa·s) | Yield Stress (Pa) |
|---|---|---|---|---|---|
| 11 | 1 | 3.0 | 58.4 | 8280 | 5.0 |
| 12 | 2 | 3.0 | 73.6 | 5160 | 3.5 |
| 13 | 3 | 2.5 | 63.8 | -- | 3.5 |
| 14 | 3 | 2.5 | 82.77 | 7580 | 3.2 |
| 15 | 4 | 3.0 | 56.2 | 5280 | 4.0 |
| 16 | 5 | 3.0 | 57.4 | 3780 | 1.3 |
| 17 | 6 | 3.0 | 57.43 | 1250 | 0.4 |
| 18 | 7 | 2.5 | 58.9 | 4250 | 2.5 |
| 19 | 8 | 3.0 | 55.0 | 3810 | 4.4 |
| 20⁴ | 9 | 2.5 | 35.5 | 470 | 0 |
| 21⁴ | 10 | 2.5 | 32.7 | 300 | 0 |
| 22¹ | C-1³ | 1.5 | 12.6 | 3350 | 5.0 |
| 23¹ | C-1³ | 2.5 | 5.93 | 9850 | 22.0 |
| 24² | -- | 0 | 90.0 | 195 | 0 |

| | | | | | |
|---|---|---|---|---|---|
| ¹Comparative example ²Blank control formulation (contains no polymer) ³Carbopol® Aqua SF-1 polymer emulsion ⁴not according to the invention | | | | | |

Silicone deposition generally decreases with increasing concentration of anionic acrylic polymers. This is seen in the results for polymer C-1. As the concentration of polymer in the two-in-one shampoo formulation increases from 1.5 wt.% to 2.5 wt.% silicone deposition decreases. It is also evident that polymers in general adversely influence silicone deposition, as the two-in-one shampoo formulated without any polymer (blank control formulation) deposits significantly more silicone than shampoos formulated with any polymer. From the data it is apparent that shampoos formulated with the nonionic, amphiphilic polymers of the invention significantly mitigate the loss of silicone deposition when included in silicone containing two-in-one conditioning shampoos. Silicon deposition, Si Peak Intensity (kcps), for the two-in-one shampoos of Examples 11-19 and 22-24 are plotted in Fig. 1.

### Example 25 (not according to the invention)

A linear copolymer of Poly(1-vinylpyrrolidone-co-vinyl acetate) (62.7 wt.% NVP residues, 37.3 wt.% VA residues) obtained from Sigma-Aldrich (CAS No. 25086-89-9) is formulated into a two-in-one shampoo composition utilizing the same components as set forth in Examples 11-24, except that 1.5 wt.% of the linear copolymer (100% active), 15 wt.% Sulfochem™ ES-2 CWK anionic surfactant, and 2 wt.% Chembetaine™ C amphoteric surfactant, (all weights based on the total weight of the formulated composition) are utilized. The amount of silicon (silicon atoms) deposited on wool muslin swatch samples treated with the 2-in-1 shampoo compositions is measured by X-Ray fluorescence (XRF) spectroscopy in accordance with the test methodology set forth above. The yield stress (24 hrs. after formulation) of the shampoo composition is also measured as set forth in the methodology above.

The measured silicon peak intensity is 71.8 kcps, and the yield stress of the shampoo formulation is 0 Pa.

## Claims

1. A personal care composition comprising:
A) at least one detersive surfactant selected from anionic, amphoteric, cationic, or nonionic surfactant;
B) at least one silicone conditioning agent
C) water; and
D) a nonionic, amphiphilic polymer prepared from a polymerizable monomer mixture comprising:
a) from 55 to 95 wt.%, from 60 to 90 wt.% in another aspect, from 65 to 85 wt.% in still another aspect, and from 70 to 80 wt.% in a further aspect, of at least one vinyl amide monomer (based on the weight of the total monomers present) ;
b) from 5 to 45 wt.%, from 10 to 40 wt.% in another aspect, from 15 to 35 wt.% in still another aspect, and from 20 to 30 wt.% in a further aspect of at least one vinyl ester of an aliphatic carboxylic acid containing an acyl moiety having 2 to 22 carbon atoms (based on the weight of the total monomers present);
c) from 0.01 to 1 wt.%, from 0.01 to 0.75 wt.% in another aspect, from 0.1 to 0.5 wt.% in still another aspect, and from 0.15 to 0.3 wt.% in a further aspect of at least one polyunsaturated crosslinking monomer containing at least two polymerizable ethylenically unsaturated moieties (based on the total dry weight of the polymer) selected from polyallyl ethers of trimethylolpropane, polyallyl ethers of pentaerythritol, polyallyl ethers of sucrose having from 2 to 8 allyl groups per molecule, and mixtures thereof;
d) from 0 to 10 wt.%, from 0.1 to 5 wt.% in another aspect, from 0.5 to 3 wt.% in still another aspect, and from 0.75 to 1 wt.% in a further aspect, of at least one C₁-C₂₂ alkyl (meth)acrylate (based on the weight of the total monomers present);
e) from 0 to 10 wt.%, and from 0.5, 1, 2, or 3 to 5 wt.% in a further aspect of an alkoxylated associative monomer (based on the weight of the total monomers present);
f) from 0 to 10 wt.%, and from 0.5, 1, 2, or 3 to 5 wt.% in a further aspect of an alkoxylated semi-hydrophobic monomer (based on the weight of the total monomers present); and
g) from 0 or 0.5, 1, 2 or 3 to 5 wt.% of at least one vinyl ester of an aliphatic carboxylic acid containing an acyl moiety having 2 to 22 carbon atoms (based on the weight of the total monomers present) other than vinyl acetate.

2. A personal care composition of claim 1, wherein said monomer composition further comprises from 0.01 to 15 wt.% of at least one copolymerizable monomer selected from:
h) at least one C₁-C₅ hydroxyalkyl (meth)acrylate;
i) at least one (meth)acrylamide selected from (meth)acrylamide, N-(C₁-C₅)alkyl (meth)acrylamide, N,N-di(C₁-C₅)alkyl (meth)acrylamide, N-(C₁-C₅)alkylamino(C₁-C₅)alkyl(meth)acrylamide or N,N-di(C₁-C₅)alkylamino(C₁-C₅)alkyl(meth)acrylamide;
j) at least one alpha-olefinic monomer; and mixtures thereof.

3. A personal care composition of any of the preceding claims, wherein said at least one vinyl amide monomer is selected from N-vinylformamide, N-methyl-N-vinylformamide, N-(hydroxymethyl)-N-vinylformamide, N-vinylacetamide, N-vinylmethylacetamide, N-(hydroxymethyl)-N-vinylacetamide, and mixtures thereof.

4. A personal care composition of any of the preceding claims, wherein said at least one vinyl ester of an aliphatic carboxylic acid containing an acyl moiety having 2 to 22 carbon atoms is selected from vinyl acetate, vinyl propionate, vinyl butyrate, vinyl isobutyrate, vinyl valerate, vinyl hexanoate, vinyl 2-methylhexanate, vinyl 2-ethylhexanoate, vinyl iso-octanoate, vinyl nonanoate, vinyl neodecanoate, vinyl decanoate, vinyl versatate, vinyl laurate, vinyl palmitate, vinyl stearate, and mixtures thereof.

5. A personal care composition of any of the preceding claims, wherein the at least one polyunsaturated crosslinking monomer is selected from a monomer having an average of 2 crosslinkable unsaturated functional groups, an average of 3 crosslinkable unsaturated functional groups, and mixtures thereof.

6. A personal care composition of any of the preceding claims, wherein said C₁-C₅ hydroxyalkyl (meth)acrylate monomer is selected from 2-hydroxyethyl(meth)acrylate, 3-hydroxypropyl(meth)acrylate, 4-hydroxybutyl(meth)acrylate, and mixtures thereof.

7. A personal care composition of any of the preceding claims, wherein said at least one C₁-C₂₂ alkyl (meth)acrylate is selected from methyl (meth)acrylate, butyl (meth)acrylate, sec-butyl (meth)acrylate, iso-butyl (meth)acrylate, hexyl (meth)acrylate), heptyl (meth)acrylate, octyl (meth)acrylate, 2-ethylhexyl (meth)acrylate, decyl (meth)acrylate, isodecyl (meth)acrylate, lauryl (meth)acrylate, tetradecyl (meth)acrylate, hexadecyl (meth)acrylate, stearyl (meth)acrylate, behenyl (meth)acrylate, or mixtures thereof.

8. A personal care composition of any of the preceding claims, wherein said alkoxylated associative monomer comprises (i) a polymerizable ethylenically unsaturated end group portion, (ii) a polyoxyalkylene mid-section portion, and (iii) a hydrophobic end group portion containing 7 to 30 carbon atoms, preferably
wherein said associative monomer is represented by formulas VII and/or VIIA: 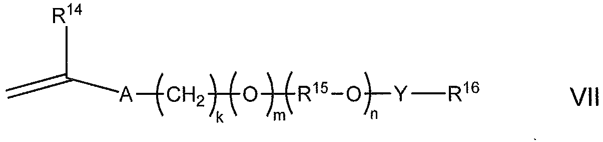 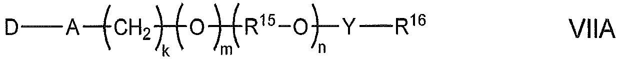
wherein R¹⁴ is hydrogen or methyl; A is -CH₂C(O)O-, -C(O)O-, -O-, -CH₂O- ,-NHC(O)NH-, -C(O)NH-,
-Ar-(CE₂)_{z}-NHC(O)O-, -Ar-(CE₂)_{z}-NHC(O)NH-, or -CH₂CH₂NHC(O)-; a divalent alkylene radical containing 1 to 5 carbon atoms; Ar is a divalent arylene; E is H or methyl; z is 0 or 1; k is an integer ranging from 0 to 30, and m is 0 or 1, with the proviso that when k is 0, m is 0, and when k is in the range of 1 to 30, m is 1; D represents a vinyl or an allyl moiety; (R¹⁵-O)ₙ is a polyoxyalkylene moiety, which can be a homopolymer, a random copolymer, or a block copolymer of C₂-C₄ oxyalkylene units, R¹⁵ is a divalent alkylene moiety selected from C₂H₄, C₃H₆, or C₄H₈, and combinations thereof; and n is an integer in the range of 2 to 150 in one aspect, from 10 to 120 in another aspect, and from 15 to 60 in a further aspect; Y is - R¹⁵O-, -R¹⁵NH-, -C(O)-, -C(O)NH-, -R¹⁵NHC(O)NH-, or -C(O)NHC(O)-; R¹⁶ is a substituted or unsubstituted alkyl selected from a C₈-C₃₀ linear alkyl, a C₈-C₃₀ branched alkyl, a C₇-C₃₀ carbocyclic alkyl, a C₂-C₃₀ alkyl-substituted phenyl, an araalkyl substituted phenyl, and an aryl-substituted C₂-C₃₀ alkyl; wherein the R¹⁶ alkyl group, carbocyclic alkyl group, aryl group, phenyl group optionally comprises one or more substituents selected from the group selected from a methyl group, hydroxyl group, an alkoxyl group, benzyl group styryl group, and a halogen group, or
wherein said associative monomer is represented by formula VIIB: 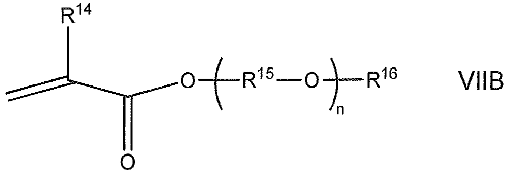 wherein R¹⁴ is hydrogen or methyl; R¹⁵ is a divalent alkylene moiety independently selected from C₂H₄, C₃H₆, and C₄H₈, and n represents an integer ranging from 10 to 60, (R¹⁵-O) can be arranged in a random or a block configuration; R¹⁶ is a substituted or unsubstituted alkyl selected from a C₈-C₃₀ linear alkyl, a C₈-C₃₀ branched alkyl, a C₇-C₃₀ carbocyclic alkyl, a C₂-C₃₀ alkyl-substituted phenyl, an araalkyl substituted phenyl, and an aryl-substituted C₂-C₃₀ alkyl, wherein the R¹⁶ alkyl group, aryl group, phenyl group optionally comprises one or more substituents selected from the group consisting of a hydroxyl group, an alkoxyl group, benzyl group styryl group, and a halogen group.

9. A personal care composition of any of the preceding claims, wherein said semi-hydrophobic monomer comprises (i) a polymerizable ethylenically unsaturated end group portion, (ii) a polyoxyalkylene mid-section portion, and (iii) an end group portion selected from hydrogen or an alkyl group containing 1 to 4 carbon atoms, preferably
wherein said semi-hydrophobic monomer is selected from at least one monomer represented by formulas VIII and IX: 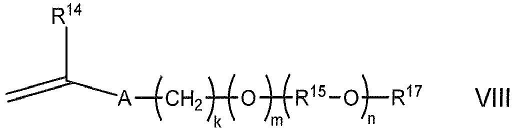 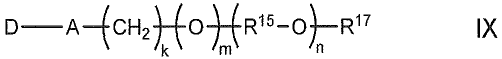
wherein R¹⁴ is hydrogen or methyl; A is -CH₂C(O)O-, -C(O)O-, -O-, -CH₂O-,-NHC(O)NH-, -C(O)NH-,
-Ar-(CE₂)_{z}-NHC(O)O-, -Ar-(CE₂)_{z}-NHC(O)NH-, or -CH₂CH₂NHC(O)-; Ar is a divalent arylene; E is H or methyl; z is 0 or 1; k is an integer ranging from 0 to 30, and m is 0 or 1, with the proviso that when k is 0, m is 0, and when k is in the range of 1 to 30, m is 1; (R¹⁵-O)ₙ is a polyoxyalkylene moiety, which can be a homopolymer, a random copolymer, or a block copolymer of C₂-C₄ oxyalkylene units, R¹⁵ is a divalent alkylene moiety selected from C₂H₄, C₃H₆, or C₄H₈, and combinations thereof; and n is an integer in the range of 2 to 150 in one aspect, from 5 to 120 in another aspect, from 10 to 60 in a further aspect, and from 15 to 30 in a still further aspect; R¹⁷ is selected from hydrogen and a linear or branched C₁-C₄ alkyl group; and D represents a vinyl or an allyl moiety, or
wherein said semi-hydrophobic monomer is selected from at least one monomer represented by formulas VIIIA and VIIIB:
CH₂=C(R¹⁴)C(O)O-(C₂H₄O)ₐ(C₃H₆O)_{b}-H VIIIA
CH₂=C(R¹⁴)C(O)O-(C₂H₄O)ₐ(C₃H₆O)_{b}-CH₃ VIIIB
wherein R¹⁴ is hydrogen or methyl, and "a" is an integer ranging from 0 or 2 to 120 in one aspect, from 5 to 45 in another aspect, and from 10 to 25 in a further aspect, and "b" is an integer ranging from 0 or 2 to 120 in one aspect, from 5 to 45 in another aspect, and from 10 to 25 in a further aspect, subject to the proviso that "a" and "b" cannot be 0 at the same time.

10. A personal care composition of any of the preceding claims, wherein said at least one (meth)acrylamide is selected from N-methyl(meth)acrylamide, N-ethyl(meth)acrylamide, N-propyl(meth)acrylamide, N-isopropyl(meth)acrylamide, N-tert-butyl(meth)acrylamide, N-tert-octyl(meth)acrylamide, N-(2-hydroxyethyl)(meth)acrylamide, N-(3-hydroxypropyl)(meth)acrylamide; N,N-dimethyl(meth)acrylamide, N,N-diethyl(meth)acrylamide, N,N-(di-2-hydroxyethyl)(meth)acrylamide, N,N-(di-3-hydroxypropyl)(meth)acrylamide, N-methyl,N-ethyl(meth)acrylamide; N,N-dimethylaminoethyl(meth)acrylamide, N,N-diethylaminoethyl(meth)acrylamide, N,N-dimethylaminopropyl(meth)acrylamide; and mixtures thereof.

11. A personal care composition of any of the preceding claims, wherein said at least one alpha-olefinic monomer is selected from ethylene, propylene, 1-butene, iso-butylene, 1-hexene, 1-heptene, 4-methyl-1-pentene, styrene, alpha-methyl styrene, and mixtures thereof.

12. A personal care composition of any of the preceding claims, wherein said nonionic, amphiphilic polymer comprises repeating units prepared from a monomer mixture comprising:
a) from 55 to 95 wt.%, from 60 to 90 wt.% in another aspect, from 65 to 85 wt.% in still another aspect, and from 70 to 80 wt.% in a further aspect, of N-vinyl pyrrolidone (based on the weight of the total monomers present);
b) from 5 to 45 wt.%, from 10 to 40 wt.% in another aspect, from 15 to 35 wt.% in still another aspect, and from 20 to 30 wt.% in a further aspect of vinyl acetate (based on the weight of the total monomers present);
c) from 0.01 to 1 wt.%, from 0.01 to 0.75 wt.% in another aspect, from 0.1 to 0.5 wt.% in still another aspect, and from 0.15 to 0.3 wt.% in a further aspect of at least one polyunsaturated crosslinking monomer containing at least two polymerizable ethylenically unsaturated moieties (based on the total dry wt. of the polymer);
d) from 0 to 10 wt.%, from 0.1 to 5 wt.% in another aspect, from 0.5 to 3 wt.% in still another aspect, and from 0.75 to 1 wt.% in a further aspect (based on the weight of the total monomers present) of at least one C₁-C₂₂ alkyl (meth)acrylate selected from methyl (meth)acrylate, butyl (meth)acrylate, sec-butyl (meth)acrylate, iso-butyl (meth)acrylate, hexyl (meth)acrylate), heptyl (meth)acrylate, octyl (meth)acrylate, 2-ethylhexyl (meth)acrylate, decyl (meth)acrylate, isodecyl (meth)acrylate, lauryl (meth)acrylate, tetradecyl (meth)acrylate, hexadecyl (meth)acrylate, stearyl (meth)acrylate, or behenyl (meth)acrylate; and
e) from 0 to 10 wt.%, and from 0.5, 1, 2, or 3 to 5 wt.% in a further aspect (based on the weight of the total monomers present) of an alkoxylated associative monomer selected from lauryl polyethoxylated (meth)acrylate, cetyl polyethoxylated (meth)acrylate, cetearyl polyethoxylated (meth)acrylate, stearyl polyethoxylated (meth)acrylate, arachidyl polyethoxylated (meth)acrylate, behenyl polyethoxylated (meth)acrylate, cerotyl polyethoxylated (meth)acrylate, montanyl polyethoxylated (meth)acrylate, melissyl polyethoxylated (meth)acrylate, phenyl polyethoxylated (meth)acrylate, nonylphenyl polyethoxylated (meth)acrylate, ω-tristyrylphenyl polyoxyethylene methacrylate, where the polyethoxylated portion of the monomer contains from 2 to 150 ethylene oxide units in one aspect, from 5 to 120 in another aspect, from 10 to 60 in a further aspect, and from 15 to 30 in a still further aspect.

13. A personal care composition of any of the preceding claims, wherein said silicone conditioning agent is non-volatile silicone selected from a silicone oil, silicone gum, silicone resin and mixtures thereof, or
wherein said silicone conditioning agent is selected from polydimethylsiloxanes (dimethicones), polydiethylsiloxanes, polydimethyl siloxanes having terminal hydroxyl groups (dimethiconols), polymethylphenylsiloxanes, phenylmethylsiloxanes, and mixtures thereof, or
wherein said silicone conditioning agent is selected from an amino functional polydimethylsiloxane (amodimethicone).

14. A personal care composition of any of the preceding claims, wherein said personal care composition is selected from shampoos, baby shampoos, body washes, shower gels, liquid hand soaps, liquid dishwashing detergents, pet cleansing product, moist cleansing wipes, or facial cleansers.

15. A method for mitigating the loss of silicone deposited from a silicone containing conditioning shampoo composition, said method comprising contacting a keratinous substrate with a detersive composition comprising the composition of any of the previous claims.

16. A method for mitigating the loss of silicone deposited from a silicone containing conditioning shampoo composition onto a keratinous substrate, said method comprising including therein from 1 to 5 wt.% of a nonionic, amphiphilic dispersion polymer, wherein said polymer is prepared from a monomer mixture comprising:
a) from 55 to 95 wt.%, from 60 to 90 wt.% in another aspect, from 65 to 85 wt.% in still another aspect, and from 70 to 80 wt.% in a further aspect, of N-vinyl pyrrolidone (based on the weight of the total monomers present);
b) from 5 to 45 wt.%, from 10 to 40 wt.% in another aspect, from 15 to 35 wt.% in still another aspect, and from 20 to 30 wt.% in a further aspect of vinyl acetate (based on the weight of the total monomers present);
c) from 0.01 to 1 wt.%, from 0.01 to 0.75 wt.% in another aspect, from 0.1 to 0.5 wt.% in still another aspect, and from 0.15 to 0.3 wt.% in a further aspect of at least one polyunsaturated crosslinking monomer containing at least two polymerizable ethylenically unsaturated moieties (based on the total dry wt. of the polymer) selected from polyallyl ethers of trimethylolpropane, polyallyl ethers of pentaerythritol, polyallyl ethers of sucrose having from 2 to 8 allyl groups per molecule, and mixtures thereof;
d) from 0 to 10 wt.%, from 0.1 to 5 wt.% in another aspect, from 0.5 to 3 wt.% in still another aspect, and from 0.75 to 1 wt.% in a further aspect (based on the weight of the total monomers present) of at least one C₁-C₂₂ alkyl (meth)acrylate selected from methyl (meth)acrylate, butyl (meth)acrylate, sec-butyl (meth)acrylate, iso-butyl (meth)acrylate, hexyl (meth)acrylate), heptyl (meth)acrylate, octyl (meth)acrylate, 2-ethylhexyl (meth)acrylate, decyl (meth)acrylate, isodecyl (meth)acrylate, lauryl (meth)acrylate, tetradecyl (meth)acrylate, hexadecyl (meth)acrylate, stearyl (meth)acrylate, or behenyl (meth)acrylate; and
e) from 0 to 10 wt.%, and from 0.5, 1, 2, or 3 to 5 wt.% in a further aspect (based on the weight of the total monomers present) of an alkoxylated associative monomer selected from lauryl polyethoxylated (meth)acrylate, cetyl polyethoxylated (meth)acrylate, cetearyl polyethoxylated (meth)acrylate, stearyl polyethoxylated (meth)acrylate, arachidyl polyethoxylated (meth)acrylate, behenyl polyethoxylated (meth)acrylate, cerotyl polyethoxylated (meth)acrylate, montanyl polyethoxylated (meth)acrylate, melissyl polyethoxylated (meth)acrylate, phenyl polyethoxylated (meth)acrylate, nonylphenyl polyethoxylated (meth)acrylate, ω-tristyrylphenyl polyoxyethylene methacrylate, where the polyethoxylated portion of the monomer contains from 2 to 150 ethylene oxide units in one aspect, from 5 to 120 in another aspect, from 10 to 60 in a further aspect, and from 15 to 30 in a still further aspect;
f) from 0 to 10 wt.%, and from 0.5, 1, 2, or 3 to 5 wt.% in a further aspect (based on the weight of the total monomers present) of a semi-hydrophobic monomer selected from methoxy polyethyleneglycol methacrylate;
g) from 0 to 10 wt.%, and 0.5, 1, 2 or 3 to 5 wt.% (based on the weight of the total monomers present) of at least one vinyl ester of an aliphatic carboxylic acid containing an acyl moiety having 2 to 22 carbon atoms other than vinyl acetate selected from vinyl propionate, vinyl butyrate, vinyl isobutyrate, vinyl valerate, vinyl hexanoate, vinyl 2-methylhexanate, vinyl 2-ethylhexanoate, vinyl iso-octanoate, vinyl nonanoate, vinyl neodecanoate, vinyl decanoate, vinyl versatate, vinyl laurate, vinyl palmitate, and vinyl stearate.

17. A method of claim 16, wherein said monomer mixture comprises:
a) 55 to 95 wt.%, of N-vinyl pyrrolidone (based on the weight of the total monomers present);
b) from 5 to 45 wt.%, in a further aspect of vinyl acetate (based on the weight of the total monomers present);
c) from 0.1 to 1 wt.% of at least one polyunsaturated crosslinking monomer containing at least two polymerizable ethylenically unsaturated moieties (based on the total dry wt. of the polymer);
d) from 0 or 1 to 5 wt.% (based on the weight of the total monomers present) of at least one monomer selected from decyl (meth)acrylate, isodecyl (meth)acrylate, lauryl (meth)acrylate, tetradecyl (meth)acrylate, hexadecyl (meth)acrylate, stearyl (meth)acrylate, or behenyl (meth)acrylate;
e) from 0 or 1 to 5 wt.% (based on the weight of the total monomers present) of an alkoxylated associative monomer selected from lauryl polyethoxylated (meth)acrylate, cetyl polyethoxylated (meth)acrylate, cetearyl polyethoxylated (meth)acrylate, stearyl polyethoxylated (meth)acrylate, behenyl polyethoxylated (meth)acrylate, where the polyethoxylated portion of the monomer contains from 10 to 60 ethylene oxide units;
f) from 0 or 1 to 5 wt.% (based on the weight of the total monomers present) of a semi-hydrophobic monomer selected from methoxy polyethyleneglycol methacrylate; and
g) from 0 or 1 to 5 wt.%, (based on the weight of the total monomers present) of at least one vinyl ester of an aliphatic carboxylic acid containing an acyl moiety having 2 to 22 carbon atoms other than vinyl acetate selected from vinyl propionate, vinyl butyrate, vinyl isobutyrate, vinyl valerate, vinyl hexanoate, vinyl 2-methylhexanate, vinyl 2-ethylhexanoate, vinyl iso-octanoate, vinyl nonanoate, vinyl neodecanoate, vinyl decanoate, vinyl versatate, vinyl laurate, vinyl palmitate, and vinyl stearate.

## Patentansprüche

1. Körperpflegezusammensetzung, umfassend:
A) wenigstens ein reinigendes Tensid, das aus anionischen, amphoteren, kationischen oder nichtionischen Tensiden ausgewählt ist;
B) wenigstens ein Silikon-Konditionierungsmittel;
C) Wasser; und
D) ein nichtionisches amphiphiles Polymer, das aus einem polymerisierbaren Monomergemisch hergestellt ist, umfassend:
a) 55 bis 95 Gew.-%, in einem anderen Aspekt 60 bis 90 Gew.-%, in noch einem anderen Aspekt 65 bis 85 Gew.-% und in einem weiteren Aspekt 70 bis 80 Gew.-% wenigstens eines Vinylamid-Monomers (bezogen auf das Gewicht der gesamten vorhandenen Monomere);
b) 5 bis 45 Gew.-%, in einem anderen Aspekt 10 bis 40 Gew.-%, in noch einem anderen Aspekt 15 bis 35 Gew.-% und in einem weiteren Aspekt 20 bis 30 Gew.-% wenigstens eines Vinylesters einer aliphatischen Carbonsäure, die eine Acyl-Struktureinheit mit 2 bis 22 Kohlenstoffatomen enthält (bezogen auf das Gewicht der gesamten vorhandenen Monomere);
c) 0,01 bis 1 Gew.-%, in einem anderen Aspekt 0,01 bis 0,75 Gew.-%, in noch einem anderen Aspekt 0,1 bis 0,5 Gew.-% und in einem weiteren Aspekt 0,15 bis 0,3 Gew.-% wenigstens eines mehrfach ungesättigten Vernetzungsmonomers, das wenigstens zwei polymerisierbare ethylenisch ungesättigte Struktureinheiten enthält (bezogen auf das Gesamttrockengewicht des Polymers) und aus Polyallylethern von Trimethylolpropan, Polyallylethern von Pentaerythrit, Polyallylethern von Saccharose mit 2 bis 8 Allylgruppen pro Molekül und Gemischen davon ausgewählt ist;
d) 0 bis 10 Gew.-%, in einem anderen Aspekt 0,1 bis 5 Gew.-%, in noch einem anderen Aspekt 0,5 bis 3 Gew.-% und in einem weiteren Aspekt 0,75 bis 1 Gew.-% wenigstens eines C₁-C₂₂-Alkyl(meth)acrylats (bezogen auf das Gewicht der gesamten vorhandenen Monomere);
e) 0 bis 10 Gew.-% und in einem weiteren Aspekt 0,5, 1, 2 oder 3 bis 5 Gew.-% eines alkoxylierten assoziativen Monomers (bezogen auf das Gewicht der gesamten vorhandenen Monomere);
f) 0 bis 10 Gew.-% und in einem weiteren Aspekt 0,5, 1, 2 oder 3 bis 5 Gew.-% eines alkoxylierten semihydrophoben Monomers (bezogen auf das Gewicht der gesamten vorhandenen Monomere); und
g) 0 oder 0,5, 1, 2 oder 3 bis 5 Gew.-% wenigstens eines Vinylesters einer aliphatischen Carbonsäure, die eine Acyl-Struktureinheit mit 2 bis 22 Kohlenstoffatomen enthält (bezogen auf das Gewicht der gesamten vorhandenen Monomere), außer Vinylacetat.

2. Körperpflegezusammensetzung gemäß Anspruch 1, wobei die Monomerzusammensetzung weiterhin 0,01 bis 15 Gew.-% wenigstens eines copolymerisierbaren Monomers umfasst, das ausgewählt ist aus:
h) wenigstens einem C₁-C₅-Hydroxyalkyl(meth)acrylat;
i) wenigstens einem (Meth)acrylamid, das aus (Meth)acrylamid, N-(C₁-C₅)Alkyl(meth)acrylamid, N,N-Di(C₁-C₅)alkyl(meth)acrylamid, N-(C₁-C₅)Alkylamino(C₁-C₅)alkyl(meth)acrylamid oder N,N-Di(C₁-C₅)alkylamino(C₁-C₅)alkyl(meth)acrylamid ausgewählt ist;
j) wenigstens einem alpha-olefinischen Monomer; und Gemischen davon.

3. Körperpflegezusammensetzung gemäß einem der vorstehenden Ansprüche, wobei das wenigstens eine Vinylamid-Monomer aus N-Vinylformamid, N-Methyl-N-vinylformamid, N-(Hydroxymethyl)-N-vinylformamid, N-Vinylacetamid, N-Vinylmethylacetamid, N-(Hydroxymethyl)-N-vinylacetamid und Gemischen davon ausgewählt ist.

4. Körperpflegezusammensetzung gemäß einem der vorstehenden Ansprüche, wobei der wenigstens eine Vinylester einer aliphatischen Carbonsäure, die eine Acyl-Struktureinheit mit 2 bis 22 Kohlenstoffatomen enthält, aus Vinylacetat, Vinylpropionat, Vinylbutyrat, Vinylisobutyrat, Vinylvalerat, Vinylhexanoat, Vinyl-2-methylhexanoat, Vinyl-2-ethylhexanoat, Vinylisooctanoat, Vinylnonanoat, Vinylneodecanoat, Vinyldecanoat, Vinylversatat, Vinyllaurat, Vinylpalmitat, Vinylstearat und Gemischen davon ausgewählt ist.

5. Körperpflegezusammensetzung gemäß einem der vorstehenden Ansprüche, wobei das wenigstens eine mehrfach ungesättigte Vernetzungsmonomer aus einem Monomer mit durchschnittlich 2 vernetzungsfähigen ungesättigten funktionellen Gruppen, durchschnittlich 3 vernetzungsfähigen ungesättigten funktionellen Gruppen und Gemischen davon ausgewählt ist.

6. Körperpflegezusammensetzung gemäß einem der vorstehenden Ansprüche, wobei das C₁-C₅-Hydroxyalkyl(meth)acrylat-Monomer aus 2-Hydroxyethyl(meth)acrylat, 3-Hydroxypropyl(meth)acrylat, 4-Hydroxybutyl-(meth)acrylat und Gemischen davon ausgewählt ist.

7. Körperpflegezusammensetzung gemäß einem der vorstehenden Ansprüche, wobei das wenigstens eine C₁-C₂₂-Alkyl(meth)acrylat aus Methyl(meth)acrylat, Butyl(meth)acrylat, sek-Butyl(meth)acrylat, Isobutyl(meth)acrylat, Hexyl(meth)acrylat, Heptyl(meth)acrylat, Octyl(meth)acrylat, 2-Ethylhexyl(meth)acrylat, Decyl(meth)acrylat, Isodecyl(meth)acrylat, Lauryl(meth)acrylat, Tetradecyl(meth)acrylat, Hexadecyl(meth)acrylat, Stearyl(meth)acrylat, Behenyl(meth)acrylat oder Gemischen davon ausgewählt ist.

8. Körperpflegezusammensetzung gemäß einem der vorstehenden Ansprüche, wobei das alkoxylierte assoziative Monomer (i) einen polymerisierbaren ethylenisch ungesättigten Endgruppenteil, (ii) einen Polyoxyalkylen-Mittelabschnittsteil und (iii) einen hydrophoben Endgruppenteil, der 7 bis 30 Kohlenstoffatome enthält, umfasst;
wobei das assoziative Monomer vorzugsweise durch die Formeln VII und/oder VIIA dargestellt wird: 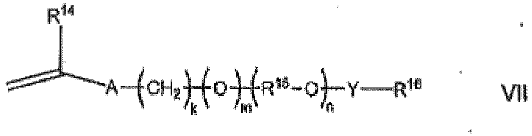 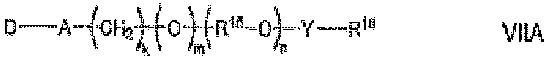
wobei R¹⁴ Wasserstoff oder Methyl ist, A = -CH₂C(O)O-, -C(O)O-, -O-, -CH₂O-, -NHC(O)NH-, -C(O)NH-, -Ar-(CE₂)_{z}-NHC(O)O-, -Ar-(CE₂)_{z}-NHC(O)NH- oder -CH₂CH₂NHC(O)-, ein zweiwertiger Alkylenrest, der 1 bis 5 Kohlenstoffatome enthält, ist, Ar ein zweiwertiges Arylen ist, E = H oder Methyl ist, z = 0 oder 1 ist, k eine ganze Zahl im Bereich von 0 bis 30 ist und m = 0 oder 1 ist, mit der Maßgabe, dass dann, wenn k = 0 ist, m = 0 ist, und wenn k im Bereich von 1 bis 30 liegt, m = 1 ist, D für eine Vinyl- oder Allyl-Struktureinheit steht, (R¹⁵-O)ₙ eine Polyoxyalkylen-Struktureinheit ist, die ein Homopolymer, statistisches Copolymer oder Blockcopolymer von C₂-C₄-Oxyalkyleneinheiten sein kann, R¹⁵ eine zweiwertige Alkylen-Struktureinheit ist, die aus C₂H₄, C₃H₆ oder C₄H₈ und Kombinationen davon ausgewählt ist, und n eine ganze Zahl im Bereich von in einem Aspekt 2 bis 150, in einem anderen Aspekt 10 bis 120 und in einem weiteren Aspekt 15 bis 60 ist, Y = -R¹⁵O-, -R¹⁵NH-, -C(O)-, -C(O)NH-, -R¹⁵NHC(O)NH- oder -C(O)NHC(O)- ist, R¹⁶ ein substituiertes oder unsubstituiertes Alkyl ist, das aus einem linearen C₈-C₃₀-Alkyl, einem verzweigten C₈-C₃₀-Alkyl, einem carbocyclischen C₇-C₃₀-Alkyl, einem mit C₂-C₃₀-Alkyl substituierten Phenyl, einem aralkylsubstituierten Phenyl und einem arylsubstituierten C₂-C₃₀-Alkyl ausgewählt ist, wobei die Alkylgruppe, carbocyclische Alkylgruppe, Arylgruppe, Phenylgruppe für R¹⁶ gegebenenfalls einen oder mehrere Substituenten umfasst, die aus der Gruppe ausgewählt sind, die aus einer Methylgruppe, Hydroxygruppe, Alkoxygruppe, Benzylgruppe, Styrylgruppe und einer Halogengruppe besteht, oder
wobei das assoziative Monomer durch Formel VIIB dargestellt wird: 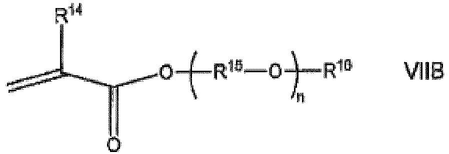 wobei R¹⁴ Wasserstoff oder Methyl ist, R¹⁵ eine zweiwertige Alkylen-Struktureinheit ist, die unabhängig aus C₂H₄, C₃H₆ und C₄H₈ ausgewählt ist, und n für eine ganze Zahl im Bereich von 10 bis 60 steht, (R¹⁵-O) in einer statistischen oder Blockkonfiguration angeordnet sein können, R¹⁶ ein substituiertes oder unsubstituiertes Alkyl ist, das aus einem linearen C₈-C₃₀-Alkyl, einem verzweigten C₈-C₃₀-Alkyl, einem carbocyclischen C₇-C₃₀-Alkyl, einem mit C₂-C₃₀-Alkyl substituierten Phenyl, einem aralkylsubstituierten Phenyl und einem arylsubstituierten C₂-C₃₀-Alkyl ausgewählt ist, wobei die Alkylgruppe, Arylgruppe, Phenylgruppe für R¹⁶ gegebenenfalls einen oder mehrere Substituenten umfasst, die aus der Gruppe ausgewählt sind, die aus einer Hydroxygruppe, Alkoxygruppe, Benzylgruppe, Styrylgruppe und einer Halogengruppe besteht.

9. Körperpflegezusammensetzung gemäß einem der vorstehenden Ansprüche, wobei das semihydrophobe Monomer (i) einen polymerisierbaren ethylenisch ungesättigten Endgruppenteil, (ii) einen Polyoxyalkylen-Mittelabschnittsteil und (iii) einen Endgruppenteil, der aus Wasserstoff oder einer Alkylgruppe, die 1 bis 4 Kohlenstoffatome enthält, ausgewählt ist, umfasst, wobei das semihydrophobe Monomer vorzugsweise aus wenigstens einem Monomer ausgewählt ist, das durch die Formeln VIII und IX dargestellt wird: 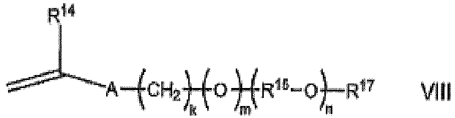 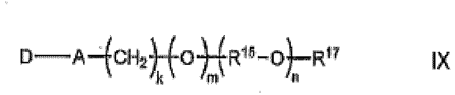
wobei R¹⁴ Wasserstoff oder Methyl ist, A = -CH₂C(O)O-, -C(O)O-, -O-, -CH₂O-, -NHC(O)NH-, -C(O)NH-, -Ar-(CE₂)_{z}-NHC(O)O-, -Ar-(CE₂)_{z}-NHC(O)NH- oder -CH₂CH₂NHC(O)- ist, Ar ein zweiwertiges Arylen ist, E = H oder Methyl ist, z = 0 oder 1 ist, k eine ganze Zahl im Bereich von 0 bis 30 ist und m = 0 oder 1 ist, mit der Maßgabe, dass dann, wenn k = 0 ist, m = 0 ist, und wenn k im Bereich von 1 bis 30 liegt, m = 1 ist, (R¹⁵-O)ₙ eine Polyoxyalkylen-Struktureinheit ist, die ein Homopolymer, statistisches Copolymer oder Blockcopolymer von C₂-C₄-Oxyalkyleneinheiten sein kann, R¹⁵ eine zweiwertige Alkylen-Struktureinheit ist, die aus C₂H₄, C₃H₆ oder C₄H₈ und Kombinationen davon ausgewählt ist, und n eine ganze Zahl im Bereich von in einem Aspekt 2 bis 150, in einem anderen Aspekt 5 bis 120, in einem weiteren Aspekt 10 bis 60 und in noch einem weiteren Aspekt 15 bis 30 ist, R¹⁷ aus Wasserstoff und einer linearen oder verzweigten C₁-C₄-Alkylgruppe ausgewählt ist und D für eine Vinyl- oder Allyl-Struktureinheit steht, oder
wobei das semihydrophobe Monomer aus wenigstens einem Monomer ausgewählt ist, das durch die Formeln VIIIA und VIIIB dargestellt wird:
CH₂=C(R¹⁴)C(O)O-(C₂H₄O)ₐ(C₃H₆O)_{b}-H VIIIA
CH₂=C(R¹⁴)C(O)O-(C₂H₄O)ₐ(C₃H₈O)_{b}-CH₃ VIIIB
wobei R¹⁴ Wasserstoff oder Methyl ist und "a" eine ganze Zahl im Bereich von in einem Aspekt 0 oder 2 bis 120, in einem anderen Aspekt 5 bis 45 und in einem weiteren Aspekt 10 bis 25 ist und "b" eine ganze Zahl im Bereich von in einem Aspekt 0 oder 2 bis 120, in einem anderen Aspekt 5 bis 45 und in einem weiteren Aspekt 10 bis 25 ist, mit der Maßgabe, dass "a" und "b" nicht gleichzeitig 0 sein können.

10. Körperpflegezusammensetzung gemäß einem der vorstehenden Ansprüche, wobei das wenigstens eine (Meth)acrylamid aus N-Methyl(meth)-acrylamid, N-Ethyl(meth)acrylamid, N-Propyl(meth)acrylamid, N-Isopropyl(meth)acrylamid, N-tert-Butyl(meth)acrylamid, N-tert-Octyl(meth)-acrylamid, N-(2-Hydroxyethyl)(meth)acrylamid, N-(3-Hydroxypropyl)-(meth)acrylamid, N,N-Dimethyl(meth)acrylamid, N,N-Diethyl(meth)acrylamid, N,N-(Di-2-hydroxyethyl)(meth)acrylamid, N,N-(Di-3-hydroxypropyl)(meth)acrylamid, N-Methyl-N-ethyl(meth)acrylamid, N,N-Dimethylaminoethyl(meth)acrylamid, N,N-Diethylaminoethyl(meth)acrylamid, N,N-Dimethylaminopropyl(meth)acrylamid und Gemischen davon ausgewählt ist.

11. Körperpflegezusammensetzung gemäß einem der vorstehenden Ansprüche, wobei das wenigstens eine alpha-olefinische Monomer aus Ethylen, Propylen, 1-Buten, Isobutylen, 1-Hexen, 1-Hepten, 4-Methyl-1-penten, Styrol, alpha-Methylstyrol und Gemischen davon ausgewählt ist.

12. Körperpflegezusammensetzung gemäß einem der vorstehenden Ansprüche, wobei das nichtionische amphiphile Polymer Repetiereinheiten umfasst, die aus einem Monomergemisch hergestellt sind, umfassend:
a) 55 bis 95 Gew.-%, in einem anderen Aspekt 60 bis 90 Gew.-%, in noch einem anderen Aspekt 65 bis 85 Gew.-% und in einem weiteren Aspekt 70 bis 80 Gew.-% N-Vinylpyrrolidon (bezogen auf das Gewicht der gesamten vorhandenen Monomere);
b) 5 bis 45 Gew.-%, in einem anderen Aspekt 10 bis 40 Gew.-%, in noch einem anderen Aspekt 15 bis 35 Gew.-% und in einem weiteren Aspekt 20 bis 30 Gew.-% Vinylacetat (bezogen auf das Gewicht der gesamten vorhandenen Monomere);
c) 0,01 bis 1 Gew.-%, in einem anderen Aspekt 0,01 bis 0,75 Gew.-%, in noch einem anderen Aspekt 0,1 bis 0,5 Gew.-% und in einem weiteren Aspekt 0,15 bis 0,3 Gew.-% wenigstens eines mehrfach ungesättigten Vernetzungsmonomers, das wenigstens zwei polymerisierbare ethylenisch ungesättigte Struktureinheiten enthält (bezogen auf das Gesamttrockengewicht des Polymers);
d) 0 bis 10 Gew.-%, in einem anderen Aspekt 0,1 bis 5 Gew.-%, in noch einem anderen Aspekt 0,5 bis 3 Gew.-% und in einem weiteren Aspekt 0,75 bis 1 Gew.-% (bezogen auf das Gewicht der gesamten vorhandenen Monomere) wenigstens eines C₁-C₂₂-Alkyl-(meth)acrylats, das aus Methyl(meth)acrylat, Butyl(meth)acrylat, sek-Butyl(meth)acrylat, Isobutyl(meth)acrylat, Hexyl(meth)acrylat, Heptyl(meth)acrylat, Octyl(meth)acrylat, 2-Ethylhexyl(meth)-acrylat, Decyl(meth)acrylat, Isodecyl(meth)acrylat, Lauryl(meth)-acrylat, Tetradecyl(meth)acrylat, Hexadecyl(meth)acrylat, Stearyl-(meth)acrylat oder Behenyl(meth)acrylat ausgewählt ist;
e) 0 bis 10 Gew.-% und in einem weiteren Aspekt 0,5, 1, 2 oder 3 bis 5 Gew.-% (bezogen auf das Gewicht der gesamten vorhandenen Monomere) eines alkoxylierten assoziativen Monomers, das aus Lauryl-polyethoxyliertem-(meth)acrylat, Cetyl-polyethoxyliertem-(meth)acrylat, Cetearyl-polyethoxyliertem-(meth)acrylat, Stearyl-polyethoxyliertem-(meth)acrylat, Arachidyl-polyethoxyliertem-(meth)acrylat, Behenyl-polyethoxyliertem-(meth)acrylat, Cerotyl-polyethoxyliertem-(meth)acrylat, Montanyl-polyethoxyliertem-(meth)acrylat, Melissyl-polyethoxyliertem-(meth)acrylat, Phenyl-polyethoxyliertem-(meth)acrylat, Nonylphenyl-polyethoxyliertem-(meth)acrylat, ω-Tristyrylphenyl-polyethoxyliertem-methacrylat ausgewählt ist, wobei der polyethoxylierte Teil des Monomers in einem Aspekt 2 bis 150, in einem anderen Aspekt 5 bis 120, in einem weiteren Aspekt 10 bis 60 und in noch einem weiteren Aspekt 15 bis 30 Ethylenoxideinheiten enthält.

13. Körperpflegezusammensetzung gemäß einem der vorstehenden Ansprüche, wobei das Silikon-Konditionierungsmittel ein nichtflüchtiges Silikon ist, das aus einem Silikonöl, Silikonkautschuk, Silikonharz und Gemischen davon ausgewählt ist, oder wobei das Silikon-Konditionierungsmittel aus Polydimethylsiloxanen (Dimethiconen), Polydiethylsiloxanen, Polydimethylsiloxanen mit terminalen Hydroxygruppen (Dimethiconolen), Polymethylphenylsiloxanen, Phenylmethylsiloxanen und Gemischen davon ausgewählt ist oder wobei das Silikon-Konditionierungsmittel aus einem aminofunktionellen Polydimethylsiloxan (Amodimethicon) ausgewählt ist.

14. Körperpflegezusammensetzung gemäß einem der vorstehenden Ansprüche, wobei die Körperpflegezusammensetzung aus Shampoos, Babyshampoos, Körperwaschmitteln, Duschgelen, flüssigen Handseifen, flüssigen Geschirrspülmitteln, Haustierreinigungsprodukten, feuchten Reinigungstüchern oder Gesichtsreinigern ausgewählt ist.

15. Verfahren zur Verringerung des Verlusts von Silikon, das aus einer silikonhaltigen konditionierenden Shampoozusammensetzung abgelagert wird, wobei das Verfahren das In-Kontakt-Bringen eines keratinösen Substrats mit einer reinigenden Zusammensetzung, die die Zusammensetzung gemäß einem der vorstehenden Ansprüche umfasst, umfasst.

16. Verfahren zur Verringerung des Verlusts von Silikon, das aus einer silikonhaltigen konditionierenden Shampoozusammensetzung auf einem keratinösen Substrat abgelagert wird, wobei das Verfahren die Mitverwendung von 1 bis 5 Gew.-% eines nichtionischen amphiphilen Dispersionspolymers umfasst, wobei das Polymer aus einem Monomergemisch hergestellt ist, umfassend:
a) 55 bis 95 Gew.-%, in einem anderen Aspekt 60 bis 90 Gew.-%, in noch einem anderen Aspekt 65 bis 85 Gew.-% und in einem weiteren Aspekt 70 bis 80 Gew.-% N-Vinylpyrrolidon (bezogen auf das Gewicht der gesamten vorhandenen Monomere);
b) 5 bis 45 Gew.-%, in einem anderen Aspekt 10 bis 40 Gew.-%, in noch einem anderen Aspekt 15 bis 35 Gew.-% und in einem weiteren Aspekt 20 bis 30 Gew.-% Vinylacetat (bezogen auf das Gewicht der gesamten vorhandenen Monomere);
c) 0,01 bis 1 Gew.-%, in einem anderen Aspekt 0,01 bis 0,75 Gew.-%, in noch einem anderen Aspekt 0,1 bis 0,5 Gew.-% und in einem weiteren Aspekt 0,15 bis 0,3 Gew.-% wenigstens eines mehrfach ungesättigten Vernetzungsmonomers, das wenigstens zwei polymerisierbare ethylenisch ungesättigte Struktureinheiten enthält (bezogen auf das Gesamttrockengewicht des Polymers) und aus Polyallylethern von Trimethylolpropan, Polyallylethern von Pentaerythrit, Polyallylethern von Saccharose mit 2 bis 8 Allylgruppen pro Molekül und Gemischen davon ausgewählt ist;
d) 0 bis 10 Gew.-%, in einem anderen Aspekt 0,1 bis 5 Gew.-%, in noch einem anderen Aspekt 0,5 bis 3 Gew.-% und in einem weiteren Aspekt 0,75 bis 1 Gew.-% (bezogen auf das Gewicht der gesamten vorhandenen Monomere) wenigstens eines C₁-C₂₂-Alkyl-(meth)acrylats, das aus Methyl(meth)acrylat, Butyl(meth)acrylat, sek-Butyl(meth)acrylat, Isobutyl(meth)acrylat, Hexyl(meth)acrylat, Heptyl(meth)acrylat, Octyl(meth)acrylat, 2-Ethylhexyl(meth)-acrylat, Decyl(meth)acrylat, Isodecyl(meth)acrylat, Lauryl(meth)-acrylat, Tetradecyl(meth)acrylat, Hexadecyl(meth)acrylat, Stearyl-(meth)acrylat oder Behenyl(meth)acrylat ausgewählt ist;
e) 0 bis 10 Gew.-% und in einem weiteren Aspekt 0,5, 1, 2 oder 3 bis 5 Gew.-% (bezogen auf das Gewicht der gesamten vorhandenen Monomere) eines alkoxylierten assoziativen Monomers, das aus Lauryl-polyethoxyliertem-(meth)acrylat, Cetyl-polyethoxyliertem-(meth)acrylat, Cetearyl-polyethoxyliertem-(meth)acrylat, Stearyl-polyethoxyliertem-(meth)acrylat, Arachidyl-polyethoxyliertem-(meth)acrylat, Behenyl-polyethoxyliertem-(meth)acrylat, Cerotyl-polyethoxyliertem-(meth)acrylat, Montanyl-polyethoxyliertem-(meth)acrylat, Melissyl-polyethoxyliertem-(meth)acrylat, Phenyl-polyethoxyliertem-(meth)acrylat, Nonylphenyl-polyethoxyliertem-(meth)acrylat, ω-Tristyrylphenyl-polyethoxyliertem-methacrylat ausgewählt ist, wobei der polyethoxylierte Teil des Monomers in einem Aspekt 2 bis 150, in einem anderen Aspekt 5 bis 120, in einem weiteren Aspekt 10 bis 60 und in noch einem weiteren Aspekt 15 bis 30 Ethylenoxideinheiten enthält;
f) 0 bis 10 Gew.-% und in einem weiteren Aspekt 0,5, 1, 2 oder 3 bis 5 Gew.-% (bezogen auf das Gewicht der gesamten vorhandenen Monomere) eines semihydrophoben Monomers, das aus Methoxypolyethylenglycolmethacrylat ausgewählt ist;
g) 0 bis 10 Gew.-% und 0,5, 1, 2 oder 3 bis 5 Gew.-% (bezogen auf das Gewicht der gesamten vorhandenen Monomere) wenigstens eines Vinylesters einer aliphatischen Carbonsäure, die eine Acyl-Struktureinheit mit 2 bis 22 Kohlenstoffatomen enthält, außer Vinylacetat, der aus Vinylpropionat, Vinylbutyrat, Vinylisobutyrat, Vinylvalerat, Vinylhexanoat, Vinyl-2-methylhexanoat, Vinyl-2-ethyl-hexanoat, Vinylisooctanoat, Vinylnonanoat, Vinylneodecanoat, Vinyldecanoat, Vinylversatat, Vinyllaurat, Vinylpalmitat und Vinylstearat ausgewählt ist.

17. Verfahren gemäß Anspruch 16, wobei das Monomergemisch umfasst:
a) 55 bis 95 Gew.-% N-Vinylpyrrolidon (bezogen auf das Gewicht der gesamten vorhandenen Monomere);
b) in einem weiteren Aspekt 5 bis 45 Gew.-% Vinylacetat (bezogen auf das Gewicht der gesamten vorhandenen Monomere);
c) 0,1 bis 1 Gew.-% wenigstens eines mehrfach ungesättigten Vernetzungsmonomers, das wenigstens zwei polymerisierbare ethylenisch ungesättigte Struktureinheiten enthält (bezogen auf das Gesamttrockengewicht des Polymers);
d) 0 oder 1 bis 5 Gew.-% (bezogen auf das Gewicht der gesamten vorhandenen Monomere) wenigstens eines Monomers, das aus Decyl(meth)acrylat, Isodecyl(meth)acrylat, Lauryl(meth)acrylat, Tetradecyl(meth)acrylat, Hexadecyl(meth)acrylat, Stearyl(meth)-acrylat oder Behenyl(meth)acrylat ausgewählt ist;
e) 0 oder 1 bis 5 Gew.-% (bezogen auf das Gewicht der gesamten vorhandenen Monomere) eines alkoxylierten assoziativen Monomers, das aus Lauryl-polyethoxyliertem-(meth)acrylat, Cetyl-polyethoxyliertem-(meth)acrylat, Cetearyl-polyethoxyliertem-(meth)-acrylat, Stearyl-polyethoxyliertem-(meth)acrylat, Behenyl-polyethoxyliertem-(meth)acrylat ausgewählt ist, wobei der polyethoxylierte Teil des Monomers 10 bis 60 Ethylenoxideinheiten enthält;
f) 0 oder 1 bis 5 Gew.-% (bezogen auf das Gewicht der gesamten vorhandenen Monomere) eines semihydrophoben Monomers, das aus Methoxypolyethylenglycolmethacrylat ausgewählt ist;
g) 0 oder 1 bis 5 Gew.-% (bezogen auf das Gewicht der gesamten vorhandenen Monomere) wenigstens eines Vinylesters einer aliphatischen Carbonsäure, die eine Acyl-Struktureinheit mit 2 bis 22 Kohlenstoffatomen enthält, außer Vinylacetat, der aus Vinylpropionat, Vinylbutyrat, Vinylisobutyrat, Vinylvalerat, Vinylhexanoat, Vinyl-2-methylhexanoat, Vinyl-2-ethylhexanoat, Vinylisooctanoat, Vinylnonanoat, Vinylneodecanoat, Vinyldecanoat, Vinylversatat, Vinyllaurat, Vinylpalmitat und Vinylstearat ausgewählt ist.

## Revendications

1. Composition de soin personnel comprenant :
A) au moins un tensioactif détersif sélectionné parmi un tensioactif anionique, amphotère, cationique, ou non ionique ;
B) au moins un agent de conditionnement à base de silicone
C) de l'eau ; et
D) un polymère amphiphile non ionique préparé à partir d'un mélange de monomères polymérisables comprenant :
a) de 55 à 95 % en poids, de 60 à 90 % en poids dans un autre aspect, de 65 à 85 % en poids dans encore un autre aspect, et de 70 à 80 % en poids dans un aspect supplémentaire, d'au moins un monomère de vinylamide (sur la base du poids des monomères totaux présents) ;
b) de 5 à 45 % en poids, de 10 à 40 % en poids dans un autre aspect, de 15 à 35 % en poids dans encore un autre aspect, et de 20 à 30 % en poids dans un aspect supplémentaire d'au moins un ester de vinyle d'un acide carboxylique aliphatique contenant un fragment acyle ayant 2 à 22 atomes de carbone (sur la base du poids des monomères totaux présents) ;
c) de 0,01 à 1 % en poids, de 0,01 à 0,75 % en poids dans un autre aspect, de 0,1 à 0,5 % en poids dans encore un autre aspect, et de 0,15 à 0,3 % en poids dans un aspect supplémentaire d'au moins un monomère polyinsaturé de réticulation contenant au moins deux fragments à insaturation éthylénique polymérisables (sur la base du poids sec total du polymère) sélectionné parmi les polyallyl éthers de triméthylolpropane, les polyallyl éthers de pentaérythritol, les polyallyl éthers de saccharose ayant 2 à 8 groupes allyle par molécule, et leurs mélanges ;
d) de 0 à 10 % en poids, de 0,1 à 5 % en poids dans un autre aspect, de 0,5 à 3 % en poids dans encore un autre aspect, et de 0,75 à 1 % en poids dans un aspect supplémentaire, d'au moins un (méth)acrylate d'alkyle en C₁ à C₂₂ (sur la base du poids des monomères totaux présents) ;
e) de 0 à 10 % en poids, et de 0,5, 1, 2, ou 3 à 5 % en poids dans un aspect supplémentaire d'un monomère associatif alcoxylé (sur la base du poids des monomères totaux présents) ;
f) de 0 à 10 % en poids, et de 0,5, 1, 2, ou 3 à 5 % en poids dans un aspect supplémentaire d'un monomère semi-hydrophobe alcoxylé (sur la base du poids des monomères totaux présents) ; et
g) de 0 ou 0,5, 1, 2, ou 3 à 5 % en poids d'au moins un ester de vinyle d'un acide carboxylique aliphatique contenant un fragment acyle ayant 2 à 22 atomes de carbone (sur la base du poids des monomères totaux présents) autre que l'acétate de vinyle.

2. Composition de soin personnel selon la revendication 1, dans laquelle ladite composition de monomères comprend en outre de 0,01 à 15 % en poids d'au moins un monomère copolymérisable sélectionné parmi :
h) au moins un (méth)acrylate d'hydroxyalkyle en C₁ à C₅ ;
i) au moins un (méth)acrylamide sélectionné parmi le (méth)acrylamide, un N-(alkyle en C₁ à C₅)(méth)acrylamide, un N,N-di(alkyle en C₁ à C₅) (méth)acrylamide, un N-((alkyle en C₁ à C₅)amino)-(alkyle en C₁ à C₅)(méth)-acrylamide ou un N,N-di((alkyle en C₁ à C₅)amino)-(alkyle en C₁ à C₅)(méth)-acrylamide ;
j) au moins un monomère alpha-oléfinique ; et leurs mélanges.

3. Composition de soin personnel selon l'une quelconque des revendications précédentes, dans laquelle ledit au moins un monomère de vinylamide est sélectionné parmi le N-vinylformamide, le N-méthyl-N-vinylformamide, le N-(hydroxyméthyl)-N-vinylformamide, le N-vinylacétamide, le N-vinylméthylacétamide, le N-(hydroxy-méthyl)-N-vinylacétamide, et leurs mélanges.

4. Composition de soin personnel selon l'une quelconque des revendications précédentes, dans laquelle ledit au moins un ester de vinyle d'un acide carboxylique aliphatique contenant un fragment acyle ayant 2 à 22 atomes de carbone est sélectionné parmi l'acétate de vinyle, le propionate de vinyle, le butyrate de vinyle, l'isobutyrate de vinyle, le valérate de vinyle, l'hexanoate de vinyle, le 2-méthyl-hexanate de vinyle, le 2-éthylhexanoate de vinyle, l'iso-octanoate de vinyle, le nonanoate de vinyle, le néodécanoate de vinyle, le décanoate de vinyle, le versatate de vinyle, le laurate de vinyle, le palmitate de vinyle, le stéarate de vinyle, et leurs mélanges.

5. Composition de soin personnel selon l'une quelconque des revendications précédentes, dans laquelle l'au moins un monomère polyinsaturé de réticulation est sélectionné parmi un monomère ayant une moyenne de 2 groupes fonctionnels insaturés réticulables, une moyenne de 3 groupes fonctionnels insaturés réticulables, et leurs mélanges.

6. Composition de soin personnel selon l'une quelconque des revendications précédentes, dans laquelle ledit monomère de (méth)acrylate d'hydroxyalkyle en C₁ à C₅ est sélectionné parmi le (méth)acrylate de 2-hydroxyéthyle, le (méth)acrylate de 3-hydroxypropyle, le (méth)acrylate de 4-hydroxybutyle, et leurs mélanges.

7. Composition de soin personnel selon l'une quelconque des revendications précédentes, dans laquelle ledit au moins un (méth)acrylate d'alkyle en C₁ à C₂₂ est sélectionné parmi le (méth)acrylate de méthyle, le (méth)acrylate de butyle, le (méth)acrylate de sec-butyle, le (méth)acrylate d'iso-butyle, le (méth)acrylate d'hexyle, le (méth)acrylate d'heptyle, le (méth)acrylate d'octyle, le (méth)acrylate de 2-éthylhexyle, le (méth)acrylate de décyle, le (méth)acrylate d'isodécyle, le (méth)acrylate de lauryle, le (méth)acrylate de tétradécyle, le (méth)acrylate d'hexadécyle, le (méth)acrylate de stéaryle, le (méth)acrylate de béhényle, ou leurs mélanges.

8. Composition de soin personnel selon l'une quelconque des revendications précédentes, dans laquelle ledit monomère associatif alcoxylé comprend (i) une partie de groupe terminal à insaturation éthylénique polymérisable, (ii) une partie de section médiane de type polyoxyalkylène, et (iii) une partie de groupe terminal hydrophobe contenant 7 à 30 atomes de carbone, de préférence dans laquelle ledit monomère associatif est représenté par les formules VII et/ou VIIA : 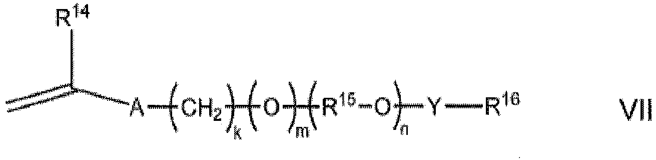 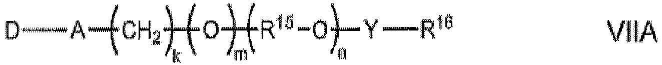
dans lesquelles R¹⁴ est un hydrogène ou un méthyle ; A est -CH₂C(O)O-, -C(O)O-, -O-, -CH₂O-, -NHC(O)NH-, -C(O)NH-,
-Ar-(CE₂)_{z}-NHC(O)O-, -Ar-(CE₂)_{z}-NHC(O)NH-, ou -CH₂CH₂NHC(O)- ; un radical alkylène divalent contenant 1 à 5 atomes de carbone ; Ar est arylène divalent ; E est H ou un méthyle ; z est 0 ou 1 ; k est un nombre entier allant de 0 à 30, et m est 0 ou 1, à condition que quand k est 0, m soit 0, et quand k est situé dans la plage allant de 1 à 30, m soit 1 ; D représente un fragment vinyle ou allyle ; (R¹⁵-O)ₙ est un fragment polyoxyalkylène, qui peut être un homopolymère, un copolymère statistique, ou un copolymère séquencé de motifs oxyalkylène en C₂ à C₄, R¹⁵ est un fragment alkylène divalent sélectionné parmi C₂H₄, C₃H₆, ou C₄H₈, et leurs combinaisons ; et n est un nombre entier situé dans la plage allant de 2 à 150 dans un aspect, de 10 à 120 dans un autre aspect, et de 15 à 60 dans un aspect supplémentaire ; Y est -R¹⁵O-, -R¹⁵NH-, -C(O)-, -C(O)NH-, -R¹⁵NHC(O)NH-, ou -C(O)NHC(O)- ; R¹⁶ est un alkyle substitué ou non substitué sélectionné parmi un alkyle linéaire en C₈ à C₃₀, un alkyle ramifié en C₈ à C₃₀, un alkyle carbocyclique en C₇ à C₃₀, un phényle substitué avec un alkyle en C₂ à C₃₀, un phényle substitué avec un aralkyle, et un alkyle en C₂ à C₃₀ substitué avec un aryle ; dans laquelle le groupe alkyle R¹⁶, le groupe alkyle carbocyclique, le groupe aryle, le groupe phényle comprennent facultativement un ou plusieurs substituants sélectionnés parmi le groupe sélectionné parmi un groupe méthyle, un groupe hydroxyle, un groupe alcoxyle, un groupe benzyle, un groupe styryle, et un groupe halogène, ou
dans laquelle ledit monomère associatif est représenté par la formule VIIB : 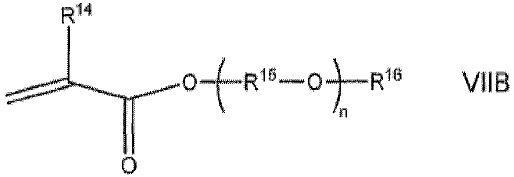 dans laquelle R¹⁴ est un hydrogène ou un méthyle ; R¹⁵ est un fragment alkylène divalent sélectionné indépendamment parmi C₂H₄, C₃H₆, ou C₄H₈, et n représente un nombre entier allant de 10 à 60, (R¹⁵-O) peut être agencé selon une configuration aléatoire ou séquencée ; R¹⁶ est un alkyle substitué ou non substitué sélectionné parmi un alkyle linéaire en C₈ à C₃₀, un alkyle ramifié en C₈ à C₃₀, un alkyle carbocyclique en C₇ à C₃₀, un phényle substitué avec un alkyle en C₂ à C₃₀, un phényle substitué avec un aralkyle, et un alkyle en C₂ à C₃₀ substitué avec un aryle, dans laquelle le groupe alkyle R¹⁶, le groupe aryle, le groupe phényle comprennent facultativement un ou plusieurs substituants sélectionnés dans le groupe consistant en un groupe hydroxyle, un groupe alcoxyle, un groupe benzyle, un groupe styryle, et un groupe halogène.

9. Composition de soin personnel selon l'une quelconque des revendications précédentes, dans laquelle ledit monomère semi-hydrophobe comprend (i) une partie de groupe terminal à insaturation éthylénique polymérisable, ii) une partie de section médiane de type polyoxyalkylène, et iii) une partie de groupe terminal sélectionnée parmi un hydrogène ou un groupe alkyle contenant 1 à 4 atomes de carbone, de préférence
dans laquelle ledit monomère semi-hydrophobe est sélectionné parmi au moins un monomère représenté par les formules VIII et IX : 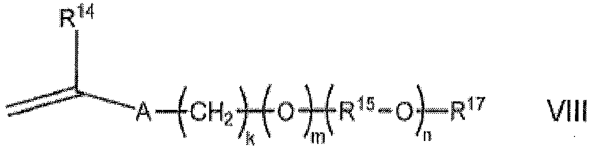 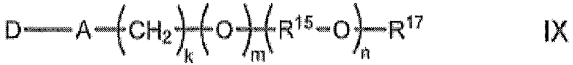
dans lesquelles R¹⁴ est un hydrogène ou un méthyle ; A est -CH₂C(O)O-, -C(O)O-, -O-, -CH₂O- ,-NHC(O)NH-, -C(O)NH-,
-Ar-(CE₂)_{z}-NHC(O)O-, -Ar-(CE₂)_{z}-NHC(O)NH-, ou -CH₂CH₂NHC(O)-; Ar est arylène divalent ; E est H ou un méthyle ; z est 0 ou 1 ; k est un nombre entier allant de 0 à 30, et m est 0 ou 1, à condition que quand k est 0, m soit 0, et quand k est situé dans la plage allant de 1 à 30, m soit 1 ; (R¹⁵-O)ₙ est un fragment polyoxyalkylène, qui peut être un homopolymère, un copolymère statistique, ou un copolymère séquencé de motifs oxyalkylène en C₂ à C₄, R¹⁵ est un fragment alkylène divalent sélectionné parmi C₂H₄, C₃H₆, ou C₄H₈, et leurs combinaisons ; et n est un nombre entier situé dans la plage allant de 2 à 150 dans un aspect, de 5 à 120 dans un autre aspect, de 10 à 60 dans un aspect supplémentaire, et de 15 à 30 dans encore un aspect supplémentaire ; R¹¹ est sélectionné parmi un hydrogène et un groupe alkyle linéaire ou ramifié en C₁ à C₄ ; et D représente un fragment vinyle ou allyle, ou
dans laquelle ledit monomère semi-hydrophobe est sélectionné parmi au moins un monomère représenté par les formules VIIIA et VIIIB :
CH₂=C(R¹⁴)C(O)O-(C₂H₄O)ₐ(C₃H₆O)_{b}-H VIIIA
CH₂=C(R¹⁴)C(O)O-(C₂H₄O)ₐ(C₃H₆O)_{b}-CH₃ VIIIB
dans lesquelles R¹⁴ est un hydrogène ou un méthyle, et « a » est un nombre entier allant de 0 ou 2 à 120 dans un aspect, de 5 à 45 dans un autre aspect, et de 10 à 25 dans un aspect supplémentaire, et « b » est un nombre entier allant de 0 ou 2 à 120 dans un aspect, de 5 à 45 dans un autre aspect, et de 10 à 25 dans un aspect supplémentaire, à condition que « a » et « b » ne puissent pas être 0 en même temps.

10. Composition de soin personnel selon l'une quelconque des revendications précédentes, dans laquelle ledit au moins un (méth)acrylamide est sélectionné parmi le N-méthyl(méth)acrylamide, le N-éthyl(méth)acrylamide, le N-propyl(méth)-acrylamide, le N-isopropyl(méth)acrylamide, le N-tert-butyl(méth)acrylamide, le N-tert-octyl(méth)acrylamide, le N-(2-hydroxyéthyl)(méth)acrylamide, le N-(3-hydroxypropyl)(méth)acrylamide ; le N,N-diméthyl(méth)acrylamide, le N,N-di-éthyl(méth)acrylamide, le N,N-(di-2-hydroxyéthyl)(méth)acrylamide, le N,N-(di-3-hydroxypropyl)(méth)acrylamide, le N-méthyl-N-éthyl(méth)acrylamide ; le N,N-di-méthylaminoéthyl(méth)acrylamide, le N,N-diéthylaminoéthyl(méth)acrylamide, le N,N-diméthylaminopropyl(méth)acrylamide ; et leurs mélanges.

11. Composition de soin personnel selon l'une quelconque des revendications précédentes, dans laquelle ledit au moins un monomère alpha-oléfinique est sélectionné parmi l'éthylène, le propylène, le 1-butène, l'iso-butylène, le 1-hexène, le 1-heptène, le 4-méthyl-1-pentène, le styrène, l'alpha-méthyl styrène, et leurs mélanges.

12. Composition de soin personnel selon l'une quelconque des revendications précédentes, dans laquelle ledit polymère amphiphile non ionique comprend des motifs répétitifs préparés à partir d'un mélange de monomères comprenant :
a) de 55 à 95 % en poids, de 60 à 90 % en poids dans un autre aspect, de 65 à 85 % en poids dans encore un autre aspect, et de 70 à 80 % en poids dans un aspect supplémentaire, de N-vinyl pyrrolidone (sur la base du poids des monomères totaux présents) ;
b) de 5 à 45 % en poids, de 10 à 40 % en poids dans un autre aspect, de 15 à 35 % en poids dans encore un autre aspect, et de 20 à 30 % en poids dans un aspect supplémentaire d'acétate de vinyle (sur la base du poids des monomères totaux présents) ;
c) de 0,01 à 1 % en poids, de 0,01 à 0,75 % en poids dans un autre aspect, de 0,1 à 0,5 % en poids dans encore un autre aspect, et de 0,15 à 0,3 % en poids dans un aspect supplémentaire d'au moins un monomère polyinsaturé de réticulation contenant au moins deux fragments à insaturation éthylénique polymérisables (sur la base du poids sec total du polymère) ;
d) de 0 à 10 % en poids, de 0,1 à 5 % en poids dans un autre aspect, de 0,5 à 3 % en poids dans encore un autre aspect, et de 0,75 à 1 % en poids dans un aspect supplémentaire (sur la base du poids des monomères totaux présents) d'au moins un (méth)acrylate d'alkyle en C₁ à C₂₂ sélectionné parmi le (méth)acrylate de méthyle, le (méth)acrylate de butyle, le (méth)acrylate de sec-butyle, le (méth)acrylate d'iso-butyle, le (méth)acrylate d'hexyle, le (méth)acrylate d'heptyle, le (méth)acrylate d'octyle, le (méth)acrylate de 2-éthylhexyle, le (méth)acrylate de décyle, le (méth)acrylate d'isodécyle, le (méth)acrylate de lauryle, le (méth)acrylate de tétradécyle, le (méth)acrylate d'hexadécyle, le (méth)acrylate de stéaryle, ou le (méth)acrylate de béhényle ; et
e) de 0 à 10 % en poids, et de 0,5, 1, 2, ou 3 à 5 % en poids dans un aspect supplémentaire (sur la base du poids des monomères totaux présents) d'un monomère associatif alcoxylé sélectionné parmi le (méth)acrylate de lauryle polyéthoxylé, le (méth)acrylate de cétyle polyéthoxylé, le (méth)acrylate de cétéaryle polyéthoxylé, le (méth)acrylate de stéaryle polyéthoxylé, le (méth)acrylate d'arachidyle polyéthoxylé, le (méth)acrylate de béhényle polyéthoxylé, le (méth)acrylate de cérotyle polyéthoxylé, le (méth)acrylate de montanyle polyéthoxylé, le (méth)acrylate de mélissyle polyéthoxylé, le (méth)acrylate de phényle polyéthoxylé, le (méth)acrylate de nonylphényle polyéthoxylé, le (méth)acrylate de ω-tristyrylphényle polyoxyéthyléné, où la partie polyéthoxylée du monomère contient de 2 à 150 motifs oxyde d'éthylène dans un aspect, de 5 à 120 dans un autre aspect, de 10 à 60 dans un aspect supplémentaire, et de 15 à 30 dans encore un aspect supplémentaire.

13. Composition de soin personnel selon l'une quelconque des revendications précédentes, dans laquelle ledit agent de conditionnement à base de silicone est une silicone non volatile sélectionnée parmi une huile de silicone, une gomme de silicone, une résine de silicone et leurs mélanges, ou
dans laquelle ledit agent de conditionnement à base de silicone est sélectionné parmi les polydiméthylsiloxanes (diméthicones), les polydiéthylsiloxanes, les polydiméthyl siloxanes ayant des groupes hydroxyle terminaux (diméthiconols), les polyméthyl-phénylsiloxanes, les phénylméthylsiloxanes, et leurs mélanges, ou
dans laquelle ledit agent de conditionnement à base de silicone est sélectionné parmi un polydiméthylsiloxane à fonction amino (amodiméthicone).

14. Composition de soin personnel selon l'une quelconque des revendications précédentes, dans laquelle ladite composition de soin personnel est sélectionnée parmi les shampooings, les shampooings pour bébé, les nettoyants corporels, les gels douches, les savons liquides pour les mains, les détergents liquides pour la vaisselle, un produit nettoyant pour les animaux de compagnie, les lingettes nettoyantes humides, ou les nettoyants pour le visage.

15. Procédé de réduction de la perte de silicone déposée à partir d'une composition de shampooing conditionneur contenant une silicone, ledit procédé comprenant la mise en contact d'un substrat kératineux avec une composition détersive comprenant la composition selon l'une quelconque des revendications précédentes.

16. Procédé de réduction de la perte de silicone déposée à partir d'une composition de shampooing conditionneur contenant une silicone sur un substrat kératineux, ledit procédé comprenant le fait d'y inclure de 1 à 5 % en poids d'un polymère de dispersion amphiphile non ionique, dans lequel ledit polymère est préparé à partir d'un mélange de monomères comprenant :
a) de 55 à 95 % en poids, de 60 à 90 % en poids dans un autre aspect, de 65 à 85 % en poids dans encore un autre aspect, et de 70 à 80 % en poids dans un aspect supplémentaire, de N-vinyl pyrrolidone (sur la base du poids des monomères totaux présents) ;
b) de 5 à 45 % en poids, de 10 à 40 % en poids dans un autre aspect, de 15 à 35 % en poids dans encore un autre aspect, et de 20 à 30 % en poids dans un aspect supplémentaire d'acétate de vinyle (sur la base du poids des monomères totaux présents) ;
c) de 0,01 à 1 % en poids, de 0,01 à 0,75 % en poids dans un autre aspect, de 0,1 à 0,5 % en poids dans encore un autre aspect, et de 0,15 à 0,3 % en poids dans un aspect supplémentaire d'au moins un monomère polyinsaturé de réticulation contenant au moins deux fragments à insaturation éthylénique polymérisables (sur la base du poids sec total du polymère) sélectionné parmi les polyallyl éthers de triméthylolpropane, les polyallyl éthers de pentaérythritol, les polyallyl éthers de saccharose ayant 2 à 8 groupes allyle par molécule, et leurs mélanges ;
d) de 0 à 10 % en poids, de 0,1 à 5 % en poids dans un autre aspect, de 0,5 à 3 % en poids dans encore un autre aspect, et de 0,75 à 1 % en poids dans un aspect supplémentaire (sur la base du poids des monomères totaux présents) d'au moins un (méth)acrylate d'alkyle en C₁ à C₂₂ sélectionné parmi le (méth)acrylate de méthyle, le (méth)acrylate de butyle, le (méth)acrylate de sec-butyle, le (méth)acrylate d'iso-butyle, le (méth)acrylate d'hexyle, le (méth)acrylate d'heptyle, le (méth)acrylate d'octyle, le (méth)acrylate de 2-éthylhexyle, le (méth)acrylate de décyle, le (méth)acrylate d'isodécyle, le (méth)acrylate de lauryle, le (méth)acrylate de tétradécyle, le (méth)acrylate d'hexadécyle, le (méth)acrylate de stéaryle, ou le (méth)acrylate de béhényle ; et
e) de 0 à 10 % en poids, et de 0,5, 1, 2, ou 3 à 5 % en poids dans un aspect supplémentaire (sur la base du poids des monomères totaux présents) d'un monomère associatif alcoxylé sélectionné parmi le (méth)acrylate de lauryle polyéthoxylé, le (méth)acrylate de cétyle polyéthoxylé, le (méth)acrylate de cétéaryle polyéthoxylé, le (méth)acrylate de stéaryle polyéthoxylé, le (méth)acrylate d'arachidyle polyéthoxylé, le (méth)acrylate de béhényle polyéthoxylé, le (méth)acrylate de cérotyle polyéthoxylé, le (méth)acrylate de montanyle polyéthoxylé, le (méth)acrylate de mélissyle polyéthoxylé, le (méth)acrylate de phényle polyéthoxylé, le (méth)acrylate de nonylphényle polyéthoxylé, le (méth)acrylate de ω-tristyrylphényle polyoxyéthyléné, où la partie polyéthoxylée du monomère contient de 2 à 150 motifs oxyde d'éthylène dans un aspect, de 5 à 120 dans un autre aspect, de 10 à 60 dans un aspect supplémentaire, et de 15 à 30 dans encore un aspect supplémentaire ;
f) de 0 à 10 % en poids, et de 0,5, 1, 2, ou 3 à 5 % en poids dans un aspect supplémentaire (sur la base du poids des monomères totaux présents) d'un monomère semi-hydrophobe sélectionné parmi un méthacrylate de méthoxy polyéthylèneglycol ;
g) de 0 à 10 % en poids, et de 0,5, 1, 2, ou 3 à 5 % en poids (sur la base du poids des monomères totaux présents) d'au moins un ester de vinyle d'un acide carboxylique aliphatique contenant un fragment acyle contenant 2 à 22 atomes de carbone autre que l'acétate de vinyle sélectionné parmi le propionate de vinyle, le butyrate de vinyle, l'isobutyrate de vinyle, le valérate de vinyle, l'hexanoate de vinyle, le 2-méthylhexanate de vinyle, le 2-éthylhexanoate de vinyle, l'iso-octanoate de vinyle, le nonanoate de vinyle, le néodécanoate de vinyle, le décanoate de vinyle, le versatate de vinyle, le laurate de vinyle, le palmitate de vinyle, et le stéarate de vinyle.

17. Procédé selon la revendication 16, dans lequel ledit mélange de monomères comprend :
a) de 55 à 95 % en poids, de N-vinyl pyrrolidone (sur la base du poids des monomères totaux présents) ;
b) de 5 à 45 % en poids, dans un aspect supplémentaire d'acétate de vinyle (sur la base du poids des monomères totaux présents) ;
c) de 0,1 à 1 % en poids d'au moins un monomère polyinsaturé de réticulation contenant au moins deux fragments à insaturation éthylénique polymérisables (sur la base du poids sec total du polymère) ;
d) de 0 ou 1 à 5 % en poids (sur la base du poids des monomères totaux présents) d'au moins un monomère sélectionné parmi le (méth)acrylate de décyle, le (méth)acrylate d'isodécyle, le (méth)acrylate de lauryle, le (méth)acrylate de tétradécyle, le (méth)acrylate d'hexadécyle, le (méth)acrylate de stéaryle, ou le (méth)acrylate de béhényle ;
e) de 0 ou 1 à 5 % en poids (sur la base du poids des monomères totaux présents) d'un monomère associatif alcoxylé sélectionné parmi le (méth)acrylate de lauryle polyéthoxylé, le (méth)acrylate de cétyle polyéthoxylé, le (méth)acrylate de cétéaryle polyéthoxylé, le (méth)acrylate de stéaryle polyéthoxylé, le (méth)acrylate de béhényle polyéthoxylé, où la partie polyéthoxylée du monomère contient de 10 à 60 motifs oxyde d'éthylène ;
f) de 0 ou 1 à 5 % en poids (sur la base du poids des monomères totaux présents) d'un monomère semi-hydrophobe sélectionné parmi un méthacrylate de méthoxy polyéthylèneglycol ; et
g) de 0 ou 1 à 5 % en poids (sur la base du poids des monomères totaux présents) d'au moins un ester de vinyle d'un acide carboxylique aliphatique contenant un fragment acyle ayant 2 à 22 atomes de carbone autre que l'acétate de vinyle sélectionné parmi le propionate de vinyle, le butyrate de vinyle, l'isobutyrate de vinyle, le valérate de vinyle, l'hexanoate de vinyle, le 2-méthyl-hexanate de vinyle, le 2-éthylhexanoate de vinyle, l'iso-octanoate de vinyle, le nonanoate de vinyle, le néodécanoate de vinyle, le décanoate de vinyle, le versatate de vinyle, le laurate de vinyle, le palmitate de vinyle, et le stéarate de vinyle.
